# EUROPEAN PATENT SPECIFICATION

(11) **EP 0 925 302 B1**
(45) Date of publication and mention of the grant of the patent: **20.11.2002**
(21) Application number: 97938139.9
(22) Date of filing: 08.08.1997
(51) Int. Cl.: C07F 7/08, C07D 209/04

(54) **PALLADIUM CATALYZED INDOLIZATION**
PALLADIUMKATALYSIERTE INDOLISIERUNG
INDOLISATION CATALYSEE AU PALLADIUM

(30) Priority: 13.08.1996 US 23860 P; 12.09.1996 GB 9619064; 31.10.1996 US 30155 P
(43) Date of publication of application: 30.06.1999
(73) Proprietor: MERCK & CO., INC., Rahway, New Jersey 07065 (US)
(72) Inventor: CHEN, Cheng-Yi, Rahway, NJ 07065 (US); LARSEN, Robert, D., Rahway, NJ 07065 (US)
(74) Representative: Thompson, John Dr.
(86) International application number: US9713799
(87) International publication number: WO98006725

(56) References cited:
- WO-A-95/32197
- MURAKAMI, M. ET AL.: "the preparation of (1-(arylimino)alkyl)zinc by the alpha-addition of organozinc to isocyanide" THE JOURNAL OF ORGANIC CHEMISTRY, 1988, pages 4158-4159, XP002043166
- CHEMICAL ABSTRACTS, vol. 89, no. 25, 18 December 1978 Columbus, Ohio, US; abstract no. 215478k, BROOK, A.G. ET AL.: "the syntheses and properties of c-silylimines and their thermal rearrangements to n-silenamines" XP002043170 & CANADIAN JOURNAL OF CHEMISTRY, vol. 56, no. 17, 1978, pages 2286-2291,
- LAROCK, R.C. ET AL.: "synthesis of indoles via palladium-catalyzed heteroannulation of internal alkynes" JOURNAL OF THE AMERICAN CHEMICAL SOCIETY, vol. 113, 1991, pages 6689-6690, XP002043167 cited in the application
- CHEN, C.Y. ET AL.: "synthesis of the 5-ht1d receptor agonist mk-0462 via a pd-catalyzed coupling reaction" TETRAHEDRON LETTERS, vol. 35, 1994, pages 6981-6984, XP000608320
- IRITANI, K. ET AL.: "palladium catalyzed reaction of 2-alkynylanilines with allyl chlorides. formation of 3-allylindoles" TETRAHEDRON LETTERS, vol. 29, 1988, pages 1799-1802, XP002043168
- CHEN, C.-Y. ET AL.: "syntheses of indoles via a palladium-catalyzed annulation between iodoanilines and ketones" JOURNAL OF ORGANIC CHEMISTRY, May 1997, pages 2676-2677, XP002043169

## Description

### BACKGROUND OF THE INVENTION

The present invention relates to the preparation of 2-silyl protected indoles from the palladium catalyzed reaction of halo-anilines and acyl silanes (which act as aldehyde synthons). These 2-silyl derivitives may, according to the present invention, be converted to the corresponding 2-unsubstituted indole derivative. The present invention is particularly useful in the preparation of 5-heterocyclic substituted tryptamines such as 5-(1,2,4-triazol-1-yl) tryptamine. These compounds are therapeutically active as anti-migraine agents.

Generally, indoles are prepared via the Fischer-indole reaction. For example, Chen *et al., J. Org. Chem., 59*:3738 (1994) disclose the preparation of N,N-dimethyl tryptamines from 4-substituted hydrazines and dimethylaminobutyraldehyde dimethyl acetal using 4% H₂SO₄. However, the yields are often low, particularly for compounds having triazole substitution. Benzyltriazoles are unstable to the Fischer indole reaction conditions, which generally lead to polymerization of the triazole moiety, and the production of oligomers.

Larock *et al., J. Am. Chem. Soc., 113*:6689 (1991) have shown that coupling of an iodoaniline species with an internal acetylene using palladium catalysis gives 2,3-disubstituted indoles in good-to-excellent yields. Smith *et al* ., have also demonstrated this for 4-pyrimidinyl and pyridinyl derivatives of indol-3-yl-alkyl piperazines as in published EPO 548 831 Al. Two other applications of this methodology have been demonstrated in the syntheses of hetero-condensed pyrroles and tryptophans. See Wensbo *et al., Tetrahedron Lett., 34*:2823 (1993); Wensbo *et al*., *Tetrahedron Lett., 34*:6471 (1993). However, all of these methods require triphenylphosphine, as part of the catalyst system, which is an environmental hazard. An alternate process has been developed to overcome the low yield of the Fischer-indole reaction with certain starting materials and to avoid the use of the environmentally hazardous triphenylphosphine. This process is detailed in PCT publication WO 95/32197 and involves the palladium-catalyzed coupling/ring closure of a 3-iodo-4-aminobenzyltriazole with a suitably protected butynol derivative to the corresponding tryptophol, followed by conversion of the hydroxyethyl moiety to a dimethyl amino ethyl; as shown below: 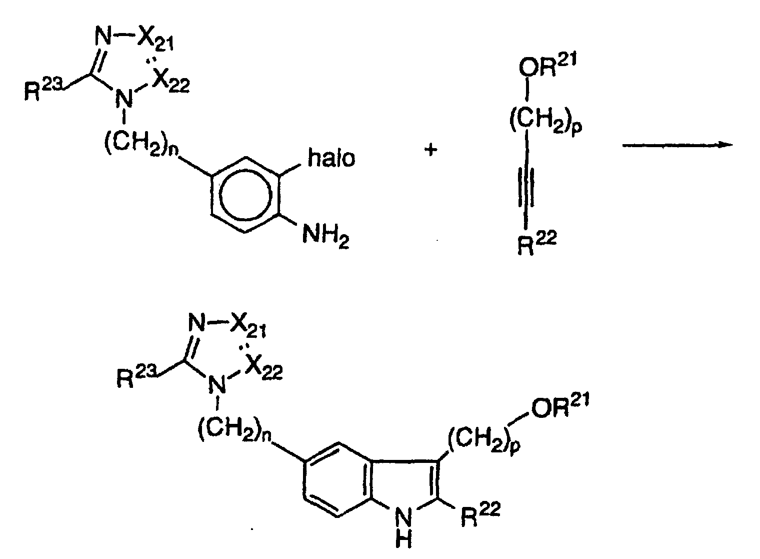 said process being carried out in a dry inert organic solvent for the starting materials at a temperature in the range of about 70 to 120°C, in the presence of a palladium catalyst, and in the presence of an inorganic or organic amine compound,
wherein:
X₂₁ and X₂₂ are independently ring nitrogen or carbon atoms;
halo represents Br or I;
n is an integer from 0-1;
p is an integer from 1-4;
R²³ is H or linear or branched C₁-C₄ alkyl;
R²¹ is H or a radical with functions as a hydroxy protecting group, which is removable, under mild acid hydrolyses, for example, by contacting with a mixture of HCl/MeOH, e.g. 1:1 2N HCl/MeOH at 0-30°C, and
R²² is a radical which functions as a terminal acetylene carbon protecting group.

However, although the chemistry of this alternate process is effective, the starting materials, butynol and triethylsilyl chloride, are relatively expensive. The present invention provides for a cost-effective indolization procedure.

Iida et al., J. Org. Chem. 45:2938-2942 (1980) describe intramolecular cyclization of 3-((2-bromoaryl)amino) cyclohex-2-en-1-ones with catalytic palladium in the presence of triphenyl phosphine, as well as the reaction of aryl amines with β-diketones to form the corresponding secondary enaminone followed by N-ethylation to form the corresponding tertiary enaminones and subsequent intramolecular cyclization in the presence of equimolar palladium acetate.

Sakamota et al., Synthesis, p. 215-218 (1990), describe palladium-catalyzed cyclization of β-(2-halophenyl)amino substituted α,β-unsaturated ketones and esters to produce 2,3-disubstituted indoles. The procedure of Sakamota et al., also employs phosphine.

The present invention has particular application in the synthesis of 5HT_{1D} receptor agonists. These agonists mimic the effects of the neurotransmitter serotonin which acts as a vasoconstrictor in the brain. 5HT_{1D} receptor agonists display beneficial properties in migraine therapies. Over the past few years an extensive effort has been devoted to the development of *N,N*-dialkyltryptamines as 5-HT_{1D} receptor agonists to achieve the desired activity and selectivity for the treatment of migraine. Sumatriptan is the first of this class of drugs to be approved for this purpose. MK-0462 (developed by Merck & Co.), is described in U.S. 5,298,520 and is also a potent 5-HT_{1D} receptor agonist that is undergoing clinical studies.

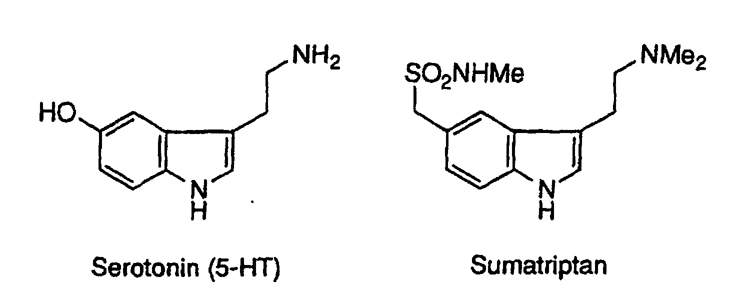

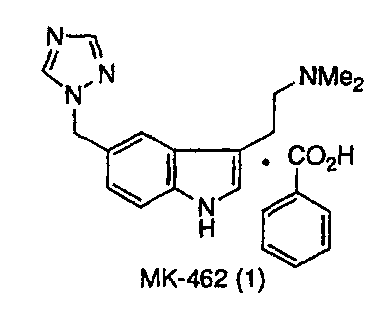

Thus, the present invention also provides for an efficient and cost-effective synthesis of 5-heterocyclic-substituted tryptamines useful in the treatment of migraine headaches.

### SUMMARY OF THE INVENTION

We have found that 2-unsubstituted indoles can be cost-effectively synthesized in high yield by the palladium-catalyzed coupling/ring closure of a 2-halo or 2-trifluoromethylsulfonyloxy (OTf)- aniline and an acyl silane derivative, followed by deprotection of the silyl protecting groups. The process of the present invention is particularly useful to form indoles containing acid-labile substituents such as triazole, acetyl, ketal, cyano, and carbamate, or indoles having a good leaving group in the benzyl position. The advantages to the present process are that it does not require the use of triphenylphosphine or tetrabutyl ammonium chloride or lithium chloride. When applied to 5-triazolyl substituted indoles, the present process also eliminates the tendency of triazolyl polymerization in the Fischer indole synthesis.

By this invention, there is provided a process comprising the step of contacting a compound of Structure I with a compound of Structure II to form a compound of Structure III: 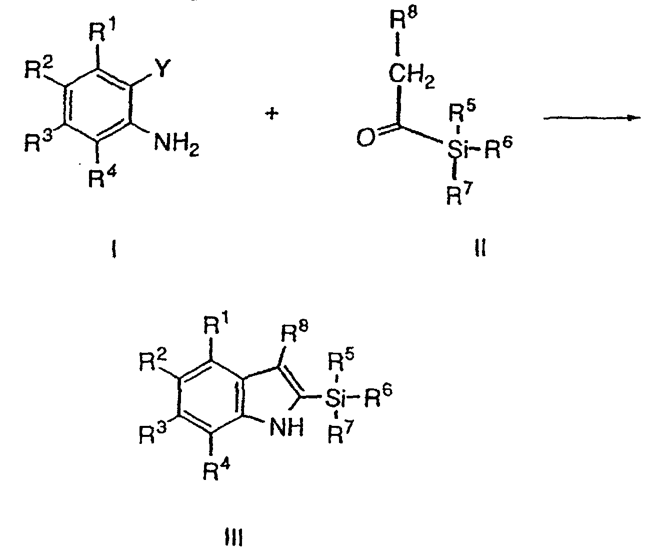 in the presence of a palladium catalyst and a proton acceptor,
wherein:
Y is selected from Br, I and triflate;
R¹, R², R³ and R⁴ are each independently selected from:
   (1) hydrogen;
   (2) 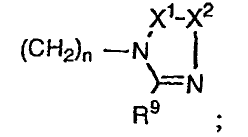
   (3) C₁₋₆ alkyl;
   (4) -(CH₂)ₙ-Z
      wherein Z represents:
      (a) hydrogen,
      (b) halogen,
      (c) cyano,
      (d) nitro,
      (e) trifluoromethyl,
      (f) -OR¹⁰,
      (g) -OCOR¹⁰,
      (h) -OCONR¹⁰R¹¹,
      (i) -OCH₂CN,
      (j) -OCH₂CONR¹⁰R¹¹,
      (k) -SR¹⁰, provided that R¹⁰ is not hydrogen,
      (l) -SOR¹⁰,
      (m) -SO₂R¹⁰,
      (n) SO₂NR¹⁰R¹¹,
      (o) -NR¹⁰R¹¹,
      (p) -NR¹⁰COR¹¹,
      (q) -NR¹⁰CO₂R¹¹,
      (r) -NR¹⁰SO₂R¹¹,
      (s) -COR¹⁰,
      (t) -CO₂R¹⁰,
      (u) -CONR¹⁰R¹¹,
      or Z is a group of formula (Za), (Zb), (Zc), or (Zd): 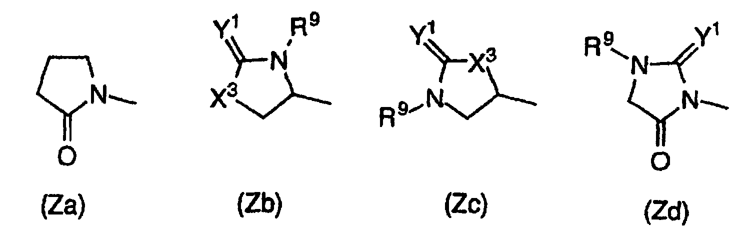 or Z represents an optionally substituted five-membered heteroaromatic ring selected from furan, thiophene, pyrrole, oxazole, thiazole, isoxazole, isothiazole, imidazole, pyrazole, oxadiazole, thiadiazole, triazole and tetrazole;
R⁵, R⁶, and R⁷ are each independently selected from:
   (1) C₁₋₆alkyl,
   (2) -O-C₁₋₆ alkyl, and
   (3) phenyl;
R⁸ is selected from:
   (1) hydrogen,
   (2) -R¹⁹-OH,
   (3) -R¹⁹-O-R¹⁷, and
   (4) -R¹⁹NR¹²R¹³, and
   (5) -R¹⁹-Z¹
      wherein: Z¹ is a 3 to 7 membered heterocyclic ring wherein the ring members are selected from 1 to 2 nitrogen atoms and wherein the heterocyclic ring may be subsituted by one or more R¹⁴;
R⁹ is selected from:
   (1) hydrogen, and
   (2) C₁₋₄alkyl;
R¹⁰ and R¹¹ are each independently selected from:
   (1) hydrogen,
   (2) C₁₋₆ alkyl,
   (3) trifluoromethyl,
   (4) phenyl, optionally substituted with one or more R²⁰ substituents
   (5) methylphenyl, optionally substituted with one or more R²⁰ substituents, and
   (6) an arylC₁₋₆alkyl- or heteroaryl C₁₋₆alkyl- group. optionally substituted with one or more R²⁰ substituents, or
   R¹⁰ and R¹¹ when linked through a nitrogen atom, together represent the residue of an optionally substituted azetidine, pyrrolidine, piperidine, morpholine or piperazine ring, optionally substituted with one or more R¹⁸ substituents;
R¹² and R¹³ are each independently selected from:
   (1) C₁₋₄alkyl,
   (2) C₆aryl-C₁₋₄ alkyl- wherein aryl may be unsubstituted or substituted with one to three substituents selected from methyl, halo, and halomethyl,
R¹⁴ is selected from:
   (1) aryl-C₁₋₆alkyl-, unsubstituted or substituted with one to three R²⁰ substitutents, and
   (2) heteroaryl-C₁₋₆alkyl-, unsubstituted or substituted with one to three R²⁰ substitutents,
R¹⁵ and R¹⁶ are each independently selected from
   (1) hydrogen,
   (2) C₁₋₆alkyl,
   (3) C₃₋₇cycloalkyl,
   (4) C₃₋₇cycloalkylC₁₋₆alkyl-,
   (5) indanyl,
   (6) aryl,
   (7) arylC₁₋₆alkyl-,
   (8) C₃₋₇heterocycloalkyl-,
   (9) C₃₋₇heterocycloalkylC₁₋₆alkyl-,
   (10) heteroaryl, and
   (11) heteroarylC₁₋₆alkyl-;
R¹⁷ is selected from a hydroxy protecting group that is removable under mild acid hydrolysis;
R¹⁸ is selected from:
   (1) C₁₋₆alkyl-,
   (2) arylC₁₋₆alkyl-,
   (3) C₁₋₆alkoxy-,
   (4) C₂₋₆alkyoxycarbonyl-, and
   (5) C₁₋₆alkylaminocarbonyl-;
R¹⁹ is a straight or branched C₁₋₆alkyl chain;
R²⁰ is selected from:
   (1) fluoro,
   (2) cyano,
   (3) trifluoromethyl,
   (4) C₁₋₆alkyl,
   (5) haloC₁₋₆alkyl-,
   (6) aryl,
   (7) triazolyl,
   (8) tetrazolyl,
   (9) tetrazolyl-C₁₋₆alkyl-,
   (10) hydroxy,
   (11) C₁₋₆alkoxy-,
   (12) C₁₋₆alkylthio-,
   (13) C₂₋₆alkoxycarbonyl-,
   (14) C₂₋₆alkylcarbonyl-,
   (15) C₁₋₆alkylsulphonyl-,
   (16) arylsulfonyl-,
   (17) C₂₋₆alkylcarbonylamino-,
   (18) arylcarbonylamino-,
   (19) C₂₋₆alkoxycarbonylamino-,
   (20) N-C₁₋₆alkyl-N-C₂₋₆alkoxyamino-,
   (21) carbonylamino-,
   (22) mono- or diarylaminocarbonylamino-,
   (23) pyrrolidinylcarbonylamino-,
   (24) piperidinylcarbonylamino-,
   (25) aminocarbonyl-,
   (26) aminocarbonylamino-,
   (27) C₁₋₆alkylaminocarbonyl-,
   (28) C₁₋₆alkylaminocarbonylamino-,
   (29) diC₁₋₆alkylaminocarbonyl-,
   (30) diC₁₋₆alkylaminocarbonylamino-,
   (31) pyrrolidinylcarbonylamino-,
   (32) piperidinylcarbonylamino-,
   (33) aminosulfonyl-,
   (34) C₁₋₆alkylaminosulfonyl-,
   (35) C₁₋₆alkylsulfonylamino-,
   (36) C₁₋₆alkylsulfonylaminomethyl-,
   (37) arylsulfonylamino-,
   (38) diC₁₋₆alkylaminosulfonyl-,
   (39) aminosulphonylmethyl-,
   (40) C₁₋₆alkylaminosulfonylmethyl-,
   (41) diC₁₋₆alkylaminosulfonylmethyl-,
   (42) -(CH₂)ₘOR¹⁵,
   (43) -(CH₂)ₘSR¹⁵, provided that R¹⁵ is not hydrogen,
   (44) -(CH₂)ₘSOR¹⁵,
   (45) -(CH₂)ₘSO₂R¹⁵,
   (46) -(CH₂)ₘNR¹⁵R¹⁶,
   (47) =O, and
   (48)

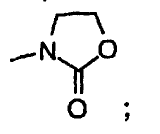

- X¹ and X²: are each independently selected from ring nitrogen or ring carbon atoms;
- X³: is selected from the group consisting of oxygen, sulfur, -NH- or methylene;
- Y¹: is oxygen or sulfur;
- n: is an integer independently selected at each occurrence from 0 and 1;
- m: is an integer selected independently at each occurrence from 0 to 4; and
- p: is an integer from 0 to 3.

The process is preferably carried out in a dry organic solvent inert for the starting materials at a temperature range of 90 to 120°C in the presence of a palladium catalyst, and in the presence of a proton acceptor which may be an inorganic or organic amine compound.

Where Z in the compounds of formulae I and III above represents a five-membered heteroaromatic ring, this ring may be optionally substituted by one or, where possible, two substituents. As will be appreciated, where Z represents oxadiazole, thiadiazole or tetrazole ring, only one subsitutent will be possible; otherwise, one or two optional substituents may be accommodated around the five-membered heteroaromatic ring Z. Examples of suitable substituents on the five-membered heteroaromatic ring Z include C₁₋₆ alkyl, C₂₋₆alkenyl, C₂₋₆alkynyl, C₃₋₇ cycloalkyl, aryl, aryl C₁₋₆alkyl, C₃₋₇ heterocycloalkyl, heteroaryl, C₁₋₆alkoxy, C₁₋₆alkylthio, amino, C₁₋₆alkylamino, diC₁₋₆alkylamino, halogen, cyano, and trifluoromethyl.

Further, the present invention relates to deprotecting the compound of structural formula III to obtain the 2-unsubstituted indole of structural formula IV: 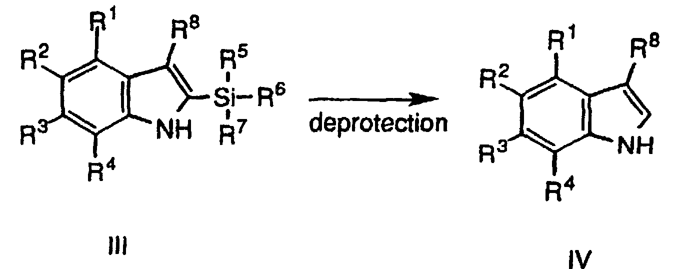 wherein R¹, R², R³, R⁴, R⁵, R⁶, R⁷, and R⁸ are as defined above.

Still further, the present invention is also directed to the novel intermediates of structural formulae (V) and (VI).

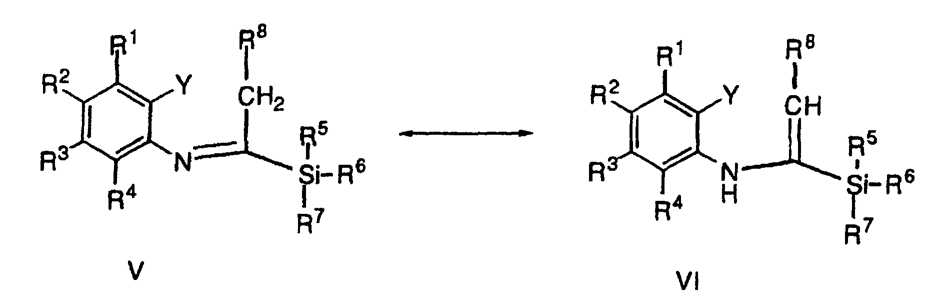

### DETAILED DESCRIPTION OF THE INVENTION

Combinations of substituents and/or variables are permissible only if such combinations result in stable compounds.

In one embodiment of the present invention, R¹, R³, and R⁴ are each hydrogen and R², R⁵, R⁶, R⁷, R⁸, and Y are as defined above. Representative examples of Si(R⁵)(R⁶)(R⁷) radicals include trimethyl silyl, triethyl silyl, tributyl silyl, triphenyl silyl, dimethyl-t-butyl silyl, dimethylphenyl silyl, diphenylmethyl silyl, triisopropyl silyl, and the like, as well as any mixture of -Si(OC₁₋₆ alkyl)₃₋ₙ(C₁₋₆ alkyl)ₙ, where n is 0, 1, or 2.

R¹⁷ acts as a protecting group for the hydroxyl group and may have the structure Si(R⁵)(R⁶)(R⁷), as described above.

The term "triflate" or "OTf" refers to the trifluoromethane sulfonyloxy group.

When an amine is included as a substituent on a compound in the present invention, in order to optimize the conditions of the reaction and to obtain better yields, the amine may have to be protected, as is known in the art, and the protecting group removed following the coupling reaction.

When a carbonyl group is included as a substituent on a compound in the present invention, in order to optimize the conditions of the reaction and to obtain better yields, the carbonyl group may have to be protected, as is known in the art, and the protecting group removed following the coupling reaction.

When an alkenyl or alkynyl group is included as a substituent on a compound in the present invention, in order optimize the conditions of the reaction and obtain better yields, the alkenyl or alkynyl group may be protected by conversion to an oxide, followed by reduction. Alternatively, an additional elimination strategy may be employed.

As used herein "alkyl", particularly the expression "C₁₋₆ alkyl", includes methyl and ethyl groups and straight chained or branched propyl, butyl, pentyl and hexyl groups. Particular alkyl groups are methyl, ethyl, n-propyl, isopropyl and t-butyl. Derived expressions such as C₁₋₆alkyoxy, C₁₋₆alkylthio, and C₁₋₆alkyl amino are to be construed accordingly.

The expression "C₂₋₆alkenyl" as used herein refers to straight chained and branched alkenyl groups containing from 2 to 6 carbon atoms. Typical examples include vinyl, allyl, dimethylallyl and butenyl groups.

The expression "C₂₋₆alkynyl" as used herein refers to straight-chained and branched alkynyl groups containing from 2 to 6 carbon atoms. Typical examples include ethynyl and propargyl groups.

Typical C₃₋₇cycloalkyl groups include cyclopropyl, cyclobutyl, cyclopentyl and cyclohexyl.

Typical aryl groups include phenyl and naphthyl. More particularly, aryl is phenyl.

Particular indanyl groups include indan-1-yl and indan-2-yl.

Particular arylC₁₋₆alkyl groups include benzyl, phenylethyl, phenylpropyl and naphthylmethyl.

Suitable heterocycloalkyl groups include azetidinyl, pyrrolidinyl, piperidinyl, piperazinyl and morpholinyl.

Suitable heteroaryl groups include pyridyl, quinolyl, isoquinolyl, pyridazinyl, pyrimidinyl, pyrazinyl, pyranyl, furyl, benzofuryl, dibenzofuryl, thienyl, benzthienyl, pyrrolyl, indolyl, pyrazolyl, indazolyl, oxazolyl, isoxazolyl, thiazolyl, isothiazolyl, imidazolyl, benzimidazolyl, oxadiazolyl, thiadiazolyl, triazolyl, and tetrazolyl groups.

The expression "heteroaryl C₁₋₆alkyl" as used herein includes furylmethyl, furylethyl, thienylmethyl, thienylethyl, oxazolylmethyl, oxazolylethyl, thiazolylmethyl, thiazolylethyl., imidazolylmethyl, imidazolylethyl, oxadiazolylmethyl, oxadiazolylethyl, thiadiazolylmethyl, thiadiazolylethyl, triazolylmethyl, triazolylethyl, tetrazolylmethyl, tetrazolylethyl, pyridylmethyl, pyridylethyl, pyridinylmethyl, pyrazinylmethyl, quinolylmethyl, and isoquinolylmethyl.

The term "halogen" as used herein includes fluorine, chlorine, bromine and iodine, especially fluorine, unless otherwise specified.

The process of the present invention is preferably carried out in a dry organic solvent inert for the starting materials in the presence of a palladium catalyst, and in the presence of an inorganic or organic base which is not a "catalyst poison". Preferably, the present process is carried out at an elevated temperature.

In the process of the present invention, Structure I is coupled with Structure II to form Structure III via a palladium catalyzed reaction in a dry inert organic solvent containing a soluble palladium catalyst and in the presence of a proton acceptor, being an aromatic amine, alkylamine or inorganic base, which is not a "catalyst poison," at a temperature of about 90-120°C.

The organic solvent useful in the process of the present invention must be one in which Structure I, Structure II and the palladium catalyst are soluble and compatible and is chemically inert under the reaction conditions. Preferred are DMSO (dimethylsulfoxide) and amide solvents such as DMF (N,N-dimethylformamide), DMAC (N,N-dimethylacetamide), and NMP (N-methyl-pyrrolidinone). Most preferred is DMF.

The acyl silane of structural formula (II) is generally employed in excess based on the the 2-halo or 2-(OTf)- aniline of structural formula (I). A useful range is about 1.0 to 3 fold, based on the 2-halo or 2-(OTf)- aniline of structural formula I. The acyl silane may be favorably employed at a two-fold excess, based on the 2-halo or 2-(OTf)- aniline of structural formula I.

The proton acceptor useful in the process of the present invention is a basic compound which can be organic or inorganic and acts as a proton acceptor and is not a "catalyst poison". By the term "catalyst poison" is meant interaction with the catalyst to inhibit its catalytic activity and prevent the coupling/ring closure between structures I and II from occurring. Suitable classes of proton acceptors include alkylamines, aromatic amines, heterocyclic amines, phosphates and the like.
Alkylamines are the preferred proton acceptor in the process of the present invention. Particular alkylamines that may be employed include: DABCO (1,4-diazabicyclo[2.2.2]octane), quinuclidine, t-butylamine, 2,2,6,6,-tetramethylpiperidine and di-t-butyl-amine. DABCO is particularly preferred because it reduces the appearance of impurities in the reaction because it is resistant to oxidation to the imine in the reaction conditions of the process of the present invention.

The proton acceptor is generally employed in excess based on the the 2-halo or 2-(OTf)- aniline of structural formula (I). A useful range is about 2 to 4 fold excess, based on the 2-halo or 2-(OTf)- aniline of structural formula (I). The proton acceptor may be favorably employed at a three-fold excess, based on the 2-halo or 2-(OTf)- aniline of structural formula (I).

The palladium catalyst useful in the reaction can be selected from the following classes: Pd alkanoates, Pd acetonates, Pd halides, Pd halide complexes, Pd-benzylidine acetone complexes, as well as triaryl Pd phosphine complexes. Representative examples include, but are not limited to: Pd(II) acetate, Pd(II) acetylacetonate, Pd(O)bis-dibenzylidene acetone ("dba"), Pd(II) bromide, Pd(II) chloride, Pd(II) iodide, Pd(II) sulfate, Pd(II)trifluoroacetate, Pd(II) Cl₂(CH₃CN)₂, Pd₂ (dba)₃, and Pd(II)Cl₂(PhCN)₂. A useful catalyst is palladium acetate.

The palladium catalyst is employed in an amount of about 0.5 to 5 mole percent based on the halo aniline of structural formula I. A useful range is about 2 to 3 mole percent of soluble palladium catalyst, based on the halo aniline of structural formula I.

A dehydrating agent, such as magnesium sulfate may also be favorably employed in the process of coupling Structure I with Structure II to form Structure III according to the present invention. Although not necessary to the process, a dehydrating agent can assist the enamine formation by removal of the water of condensation.

The reaction is carried out in the temperature range of 90 to 120°C. A useful temperature is about 100-105°C. Generally, the reaction is carried out under a dry, inert atmosphere at atmospheric pressure. It is useful to carry out the reaction under a nitrogen atmosphere.

The progress of the reaction may be monitored by means known in the art, including thin-layer silica gel chromatography (TLC), high pressure liquid chromatography (HPLC), gas chromatography (GC), and nuclear magnetic resonance spectroscopy (NMR). Preferably HPLC or TLC is employed, most preferably HPLC. When the reaction is complete, generally in 8 to 72 hours, the reaction mixture is cooled to room temperature and the product is separated by traditional means, e.g. by taking up with organic solvent, such as isopropyl acetate and washing with water and/or other aqueous solutions. The product may then be purified by means known in the art, including preparative thin-layer silica gel chromatography, silica gel chromatography, HPLC, crystallization, and solid-phase extraction. Preferably, the product is purified by silica gel chromatography or crystallization.

Further, the present invention relates to deprotecting the compound of structural formula III to obtain the 2-unsubstituted indole of structural formula IV: 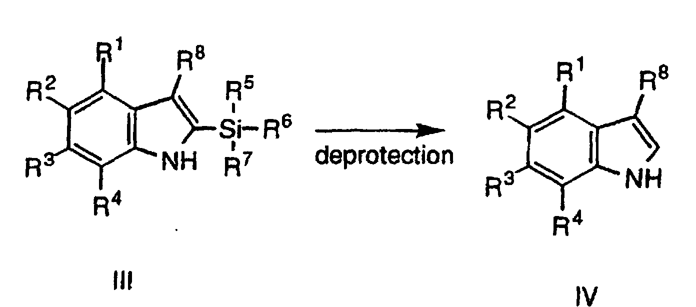 wherein R¹, R², R³, R⁴, R⁵, R⁶, R⁷, and R⁸ are as defined above.

The Si(R⁵)(R⁶)(R⁷) group is removable by Lewis acid catalyzed deprotection with a Lewis acid such as AlCl₃, or aqueous HCI HF, HBr, and HF. The silyl group may be removed, for example, by treatment with nucleophilic acid, e.g., contacting with about a 1:1 by volume 2N HCl/MeOH solvent mixture at 0-30°C for 1 to 24 hours to completely remove the silyl protecting groups. Alternatively, the silyl group may be removed by fluoride deprotection. This step is referred to herein as "deprotection". Removal of the Si(R⁵)(R⁶)(R⁷) group produces the 2-unsubstituted indole.

Particular 2-unsubstituted indole compounds according to structural formula (IV) that may be made according to the process of the present invention include:
(1) 1-benzyl-4-[3-(5-(1,2,4-triazol-4-yl)-1H-indol-3-yl)propyl]piperazin-2-one;
(2) 1-(2-phenylethyl)-4-[3-(5-(1,2,4-triazol-4-yl)-1H-indol-3-yl)propyl]piperazin-2-one;
(3) 1-[2-(3-fluorophenyl)ethyl]-4-[3-(5-(1,2,4-triazol-4-yl)-1H-indol-3-yl)propyl]piperazin-2-one;
(4) (3*S*)-3-(*N*-benzyl)aminomethyl-1-[2-(5-(*N*-methyl)-aminosulphonylmethyl)-1*H*-indol-3-yl)ethyl]pyrrolidine;
(5) (3*S*)-3-(*N*-benzyl)aminomethyl-1-[2-(5-(aminosulphonylmethyl)-1*H*-indol-3-yl)ethyl]pyrrolidine;
(6) (3*S*)-3-(*N*-benzyl)aminomethyl-(*S*)-1-[2-(5-(2-oxo-1,3-oxazolidin-4-ylmethyl)-1*H*-indol-3-yl)ethyl]pyrrolidine;
(7) (3*S*)-3-[*N*-(*R*)-α-(hydroxymethyl)benzyl]aminomethyl-(*S*)-1-[2-(5-(2-oxo-1,3-oxazolidin-4-ylmethyl)-1*H*-indol-3-yl)ethyl]pyrrolidine;
(8) (3*S*)-3-[*N*-(*S*)-α-methylbenzyl]aminomethyl-(*S*)-1-[2-(5-(2-oxo-1,3-oxazolidin-4-ylmethyl)-1*H*-indol-3-yl)ethyl]-pyrrolidine;
(9) 4-[*N*-(*R*)-α-(hydroxymethyl)benzyl]amino-(*S*)-1-[3-(5-(2-oxo-1,3-oxazolidin-4-ylmethyl)-1*H*-indol-3-yl)propyl]-piperidine;
(10) (35)-3-(*N*-benzyl-N-methyl)aminomethyl-(*S*)-1-[2-(5-(3-methyl-2-oxo-1,3-oxazolidin-4-ylmethyl)-1*H*-indol-3-yl)ethyl]pyrrolidine;
(11) (3*R*)-3-[*N*-(*S*)-α-methylbenzyl-*N*-methyl]aminomethyl-(*S*)-1-[2-(5-(2-oxo-1,3-oxazolidin-4-ylmethyl)-1*H*-indol-3-yl)ethyl]pyrrolidine;
(12) (3*R*)-3-[*N*-(*S*)-α-methylbenzyl-*N*-methyl]aminomethyl-(*S*)-1-[2-(5-(3-methyl-2-oxo-1,3-oxazolidin-4-ylmethyl)-1*H*-indol-3-yl)ethyl]pyrrolidine;
(13) (3*S*)-3-[*N*-(4-fluorobenzyl)-*N*-methyl]aminomethyl-(*S*)-1-[2-(5-(3-methyl-2-oxo-1,3-oxazolidin-4-ylmethyl)-1*H*-indol-3-yl)ethyl]pyrrolidine;
(14) 4-benzyl12-{2-fluoromethyl-3-(5-(1,2,4-triazol-4-yl)-1H-indol-3-yl)propyl]piperazine;
(15) 4-[2-(3-fluorophenyl)ethyl]-1-[2-fluoro-3-(5-(1,2,4-triazol-4-yl)-1H-indol-3-yl)propyl]piperazine;
(16) 4-benzyl-1-[2-fluoro-3-(5-(1,2,4-triazol-4-yl)-1H-indol-3-yl)propyl]piperidine;
(17) 4-(N-benzyl-N-methylamino)-1-[2-fluoro-3-(5-(1,2,4-triazol-4-yl)-1H-indol-3-yl)propyl]piperidine;
(18) 4-[(R)-2-hydroxy-1-(4-fluorophenyl)ethylamino]-1-[2-fluoro-3-(5-(1,2,4-triazol-4-yl)-1H-indol-3-yl)propyl]-piperidine;
(19) 7-benzyl-2-[2-(5-(1,2,4-triazol-4-yl)-1H-indol-3-yl)ethyl]-*cis*-2,7-diazabicyclo[3.3.0]octane;
(20) 7-(3-furylmethyl-2-[2-(5-(1,2,4-triazol-4-yl)-1H-indol-3-yl)ethyl]-*cis*-2,7-diazabicyclo[3.3.0]octane;
(21) 7-(2-phenylethyl-2-[2-(5-(1,2,4-triazol-4-yl)-1H-indol-3-yl)ethyl]*-cis*-2,7-diazabicyclo[3.3.0]octane;
(22) 7-(4-fluorobenzyl-2-[2-(5-(1,2,4-triazol-4-yl)-1H-indol-3-yl)ethyl]-*cis*-2,7-diazabicyclo[3.3.0]octane;
(23) 7-(2,4-difluorobenzyl-2-[2-(5-(1,2,4-triazol-4-yl)-1H-indol-3-yl)ethyl]*-cis*-2,7-diazabicyclo[3.3.0]octane;
(24) 4-(2,2-difluoro-1-oxo-2-phenylethyl)-1-[3-(5-(1,2,4-triazol-4-yl)-1H-indol-3-yl)propyl]piperazine;
(25) 4-benzyl-3-methyl-1-[3-(5-(1,2,4-triazol-4-yI)-1H-indol-3-yl)propyl]-1,2,5,6-tetrahydropyridine;
(26) 4-benzyl-3-methoxymethyl-1-[3-(5-(1,2,4-triazol-4-yl)-1H-indol-3-yl)propyl]-1,2,5,6-tetrahydropyridine;
(27) 1-(2-hydroxy-1-phenylethyl)-4-[3-(5-(1,2,4-triazol-4-yl)-1*H*-indol-3-yl)propyl]piperidine;
(28) 1-[2-(2-fluorophenyl)ethyl]-4-[3-(5-(1,2,4-triazol-4-yl)-1*H*-indol-3-yI)propyl]piperidine;
(29) 1-benzyl-4-[3-(5-(1,2,4-triazol-4-yl)-1*H*-indol-3-yl)propyl]-piperidine;
(30) 1-(3,3-dimethylbutyl)-4-[3-(5-(1,2,4-triazol-4-yl)-1*H*-indol-3-yl)propyl]piperidine;
(31) 1-(2-phenylethyl)-4-[3-(5-(1,2,4-triazol-4-yl)-1*H*-indol-3-yl)propyl]piperidine;
(32) 1 -cyclohexylmethyl-4-[3-(5-(1,2,4-triazol-4-yl)-1*H*-indol-3-yl)propyl]piperidine;
(33) 1-(3-phenylpropyl)-4-[3-(5-(1,2,4-triazol-4-yl)-1*H*-indol-3-yl)propyl]piperidine;
(34) 1-[2-(3-fluorophenyl)ethyl]-4-[3-(5-(1,2,4-triazol-4-yl)-1*H*-indol-3-yl)propyl]piperidine;
(35) 1-[2-(4-trifluoromethylphenyl)ethyl]-4-[3-(5-(1,2,4-triazol-4-yl)-1*H*-indol-3-yl)propyl]piperidine;
(36) 1 -[2-(3,4-difluorophenyl)ethyl]-4-[3-(5-(1,2,4-triazol-4-yl)-1*H*-indol-3-yl)propyl]piperidine;
(37) N-methyl-2-phenyl-2-[4-(3-(5-(1,2,4-triazol-4-yl)-1*H*-indol-3-yl)propyl)piperidin-1-yl]acetamide;
(38) 1-(2-oxo-2-phenylethyl)-4-[3-(5-(1,2,4-triazol-4-yl)-1*H*-indol-3-yl)propyl]piperidine;
(39) 1-(2-phenylpropyl)-4-[3-(5-(1,2,4-triazol-4-yl)-1*H*-indol-3-yl)propyl]piperidine;
(40) 4-benzyl-4-fluoro-1-[3-(5-(1,2,4-triazol-4-yl)-1*H*-indol-3-yl)propyl]piperidine;
(41) 4-fluoro-4-[2-(3-fluorophenyl)ethyl]-1-[3-(5-(1,2,4-triazol-4-yl)-1*H*-indol-3-yl)propyl]piperidine;
(42) 4-fluoro-4-(3-fluorobenzyl)-1-[3-(5-(1,2,4-triazol-4-yl)-1*H*-indol-3-yl)propyl]piperidine;
(43) 4-fluoro-4-(2-fluorobenzyl)-1-[3-(5-(1,2,4-triazol-4-yl)-1*H*-indol-3-yl)propyl]piperidine;
(44) 4-benzyl-4-methoxy-1-[3-(5-(1,2,4-triazol-4-yl)-1*H*-indol-3-yl)propyl]piperidine;
(45) 4-benzyl-4-methoxy-1-[3-(5-(1,2,4-triazol-1-ylmethyl)-1*H*-indol-3-yl)propyl]piperidine;
(46) 4-(2-fluorobenzyl)-4-methoxy-1-[3-(5-(1,2,4-triazol-4-yl)-1 *H*-indol-3-yl)propyl]piperidine;
(47) 4-(3-fluorobenzyl)-4-methoxy-1-[3-(5-(1,2,4-triazol-4-yl)-1*H*-indol-3-yl)propyl]piperidine;
(48) 4-(4-fluorobenzyl)-4-methoxy-1-[3-(5-(1,2,4-triazol-4-yl)-1*H*-indol-3-yl)propyl]piperidine;
(49) 1-[3-(5-(1,2,4-triazol-4-yl)-1*H*-indol-3-yl)propyl]-4-[2-(oxazol-2-on-3-yl)-1-phenylethyl]piperazine;
(50) 1-[3-(5-(1,2,4-triazol-4-yl)-1*H*-indol-3-yl)propyl]-4-[2-(oxazolidin-2-on-3-yl)-1-phenylethyl]piperazine;
(51) 1-[3-(5-(1,2,4-triazol-4-yl)-1*H*-indol-3-yl)propyl]-4-[1-(4-fluorophenyl)-2-(oxazolidin-2-on-3-yl)ethyl]piperazine;
(52) 1-[3-(5-(1,2,4-triazol-4-yl)-1*H*-indol-3-yl)propyl]-4-(3-hydroxy-1-phenylpropyl)piperazine;
(53) 1-[3-(5-(1,2,4-triazol-4-yl)-1*H*-indol-3-yl)propyl]-4-[2-(imidazol-1-yl)-1-phenylethyl)piperazine;
(54) 1-[3-(5-(1,2,4-triazol-4-yl)-1*H*-indol-3-yl)propyl]-4-[1-(4-fluorophenyl)-2-hydroxyethyl]piperazine;
(55) 1-[3-(5-(1,2,4-triazol-4-yl)-1*H*-indol-3-yl)propyl]-4-[1-(4-fluorophenyl)-2-methoxyethyl]piperazine;
(56) 1-[3-(5-(1,2,4-triazol-4-yl)-1*H*-indol-3-yl)propyl]-4-[3-(5-methyl-1,2,4-oxadiazol-3-yl)-1-phenylpropyl]piperazine;
(57) 1-[3-(5-(1,2,4-triazol-4-yl)-1*H*-indol-3-yl)propyl]-4-[2-benzyloxy-1-(4-fluorophenyl)ethyl]piperazine;
(58) 1-[3-(5-(1,2,4-triazol-4-yl)-1*H*-indol-3-yl)propyl]-4-[1-(4-fluorophenyl)-3-methoxypropyl]piperazine;
(59) 1-[3-(5-(1,2,4-triazol-4-yl)-1*H*-indol-3-yl)propyl]-4-[1-(4-fluorophenyl)-2-(imidazol- 1-yl)ethyl]piperazine;
(60) 1-[3-(5-(1,2,4-triazol-4-yl)-1*H*-indol-3-yl)propyl]-4-[1-phenyl-2-(pyrrolidin-1-yl)ethyl]piperazine;
(61) 1-[3-(5-(1,2,4-triazol-4-yl)-1*H*-indol-3-yl)propyl]-4-[1-(4-fluorophenyl)-3-hydroxypropyl]piperazine;
(62) 1 -[3-(5-(1,2,4-triazol-4-yl)-1*H*-indol-3-yl)propyl]-4-[3-(imidazol-1-yl)-1-phenylpropyl]piperazine;
(63) 1-[3-(5-(1,2,4-triazol-4-yl)-1*H*-indol-3-yl)propyl]-4-(3-hydroxy-2-phenylpropyl)piperazine;
(64) 1 -[3-(5-(1,2,4-triazol-4-yl)-1*H*-indol-3-yl)propyl]-4-(3-methoxy-2-phenylpropyl)piperazine;
(65) 1-[3-(5-(1,2,4-triazol-4-yl)-1*H*-indol-3-yl)propyl]-4-[2-(4-fluorophenyl)-3-hydroxypropyl]piperazine;
(66) 1 -[3-(5-(1,2,4-triazol-4-yl)-1*H*-indol-3-yl)propyl]-4-[1-(4-fluorophenyl)prop-2-yl]piperazine;
(67) 1-[3-(5-(1,2,4-triazol-4-yl)-1*H*-indol-3-yl)propyl]-4-[2-(4-fluorophenyl)propyl]piperazine;
(68) 1-[3-(5-(1,2,4-triazol-4-yl)-1*H*-indol-3-yl)propyl]-4-[1-(4-fluorophenyl)-3-hydroxyprop-2-yl]piperazine;
(69) 1-[3-(5-(1,2,4-triazol-4-yl)-1*H*-indol-3-yl)propyl]-4-[1-(4-fluorophenyl)-3-methoxyprop-2-yl]piperazine;
(70) 1-[3-(5-(1,2,4-triazol-4-yl)-1*H*-indol-3-yl)propyl]-4-[2-(3-fluorophenyl)-ethyl]piperazine;
(71) 1-[3-(5-(1,2,4-triazol-4-yl)-1*H*-indol-3-yl)propyl]-4-[2-(2-fluorophenyl)-ethyl]piperazine;
(72) 1 -[3-(5-(1,2,4-triazol-4-yl)-1*H*-indol-3-yl)propyl]-4-[2-(4-fluorophenyl)-ethyl]piperazine;
(73) 1-[3-(5-(1,2,4-triazol-4-yl)-1*H*-indol-3-yl)propyl]-4-[2-(3-methoxyphenyl)-ethyl]piperazine;
(74) 1-[3-(5-(1,2,4-triazol-4-yl)-1*H*-indol-3-yl)propyl)-4-[2-(3-trifluoromethyl-phenyl)ethyl]piperazine;
(75) 1-[3-(5-(1,2,4-triazol-4-yl)-1*H*-indol-3-yl)propyl]-4-[2-(3,4-difluorophenyl)-ethyl]piperazine;
(76) 1-[3-(5-(1,2,4-triazol-4-yl)-1*H*-indol-3-yl)propyl]-4-[2-(2,4-difluorophenyl)-ethyl]piperazine;
(77) 1-[3-(5-(1,2,4-triazol-4-yl)-1*H*-indol-3-yl)propyl]-4-[2-(3,5-difluorophenyl)-ethyl]piperazine;
(78) 1-[3-(5-(1,2,4-triazol-4-yl)-1*H*-indol-3-yl)propyl]-4-[2-(3-(oxazolidin-2-on-3-yl)phenyl)ethyl]piperazine;
(79) N-methyl-3-[5-(1,2,4-triazol-4-yl)-1H-indol-3-yl]pyrrolidine;
(80) N-methyl-4-[5-(1,2,4-triazol-4-yl)-1H-indol-3-yl]piperidine;
(81) N,N-dimethyl-2-[5-(1,2,4-triazol-4-yl)-1H-indol-3-yl]ethylamine;
(82) 4-(1-phenylethyl)-1-[3-(5-(1,2,4-triazol-4-yl)-1*H*-indol-3-yl)propyl]-1,2,5,6-tetrahydropyridine;
(83) 4-(α-isopropyloxy)phenylmethyl-1-[3-(5-(1,2,4-triazol-4-yl)-1*H*-indol-3-yl)propyl]-1,2,5,6-tetrahydropyridine;
(84) 4-(α-methoxy)phenylmethyl-1-[3-(5-(1,2,4-triazol-4-yl)-1*H*-indol-3-yl)propyl]-1,2,5,6-tetrahydropyridine;
(85) 4-[α-(2-methoxyethyl)oxy]phenylmethyl-1-[3-(5-(1,2,4-triazol-4-yl)-1*H*-indol-3-yl)propyl]-1,2,5,6-tetrahydropyridine;
(86) 4-benzyl-1-[3-(2,3-dihydro-5-(1,2,4-triazol-4-yl)-1*H*-indol-3-yl)propyl]piperazine;
(87) 1-[3-(2,3-dihydro-5-(1,2,4-triazol-4-yl)-1*H*-indol-3-yl)propyl]-4-(pyridin-3-ylmethyl)piperazine;
(88) 1-[2-(5-(1,2,4-triazol-4-yl)-1*H*-indol-3-yl)propyl]-4-[2-(4-fluorophenyl)-4-methylpiperazin-1-yl]piperidine;
(89) 1-[2-(5-(1,2,4-triazol-1-ylmethyl)-1*H*-indol-3-yl)ethyl]-3(*R*)-(3(*R*)-phenylmorpholin-4-ylmethyl)pyrrolidine;
(90) 1-[3-(5-(1,2,4-triazol-4-yl)-1*H*-indol-3-yl)propyl]-4-(3-oxo-2-phenylpiperazin-1-yl)methylpiperidine;
(91) 1-[3-(5-(1,2,4-triazol-4-yl)-1*H*-indol-3-yl)propyl]-4-(4-methyl-2-phenylpiperazin-1-yl)piperidine;
(92) 1-[3-(5-(1,2,4-triazol-4-yl)-1*H*-indol-3-yl)propyl]-4-(benzimidazol-2-on- 1-yl)piperidine;
(93) 1-[3-(5-(1,2,4-triazol-4-yl)-1*H*-indol-3-yl)propyl]-4-[6-(4-fluorophenyl)-4-methyl-3-oxopiperazin-1-yl]piperidine;
(94) [3-(3-(4-(2-(3,4-difluorophenyl)ethyl)piperazin-1-yl)propyl)-1*H*-indol-5-ylmethyl]oxazolidin-2-one;
(95) (S)-4-[3-(3-(4-(2-(3,4-difluorophenyl)ethyl)piperazin-1-yl)propyl)-1*H*-indol-5-ylmethyl]-3-methyloxazolidin-2-one;
(96) 1-[3-(5-(N-methylaminosulphonylmethyl)-1H-indol-3-yl)propyl]-4-[2-(4-(acetylamino)phenyl)ethyl]piperazine;
(97) 3-benzyl-7-[3-(5-(1,2,4-triazol-4-yl)-1*H*-indol-3-yl)propyl]-*cis*-3,7-diazabicyclo[3.3.0]octane;
(98) 3-(pyridin-3-yl)methyl-7-[3-(5-(1,2,4-triazol-4-yl)-1*H*-indol-3-yl)propyl]-*cis*-3,7-diazabicyclo[3.3.0]octane;
(99) 3-[2-(4-(acetylamino)phenyl)ethyl]-7-[3-(5-(1,2,4-triazol-4-yl)-1*H*-indol-3-yl)propyl]-*cis*-3,7-diazabicyclo[3.3.0]octane;
(100) 3-benzoyl-7-[3-(5-(1,2,4-triazol-4-yl)-1*H*-indol-3-yl)propyl]-*cis*-3,7-diazabicyclo[3.3.0]octane;
(101) (1*RS*,3*RS*,5*RS*)-7-benzyl-3-[5-(imidazol-1-yl)-1*H*-indol-3-ylmethyl]-2-methyl-2,7-diazabicyclo[3.3.0]octane;
(102) (1*RS*,3*RS*,5*RS*)-7-[2-(3-fluorophenyl)ethyl]-2-methyl-3-[5-(1,2,4-triazol-4-yl)-1*H*-indol-3-ylmethyl]-2,7-diazabicyclo[3.3.0]octane;
(103) (1*RS*,3*RS*,5*RS*)-7-(4-fluorobenzyl)-2-methyl-3-[5-(1,2,4-triazol-4-yl)-1*H*-indol-3-ylmethyl]-2,7-diazabicyclo[3.3.0]octane;
(104) 4-[1-(phenyl)-N,N-dimethylcarboxamidomethyl]-1-[3-(5-(1,2,4-triazol-4-yl)-1H-indol-3-yl)propyl]piperazine;
(105) 4-(2-methoxycarbonylamino-1-phenylethyl)-1-[3-(5-(1,2,4-triazol-4-yl)-1H-indol-3-yl)propyl]piperazine;
(106) 4-(2-dimethylamino-1-phenylethyl)-1-[3-(5-(1,2,4-triazol-4-yl)-1H-indol-3-yl)propyl]piperazine;
(107) 1-[3-(5-(1,2,4-triazol-4-yl)-1H-indol-3-yl)propyl]-4-(4-fluorophenyl)methylpiperazine;
(108) 4-[2-(N-methyl-N-methoxycarbonyl)amino-1-phenylethyl]-1-[3-(5-(1,2,4-triazol-4-yl)-1H-indol-3-yl)propyl]piperazine;
(109) 1-benzyl-4-[(R,S)-2-hydroxymethyl-3-(5-(1,2,4-triazol-4-yl)-1H-indol-3-yl)propyl]piperazine;
(110) 1-[3-(5-(1,2,4-triazol-4-yl)-1H-indol-3-yl)propyl]-4-[2-(1H-tetrazol-5-yl)phenyl]methylpiperazine;
(111) 1-[3-(5-(1,2,4-triazol-4-yl)-1H-indol-3-yl)propyl]-4-(2-phenylethyl)piperazine;
(112) 4-benzyl-1-[3-(5-(1,2,4-triazol-4-yl)-1H-indol-3-yl)propyl]-piperazine;
(113) 4-[2-(2-methyltetrazol-5-yl)phenyl]methyl-1-[3-(5-(1,2,4-triazol-4-yl)-1H-indol-3-yl)propyl]piperazine;
(114) 4-[2-(1-methyltetrazol-5-yl)phenyl]methyl-1-[3-(5-(1,2,4-triazol-4-yl)-1H-indol-3-yl)propyl]piperazine;
(115) 4-[2-(N-methylcarboxamido)phenyl]methyl-1-[3-(5-(1,2,4-triazol-4-yl)-1H-indol-3-yl)propyl]piperazine;
(116) 4-[2-(N,N-dimethylaminomethyl)phenyl]methyl-1-[3-(5-(1,2,4-triazol-4-yl)-1H-indol-3-yl)propyl]piperazine;
(117) 4-(but-3-enyl)-1-[3-(5-(1,2,4-triazol-4-yl)-1H-indol-3-yl)propyl]piperazine;
(118) 4-(3-methylbut-2-enyl)-1-[3-(5-(1,2,4-triazol-4-yl)-1H-indoi-3-yl)propyl]piperazine;
(119) 4-(prop-2-enyl)-1-[3-(5-(1,2,4-triazol-4-yl)-1H-indol-3-yl)propyl]piperazine;
(120) 4-(prop-2-ynyl)-1-[3-(5-(1,2,4-triazol-4-yl)-1H-indol-3-yl)propyl]piperazine;
(121) 4-[(R,S)-1-(phenyl)carboxamidomethyl]-1-[3-(5-(1,2,4-triazol-4-yl)-1H-indol-3-yl)propyl]piperazine;
(122) 4-[1-(phenyl)-N-methylcarboxamidomethyl]-1-[3-(5-(1,2,4-triazol-4-yl)-1H-indol-3-yl)propyl]piperazine;
(123) 1-[3-(5-(1,2,4-triazol-1-ylmethyl)-1H-indol-3-yl)propyl]-4-[2-(4-(acetylamino)phenyl)ethyl]piperazine;
(124) 1-[3-(5-(1,2,4-triazol-4-yl)-1H-indol-3-yl)propyl]-4-[2-(3-(acetylamino)phenyl)ethyl]piperazine;
(125) 1-[3-(5-(1,2,4-triazol-4-yl)-1H-indol-3-yl)propyl]-4-[4-(aminosulphonyl)phenyl]methylpiperazine;
(126) 1 -[3-(5-(1,2,4-triazol-4-yl)- 1H-indol-3-yl)propyl]-4-(furan-3-yl)methylpiperazine;
(127) 1-[3-(5-(1,2,4-triazol-4-yl)-1H-indol-3-yl)propyl]-4-(furan-2-yl)methylpiperazine;
(128) 1-[3-(5-(1,2,4-triazol-4-yl)-1H-indol-3-yl)propyl]-4-(thien-2-yl)methylpiperazine;
(129) 1 -benzyl-4-[(R,S)-2-hydroxy-3-(5-(1,2,4-triazol-4-yl)- 1H-indol-3-yl)propyl]piperazine;
(130) 1-[2-(4-(acetylamino)phenyl)ethyl]-4-[(R,S)-2-hydroxy-3-(5-(1,2,4-triazol-4-yl)-1H-indol-3-yl)propyl]piperazine;
(131) 1-[3-(5-(1,2,4-triazol-4-yl)-1H-indol-3-yl)propyl]-4-[2-(4-(aminocarbonylamino)phenyl)ethyl]piperazine;
(132) 1-[3-(5-(1,2,4-triazol-4-yl)-1H-indol-3-yl)propyl]-4-(4-cyanophenyl)methylpiperazine;
(133) 1-[3-(5-(1,2,4-triazol-4-yl)-1H-indol-3-yl)propyl]-4-[2-(4-cyanophenyl)ethyl]piperazine;
(134) 1-[3-(5-(1,2,4-triazol-4-yl)-1H-indol-3-yl)propyl]4-[2-(4-(1,2,4-triazol-4-yl)phenyl)ethyl)piperazine;
(135) 1-[3-(5-(1,2,4-triazol-1-yl)-1H-indol-3-yl)propyl]-4-[2-(4-(acetylamino)phenyl)ethyl]piperazine;
(136) 1-[3-(5-(1,2,4-triazol-1-yl)-1H-indol-3-yl)propyl]-4-benzylpiperazine;
(137) 1-[3-(5-(1,2,4-triazol-1-ylmethyl)-1H-indol-3-yl)propyl]-4-benzylpiperazine;
(138) 4-(4-acetylaminophenyl)methyl-1-[3-(5-(1,2,4-triazol-4-yl)-1 H-indol-3-yl)propyl]piperidine;
(139) 4-benzyl-1-[3-(5-(1,2,4-triazol-4-yl)-1H-indol-3-yl)propyl]-1,2,5,6-tetrahydropyridine;
(140) 1-[3-(5-(1,2,4-triazol-4-yl)-1H-indol-3-yl)propyl]-4-(2-aminopyridin-5-yl)methylpiperazine;
(141) 1-[3-(5-(1,2,4-triazol-4-yl)-1H-indol-3-yl)propyl]-4-(4-aminophenyl)methylpiperazine;
(142) 1-[4-(5-(1,2,4-triazol-4-yl)-1H-indol-3-yl)butyl]-4-benzylpiperazine;
(143) 1-[4-(5-(1,2,4-triazol-4-yl)-1H-indol-3-yl)butyl]-4-(pyridin-2-yl)methylpiperazine;
(144) 1-[4-(5-(1,2,4-triazol-4-yl)-1H-indol-3-yl)butyl]-4-(pyridin-3-yl)methylpiperazine;
(145) 1-[3-(5-(1,2,4-triazol-4-yl)-1H-indol-3-yl)propyl]-4-[2-(4-aminophenyl)ethyl]piperazine;
(146) 1-[3-(5-(1,2,4-triazol-4-yl)-1H-indol-3-yI)propyl]-4-[2-(4-(acetylamino)phenyl)ethyl]piperazine;
(147) 1-[3-(5-(1,2,4-triazol-4-yl)-1H-indol-3-yl)propyl]-4-(imidazol-2-yl)methylpiperazine;
(148) 1-[3-(5-(1,2,4-triazol-4-yl)-1H-indol-3-yl)propyl]-4-[3-(acetylamino)phenyl]methylpiperazine;
(149) 1-[3-(5-(1,2,4-triazol-4-yl)-1H-indol-3-yl)propyl]-4-[4-(acetylamino)phenyl]methylpiperazine;
(150) 1-[4-(5-(1,2,4-triazol-4-yl)-1H-indol-3-yl)butyl]-4-[4-(acetylamino)phenyl]methylpiperazine;
(151) 1-[3-(5-(1,2,4-triazol-4-yl)-1H-indol-3-yl)propyl]-4-(2-methoxyphenyl)methylpiperazine;
(152) 1-[3-(5-(1,2,4-triazol-4-yl)-1H-indol-3-yl)propyl]-4-benzylpiperazine;
(153) 1-[3-(5-(1,2,4-triazol-4-yl)-1H-indol-3-yl)propyl]-4-(pyridin-3-yl)methylpiperazine;
(154) 1-[3-(5-(1,2,4-triazol-4-yl)-1H-indol-3-yl)propyl]-4-(pyridin-2-yl)methylpiperazine;
(155) 1-[3-(5-(1,2,4-triazol-4-yl)-1H-indol-3-yl)propyl]-4-(pyridin-4-yl)methylpiperazine;
(156) (3*R*)-3-benzyloxymethyl-1-[2-(5-(1,2,4-triazol-4-yl)-1*H*-indol-3-yl)ethyl]pyrrolidine;
(157) (3*S*)-3-(*N*-benzyl-*N*-methyl)aminomethyl-1-[2-(5-(1,2,4-triazol-4-yl)-1*H*-indol-3-yl)ethyl]pyrrolidine;
(158) (2*S*)-2-(*N*-benzyl-*N*-methyl)aminomethyl-1-[2-(5-(1,2,4-triazol-4-yl)-1*H*-indol-3-yl)ethyl]pyrrolidine;
(159) (3*S*)-3-(*N*-benzyl)aminomethyl-1-[2-(5-(1,2,4-triazol-4-yl)-1*H*-indol-3-yl)ethyl]pyrrolidine;
(160) 4-(4-acetylaminophenyl)methylamino-1-[3-(5-(1,2,4-triazol-4-yl)-1*H*-indol-3-yl)propyl]piperidine;
(161) 1-[3-(5-(imidazol-1-yl)-1*H*-indol-3-yl)propyl]-4-[(*R*)-α-(methoxymethyl)benzylamino]piperidine;
(162) 1-[3-(5-(1,2,4-triazol-1-ylmethyl)-1*H*-indol-3-yl)propyl]-4-[(*R*)-1-(4-fluorophenyl)-2-methoxyethylamino]piperidine;
(163) 1-[3-(5-(1,2,4-triazol-1-ylmethyl)-1*H*-indol-3-yl)propyl]-4-[*N*-(4-fluorobenzyl)-*N*-methylamino]piperidine;
(164) 1-[3-(5-(1,2,4-triazol-4-yl)-1*H*-indol-3-yl)propyl]-4-(2-phenylpiperidin-1-yl)piperidine;
(165) 1-[3-(5-(1,2,4-triazol-4-yl)-1*H*-indol-3-yl)propyl]-4-[(*R*)-1-(4-fluorophenyl)-2-methoxyethylamino]piperidine;
(166) 1-[3-(5-(1,2,4-triazol-4-yl)-1*H*-indol-3-yl)propyl]-4-(indan-1-ylaminomethyl)piperidine;
(167) 1-[3-(5-(1,2,4-triazol-4-yl)-1*H*-indol-3-yl)propyl]-4-[*N*-(*R*)-α-(hydroxymethyl)benzyl-*N*-methylaminomethyl]piperidine;
(168) (3*R*)-3-(benzylthio)methyl-1-[2-(5-(1,2,4-triazol-4-yl)-1*H*-indol-3-yl)ethyl]pyrrolidine;
(169) (±)-1-[3-(5-(1,2,4-triazol-4-yl)-1*H*-indol-3-yl)propyl]-4-(1-benzylamino-2-hydroxyethyl)piperidine;
(170) 1-[3-(5-(1,2,4-triazol-1-yl)-1*H*-indol-3-yl)propyl]-4-[(R)-α-(hydroxymethyl)benzylamino]piperidine;
(171) 1-[3-(5-(imidazol-1-yl)-1*H*-indol-3-yl)propyl]-4-[(*R*)-α-(methyl)benzylamino]piperidine;
(172) 1-[3-(5-(imidazol-1-yl)-1*H*-indol-3-yl)propyl]-4-[(*R*)-α-(hydroxymethyl)benzylamino]piperidine;
(173) 1-[3-(5-(1,2,4-triazol-1-ylmethyl)-1*H*-indol-3-yl)propyl]-4-[(*R*)-α-(hydroxymethyl)benzylamino]piperidine;
(174) 1-[3-(5-(1,2,4-triazol-4-yl)-1*H*-indol-3-yl)propyl]-4-[(*R*)-α-(methoxymethyl)benzylamino]piperidine;
(175) 1-[3-(5-(1,2,4-triazol-4-yl)-1*H*-indol-3-yl)propyl]-4-[*N*-(*R*)-α-(methoxymethyl)benzyl-*N*-methylamino]piperidine;
(176) (3R)-3-benzyloxy-1-[2-(5-(1,2,4-triazol-4-yl)-1*H*-indol-3-yl)ethyl]pyrrolidine;
(177) (3R)-3-(4-methoxyphenyl)methoxy-1-[2-(5-(1,2,4-triazol-4-yl)-1*H*-indol-3-yl)ethyl]pyrrolidine;
(178) (3*R*)-3-(pyridin-3-yl)methoxy-1-[2-(5-(1,2,4-triazol-4-yl)-1*H*-indol-3-yl)ethyl]pyrrolidine;
(179) (3*R*)-3-benzyloxymethyl-1-[2-(5-(1,2,4-triazol-4-yl)-1*H*-indol-3-yl)ethyl]pyrrolidine;
(180) (3*S*)-3-(*N*-benzyl-*N*-methyl)aminomethyl-1-[2-(5-(1,2,4-triazol-4-yl)-1*H*-indol-3-yl)ethyl]pyrrolidine;
(181) (2*S*)-2-(*N*-benzyl-*N*-methyl)aminomethyl-1-[2-(5-(1,2,4-triazol-4-yl)-1*H*-indol-3-yl)ethyl]pyrrolidine;
(182) (35)-3-(*N*-benzyl)aminomethyl-1-[2-(5-(1,2,4-triazol-4-yl)-1*H*-indol-3-yl)ethyl]pyrrolidine;
(183) 4-(4-acetylaminophenyl)methylamino-1-[3-(5-(1,2,4-triazol-4-yl)-1*H*-indol-3-yI)propyl]piperidine;
(184) 4-benzylamino-1-[3-(5-(1,2,4-triazol-4-yl)-1*H*-indol-3-yl)propyl]piperidine;
(185) 4-(N-benzyl-N-methyl)amino-1-[3-(5-(1,2,4-triazol-4-yl)-1*H*-indol-3-yl)propyl]piperidine;
(186) 4-(*N*-benzyl-*N*-methyl)aminomethyl-1-[3-(5-(1,2,4-triazol-4-yl)-1*H*-indol-3-yl)propyl]piperidine;
(187) 1-[3-(5-(1,2,4-triazol-4-yl)-1*H*-indol-3-yl)propyl]-4-[α-(methyl)benzylamino]piperidine;
(188) 1-[3-(5-(1,2,4-triazol-4-yl)-1*H*-indol-3-yl)propyl]-4-[α-(hydroxymethyl)benzylamino]piperidine;
(189) 1-[3-(5-(1,2,4-triazol-4-yl)-1*H*-indol-3-yl)propyl]-4-[(1-hydroxymethyl-2-phenyl)ethylamino]piperidine;
(190) 4-(*N*-benzyl)aminomethyl-1-[3-(5-(1,2,4-triazol-4-yl)-1*H*-indol-3-yl)propyl]piperidine;
(191) 1-[3-(5-(1,2,4-triazol-4-yl)-1*H*-indol-3-yl)propyl]-4-[(2-hydroxy-1-methyl-2-phenyl)ethylamino]piperidine;
(192) 1-[3-(5-(1,2,4-triazol-4-yl)-1*H*-indol-3-yl)propyl]-4-[2-(4-acetylaminophenyl)ethylamino]piperidine;
(193) 1-[3-(5-(1,2,4-triazol-4-yl)-1*H*-indol-3-yl)propyl]-4-[α-(methyl)benzylamino]methylpiperidine;
(194) 1-[3-(5-(1,2,4-triazol-4-yl)-1*H*-indol-3-yl)propyl]-4-[1-(4-acetylaminophenyl)ethylamino)methylpiperidine;
(195) 1-[3-(5-(1,2,4-triazol-4-yl)-1*H*-indol-3-yl)propyl]-4-[*N*-[α-(hydroxymethyl)benzyl]-*N*-methylamino]piperidine;
(196) 1-[3-(5-(1,2,4-triazol-4-yl)-1*H*-indol-3-yl)propyl]-4-[*N*-(2-(4-acetylaminophenyl)ethyl)-*N*-methylamino]piperidine;
(197) 1-[3-(5-(1,2,4-triazol-4-yl)-1*H*-indol-3-yl)propyl]-4-[*N*-(4-acetylaminobenzyl)-N-methylamino]methylpiperidine;
(198) 1-[3-(5-(1,2,4-triazol-4-yl)-1*H*-indol-3-yl)propyl]-4-[*N*-(thien-2-yl)methyl-*N*-methylamino]piperidine;
(199) 1-[3-(5-(1,2,4-triazol-4-yl)-1*H*-indol-3-yl)propyl]-4-[(*R*)-α-(hydroxymethyl)benzylamino]methylpiperidine;
(200) 3-(4-acetylaminobenzyl)aminomethyl-1-[2-(5-(1,2,4-triazol-4-yl)-1*H*-indol-3-yl)ethyl]pyrrolidine;
(201) (3*R*)-3-(*N*-benzyl)aminomethyl-1-[2-(5-(1,2,4-triazol-4-yl)-1*H*-indol-3-yl)ethyl]pyrrolidine;
(202) (3*S*)-3-(pyridin-4-ylmethyl)aminomethyl-1-[2-(5-(1,2,4-triazol-4-yl)-1*H*-indol-3-yl)ethyl]pyrrolidine;
(203) 3-(*N*-benzyl)aminomethyl-1-[2-(5-(1,2,4-triazol-4-yl)-1*H*-indol-3-yl)ethyl]azetidine;
(204) 4-benzyl-4-hydroxy-1-[3-(5-(1,2,4-triazol-4-yl)-1*H*-indol-3-yl)propyl]piperidine;
(205) 3-(*N*-benzyl)aminomethyl-1-[3-(5-(1,2,4-triazol-4-yl)-1*H*-indol-3-yl)propyl]azetidine;
(206) 4-(*N*-benzyl)aminomethyl-4-hydroxy-1-[3-(5-(1,2,4-triazol-4-yl)-1*H*-indol-3-yl)propyl]piperidine;
(207) 4-(*N*-benzyl-*N*-methyl)aminomethyl-4-hydroxy-1-[3-(5-(1,2,4-triazol-4-yl)-1*H*-indol-3-yl)propyl]piperidine;
(208) 3-(*N*-benzyl-*N*-methyl)aminomethyl-1-[2-(5-(1,2,4-triazol-4-yl)-1*H*-indol-3-yl)ethyl]azetidine;
(209) (35)-3-[*N*-α-(methyl)benzyl]aminomethyl-1-[2-(5-(1,2,4-triazol-4-yl)-1*H*-indol-3-yl)ethyl]pyrrolidine;
(210) (3*S*)-3-[*N*-(furan-3-ylmethyl)amino]methyl-1-[2-(5-(1,2,4-triazol-4-yl)-1*H*-indol-3-yl)ethyl]pyrrolidine;
(211) (3*S*)-3-[*N*-(furan-2-ylmethyl)amino]methyl-1-[2-(5-(1,2,4-triazol-4-yl)-1*H*-indol-3-yl)ethyl]pyrrolidine;
(212) (3*S*)-3-[*N*-α-(hydroxymethyl)benzyl]aminomethyl-1-[2-(5-(1,2,4-triazol-4-yl)-1*H*-indol-3-yl)ethyl]pyrrolidine;
(213) (3*S*)-3-[*N*-benzyl-*N*-(2-hydroxy)ethyl]aminomethyl-1-[2-(5-(1,2,4-triazol-4-yl)-1*H*-indol-3-yl)ethyl]pyrrolidine;
(214) (35)-3-[*N*-(2-phenylethyl)amino]methyl-1-[2-(5-(1,2,4-triazol-4-yl)-1*H*-indol-3-yl)ethyl]pyrrolidine;
(215) (3*S*)-3-[*N*-(2-phenylethyl)-*N*-methylamino]methyl-1-[2-(5-(1,2,4-triazol-4-yl)-1*H*-indol-3-yl)ethyl]pyrrolidine;
(216) (3*S*)-3-(*N*-α-dimethylbenzyl)aminomethyl-1-[2-(5-(1,2,4-triazol-4-yl)*-*1*H*-indol-3-yl)ethyl]pyrrolidine;
(217) (3*S*)-3-[*N*-(*S*)-α-methylbenzyl]aminomethyl-1-[2-(5-(1,2,4-triazol-1-yl)-1*H*-indol-3-yl)ethyl]pyrrolidine;
(218) (3*S*)-3-[*N*-(*R*)-α-(hydroxymethyl)benzyl]aminomethyl-1-[2-(5-(1,2,4-triazol-1-yl)-1*H*-indol-3-yl)ethyl]pyrrolidine;
(219) (3*S*)-3-(N-benzyl)aminomethyl-1-[2-(5-(1,2,4-triazol-1-ylmethyl)-1*H*-indol-3-yl)ethyl]pyrrolidine;
(220) (3*S*)-3-[*N*-(*S*)-α-methylbenzyl]aminomethyl-1-[2-(5-(1,2,4-triazol-1-ylmethyl)-1*H*-indol-3-yl)ethyl]pyrrolidine;
(221) (3*S*)-3-[*N*-(*R*)-α-(hydroxymethyl)benzyl]aminomethyl-1-[2-(5-(1,2,4-triazol-1-ylmethyl)-1*H*-indol-3-yl)ethyl]-pyrrolidine;
(222) (3*S*)-3-(*N*-benzyl-*N*-methyl)aminomethyl-1-[2-(5-(imidazol-1-yl)-1*H*-indol-3-yl)ethyl]pyrrolidine;
(223) (3*S*)-3-(*N*-benzyl-*N*-methyl)aminomethyl-1-[2-(5-(1,2,4-triazol-1-ylmethyl)-1*H*-indol-3-yl)ethyl]pyrrolidine;
(224) (3*R*)-3-[*N*-methyl-*N*-(*S*)-α-methylbenzyl]aminomethyl-1-[2-(5-(1,2,4-triazol-1-ylmethyl)-1*H*-indol-3-yl)ethyl]-pyrrolidine;
(225) (3*R*)-3-[*N*-methyl-*N*-(*R*)-α-hydroxymethylbenzyl]aminomethyl-1-[2-(5-(1,2,4-triazol-1-ylmethyl)-1*H*-indol-3-yl)ethyl]pyrrolidine;
(226) (3*R*)-3-[*N*-methyl-*N*-(*S*)-α-methylcyclohexylmethyl]aminomethyl-1-[2-(5-(1,2,4-triazol-1-ylmethyl)-1*H*-indol-3-yl)ethyl]pyrrolidine;
(227) (3*R*)-3-[3-(*R*)-hydroxy-2-(*R*)-phenylpiperidin-1-yl]methyl-1-[2-(5-(1,2,4-triazol-1-ylmethyl)-1*H*-indol-3-yl)ethyl]-pyrrolidine;
(228) (3*R*)-3-[3-(*R*)-hydroxy-2-(*R*)-phenylpiperidin-1-yl]methyl-1-[2-(5-(1,2,4-triazol-1-yl)-1*H*-indol-3-yl)ethyl)pyrrolidine;
(229) 4-hydroxy-4-(phenylsulfinyl)methyl-1-[3-(5-(1,2,4-triazol-4-yl)-1*H*-indol-3-yl)propyl]piperidine;
(230) (3*R*)-3-[2-(*R*,*S*)-phenylpipenidin-1-yl]methyl-1-[2-(5-(1,2,4-triazol-1-ylmethyl)-1*H*-indol-3-yl)ethyl]pyrrolidine;
(231) 4-(3,3-dimethylpiperidin-1-yl)methyl-4-hydroxy-1-[3-(5-(1,2,4-triazol-4-yl)-1*H*-indol-3-yl)propyl]piperidine;
(232) 4-hydroxy-4-(1,2,3,4-tetrahydroisoquinolin-2-yl)methyl-1-[3-(5-(1,2,4-triazol-4-yl)-1*H*-indol-3-yl)propyl]piperidine;
(233) 4-hydroxy-4-(*N*-isobutyl-*N*-methyl)aminomethyl-1-[3-(5-(1,2,4-triazol-4-yl)-1*H*-indol-3-yl)propyl]piperidine;
(234) 4-[*N*-benzyl-*N*-(2-hydroxyethyl)amino]methyl-4-hydroxy-1-[3-(5-(1,2,4-triazol-4-yl)-1*H*-indol-3-yl)propyl]piperidine;
(235) 4-[*N*-(2,2-dimethylpropyl)-*N*-methylamino]methyl-4-hydroxy-1-[3-(5-(1,2,4-triazol-4-yl)-1*H*-indol-3-yl)propyl]-piperidine;
(236) 4-[*N*-(*R*)-α-hydroxymethylbenzyl-*N*-methylamino]methyl-4-hydroxy-1-[3-(5-(1,2,4-triazol-4-yl)-1*H*-indol-3-yl)propyl]-piperidine;
(237) 4-hydroxy-4-(2-pyridylmethyl)aminomethyl-1-[3-(5-(1,2,4-triazol-4-yl)-1*H*-indol-3-yl)propyljpiperidine;
(238) 4-hydroxy-4-(2-methylphenylmethyl)aminomethyl-1-[3-(5-(1,2,4-triazol-4-yl)-1*H*-indol-3-yl)propyl]piperidine;
(239) 4-hydroxy-4-[*N*-(2-methylphenylmethyl)-*N*-methylamino]-methyl-1-[3-(5-(1,2,4-triazol-4-yl)-1*H*-indol-3-yl)propyl]-piperidine;
(240) 3-(benzylamino)methyl-3-hydroxy-1-[3-(5-(1,2,4-triazol-4-yl)-1*H*-indol-3-yl)propyl]pyrrolidine;
(241) 3-(benzylamino)methyl-3-hydroxy-1-[2-(5-(1,2,4-triazol-4-yl)-1*H*-indol-3-yl)ethyl]pyrrolidine;
(242) 1-[3-(5-(1,2,4-triazol-4-yl)-1*H*-indol-3-yl)propyl]-4-[(*R*)-α-(carbamoyl-oxymethyl)benzylamino]piperidine;
(243) 1-[3-(5-(1,2,4-triazol-4-yl)-1*H*-indol-3-yl)propyl]-4-[(1*R*,2*S*)-2-hydroxy-1-phenylpropylamino]piperidine;
(244) 1-[3-(5-(1,2,4-triazol-4-yl)-1*H*-indol-3-yl)propyl]-4-[(1*R*,2*R*)-2-hydroxy-1-phenylpropylamino]piperidine;
(245) 1-[3-(5-(1,2,4-triazol-4-yl)-1*H*-indol-3-yl)propyl]-4-[(*R*,*S*)-1-hydroxy-2-phenylprop-2-ylamino]piperidine;
(246) 1-[3-(5-(1,2,4-triazol-4-yl)-1*H*-indol-3-yl)propyl] -4-[(*R*)-2-hydroxy-1-(4-fluorophenyl)ethylamino]piperidine;
(247) 1-[3-(5-(1,2,4-triazol-4-yl)-1*H*-indol-3-yl)propyl]-4-[(1*R*,2*R*)-2-hydroxyindan-1-ylamino)piperidine;
(248) 1-[3-(5-(1,2,4-triazol-4-yl)-1*H*-indol-3-yl)propyl)-4-[(*R*,*S*)-indan-1-ylamino]piperidine;
(249) 1-[3-(5-(1,2,4-triazol-4-yl)-1*H*-indol-3-yl)propyl]-4-[(*R*,*S*)-1-(4-fluorophenyl)ethylamino]piperidine;
(250) 1-[3-(5-(1,2,4-triazol-4-yl)-1*H*-indol-3-yl)propyl]-4-[(*R*)-1-phenylprop-2-ylamino]piperidine;
(251) 1-[3-(5-(1,2,4-triazol-4-yl)-1*H*-indol-3-yl)propyl]-4-[*N*-(thien-3-ylmethyl)-*N*-methylamino]piperidine;
(252) 1-[3-(5-(1,2,4-triazol-4-yl)-1*H*-indol-3-yl)propyl]-4-[*N*-(furan-3-ylmethyl)-N-methylamino]piperidine;
(253) 1-[3-(5-(1,2,4-triazol-4-yl)-1*H*-indol-3-yl)propyl]-4-(furan-3-ylmethyl)aminopiperidine;
(254) 1-[3-(5-(1,2,4-triazol-4-yl)-1*H*-indol-3-yl)propyl]-4-[*N,N*-di-(furan-3-ylmethyl)amino]piperidine;
(255) 1-[3-(5-(1,2,4-triazol-4-yl)-1*H*-indol-3-yl)propyl]-4-[*N*-(3,3-dimethylallyl)-*N*-methylamino]piperidine;
(256) 1-[3-(5-(1,2,4-triazol-4-yl)-1*H*-indol-3-yl)propyl]-4-(*N*-allyl-*N*-methylamino)piperidine;
(257) N,N-Dimethyl-2-[5-(1,2,4-triazol-1-ylmethyl)-1H-indol-3-yl]ethylamine;
(258) N,N-Dimethyl-2-[5-(1,3-imidazol-1-ylmethyl)-1H-indol-3-yl]ethylamine;
(259) N,N-Dimethyl-2-[5-(5-methyl-1,2,3,4-triazol-1-ylmethyl)-1H-indol-3-yl]ethylamine;
(260) N,N-Dimethyl-2-[5-(1,3,4-triazol-1-ylmethyl)-1H-indol-3-yl]ethylamine;
(261) N,N-Dimethyl-2-[5-(1,3,4-triazol-1-yl)-1H-indol-3-yl]ethyl-amine;
(262) N,N-Diethyl-2-[5-(1,2,4-triazol-1-ylmethyl)-1H-indol-3-yl]ethylamine;
(263) N,N-Diethyl-2-[5-(1,3-imidazol-1-ylmethyl)-1H-indol-3-yl]ethylamine;
(264) N,N-Diethyl-2-[5-(5-methyl-1,2,3,4-tetrazol-1-ylmethyl)-1H-indol-3-yl]ethylamine;
(265) N,N-Diethyl-2-[5-(1,3,4-triazol-1-ylmethyl)-1H-indol-3-yl]ethylamine;
(266) N,N-Diethyl-2-[5-(1,3,4-triazol-1-yl)-1H-indol-3-yl]ethylamine;
(267) N,N-Dimethyl-[5-(1,2,4-triazol-1-ylmethyl)-1H-indol-3-yl]methylamine;
(268) N,N-Dimethyl-[5-(1,3-imidazol-1-ylmethyl)-1H-indol-3-yl]methylamine;
(269) N,N-Dimethyl-[5-(5-methyl-1,2,3,4-tetrazol-1-ylmethyl)-1 H-indol-3-yl]methylamine;
(270) N,N-Dimethyl-[5-(1,3,4-triazol-1-ylmethyl)-1H-indol-3-yl]methylamine;
(271) N,N-Dimethyl-[5-(1,3,4-triazol-1-yl)-1H-indol-3-yl]methylamine;
(272) N,N-Diethyl-3-[5-(1,2,4-triazol-1-ylmethyl)-1H-indol-3-yl]propylamine;
(273) N,N-Dimethyl-3-[5-(1,3-imidazol-1-yl)-1H-indol-3-yl]propylamine;
(274) N,N-Diethyl-3-[5-(5-methyl-1,2,3,4-tetrazol-1-ylmethyl)-1 H-indol-3-yl]propylamine;
(275) N,N-Dimethyl-3-[5-(1,3,4-triazol-1-ylmethyl)-1H-indol-3-yl]propyl-amine;
(276) N,N-Diethyl-3-[5-(1,3,4-triazol-1-yl)-1H-indol-3-yl]propylamine;
(277) N,N-Dimethyl-4-[5-(3-methyl-1,2,4,5-tetrazol-1-ylmethyl)-1H-indol-3-yl]butylamine;
(278) N,N-Dimethyl-4-[5-(2-ethyl-1,3-ethyl-imidazol-1-ylmethyl)-1H-indol-3-yl]butylamine;
(279) N,N-Dimethyl-4-[5-(5-ethyl-1,2,3,4-triazol-1-ylmethyl)-1H-indol-3-yl]butylamine;
(280) N,N-Dimethyl-4-[5-(2-methyl-1,3,4-triazol-1-ylmethyl)1H-indol-3-yl]butylamine;
(281) N,N-Dimethyl-4-[5-(2-ethyl-1,3,4-triazol-1-yl)-1H-indol-3-yl]butylamine;
(282) 2-[5-(1,2,4-triazol-1-ylmethyl)-1H-indol-3-yl]ethylalcohol;
(283) 2-[5-(1,3-imidazol-1-ylmethyl)-1H-indol-3-yl]ethylalcohol;
(284) 2-[5-(5-methyl-1,2,3,4-tetrazol-1-ylmethyl)-1H-indol-3-yl]ethylalcohol;
(285) 2-[5-(1,3,4-triazol-1-ylmethyl)-1H-indol-3-yl]ethylalcohol;
(286) 2-[5-(1,3,4-triazol-1-yl)-1H-indol-3-yl]ethylalcohol;
(287) [5-(1,2,4-triazol-1-ylmethyl)-1H-indol-3-yl]-methylalcohol;
(288) 3-[5-(1,3-imidazol-1-ylmethyl)-1H-indol-3-yl]propylalcohol;
(289) 4-[5-(5-methyl-1,2,3,4-tetrazol-1-ylmethyl)-1H-indol-3-yl]butylalcohol;
(290) 2-[5-(2-methyl-1,3,4-triazol-1-ylmethyl)-1H-indol-3-yl]ethylalcohol; and
(291) 2-[5-(5-methyl-1,3,4-triazol-1-yl)-1H-indol-3-yl]ethylalcohol.

Further 2-unsubstituted indoles which may be made according to the process of the present invention include: 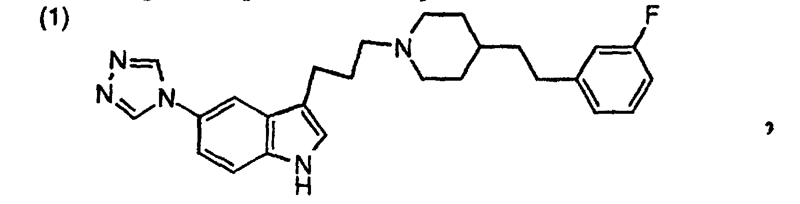 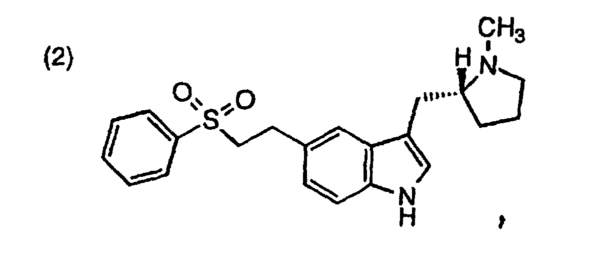 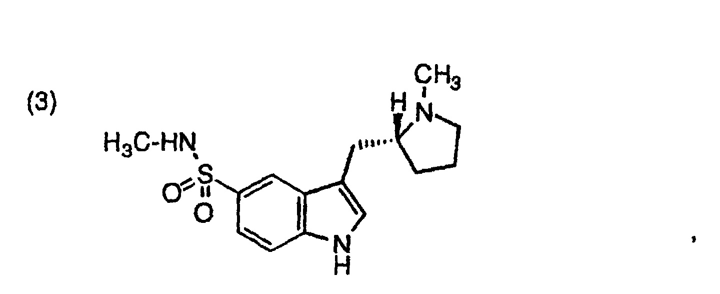 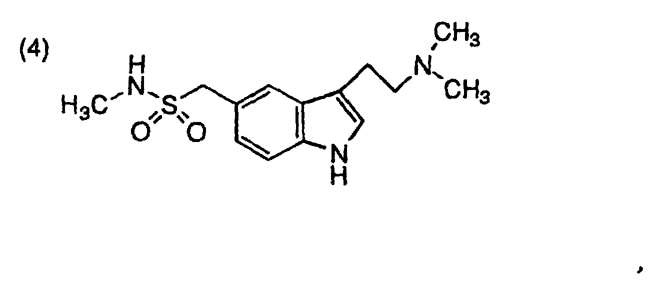 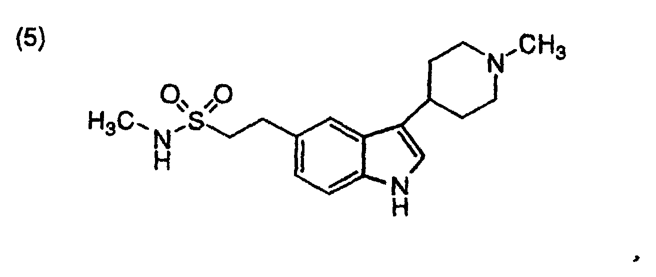 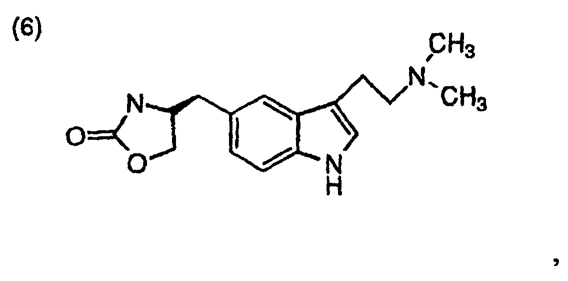 and

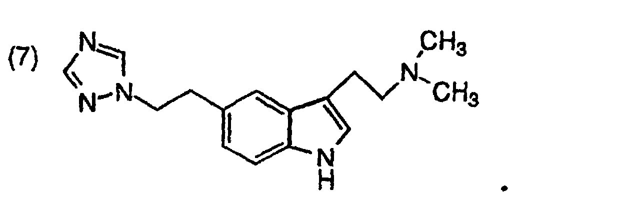

Preferred compounds that may be prepared according to the process of the present invention include: 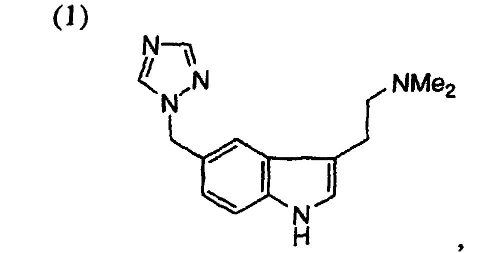 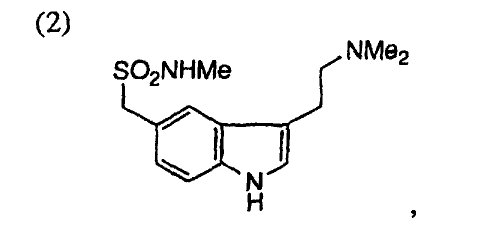 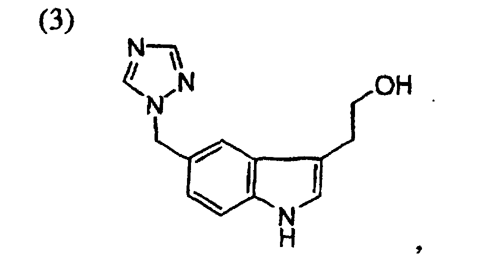 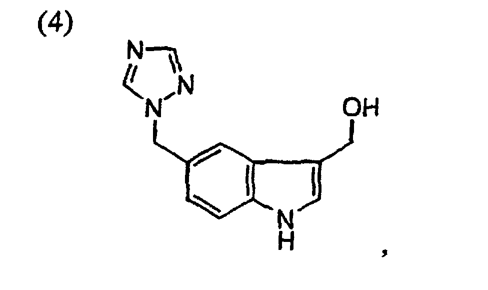 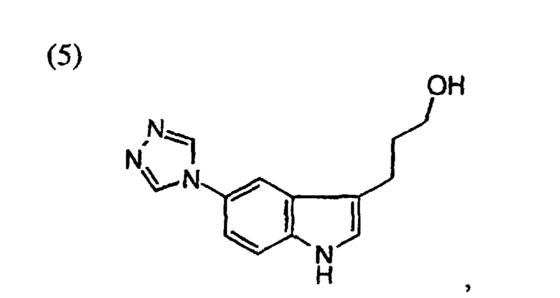 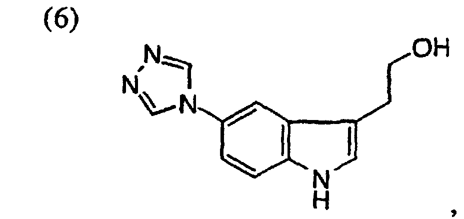 and

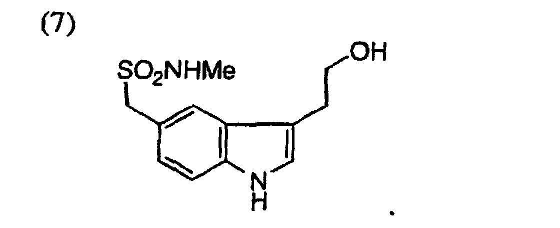

Still further, the present invention is also directed to the novel intermediates of structural formulae (V) and (VI). 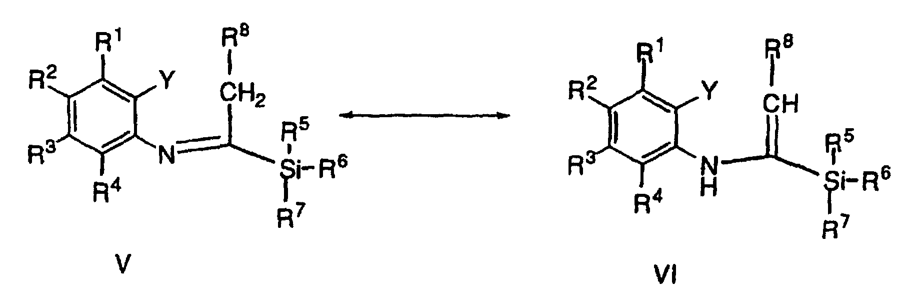 wherein Y, R¹, R², R³, R⁴, R⁵, R⁶, R⁷, and R⁸ are as defined above.

The acyl silanes employed in the present invention may be generally prepared according to the scheme below:

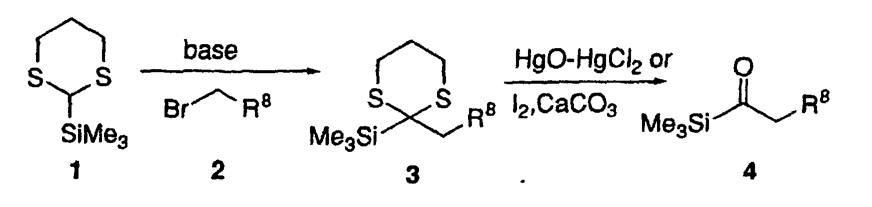

To TMS dithiane **1** in dry, polar aprotic solvent, such as THF, diethylether, t-butylmethylether, dioxane, diethoxymethane, is added an equivalent of a base such as lithium diisopropyl amide, NaH, Grignard, or an alkyl lithium, such as n-butyl lithium dropwise. Preferably, this alkylation is conducted at reduced temperatures, most preferably at -78 °C. Following the addition, the mixture is preferably warmed to -20 °C and aged at -20 °C for 0.5 h. Following aging, the reaction is preferably cooled to -78 °C. Br-CH₂-R⁸ **2**, preferably dissolved in a small volume of the solvent, is added dropwise. The mixture is warmed, preferably to room temperature, and aged, preferably for about 12 hours. The mixture is partitioned between a lipophilic solvent, such as heptane, and water. The heptane layer is separated and concentrated in vacuum to give the dithiane **3** as a pale yellow oil. This material may be directly used in the next step.

A mixture of the dithiane **3** , mercuric oxide and mercuric chloride in acetonitrile-H₂O,( preferably an 80:20 ratio) in an aprotic solvent such as ethyl acetate, isopropyl acetate, methylene chloride, acetonitrile, toluene and the like, is aged at room temperature, preferably for about 30 minutes. Alternatively, iodine in calcium carbonate may be employed. The resulting solid is filtered and washed, preferably with an aprotic solvent such as ethyl acetate, isopropyl acetate, methylene chloride, acetonitrile, toluene and the like. The filtrate and wash are combined and concentrated to an oil. This material may be chromatographed over silica gel to give the acyl silane **4** as a pale yellow oil.

Additional syntheses of acyl silanes which may be used to prepare starting materials for the compounds of the present invention are described in the following references:
(1) Ricci et al., Synthesis 1989, pp. 647-660.
(2) Page et al., Chem. Soc. Rev. 1990, vol. 19, pp. 147-195.
(3) Cirillo et al., Org. Prep. Proc. Int. 1992, vol. 24, pp. 555-582.
(4) Plantier-Royon and Portella, Tetrahedron Letters 1996, vol. 37 (34), pp. 6113-6114.

The following examples are not intended to be limitations on the scope of the instant invention in any way, and they should not be so construed. Furthermore, the compounds described in the following examples are not to be construed as forming the only genus that is considered as the invention, and any combination of the compounds or their moieties may itself form a genus. Those skilled in the art will readily understand that known variations of the conditions and processes of the following preparative procedures can be used to prepare these compounds.

### EXAMPLES

### EXAMPLE 1

### Preparation of 2-trimethylsilyl indole

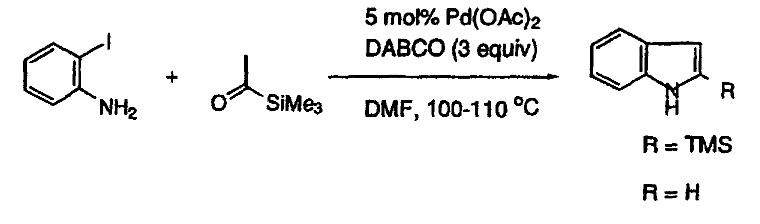

A mixture of iodoaniline (2.19 g, 10 mmol), acyl silane (2.36 g, 20 mmol, prepared according to the procedures of reference example A), DABCO (1,4-diazabicyclo[2.2.2]octane, 3.36 g, 30 mmol) and Pd(OAC)₂ (112.25 mg, 0.5 mmol) in 30 mL DMF was degassed via N₂/vacuum and heated at 105°C for 36 h. The mixture was cooled to room temperature, diluted with IPAc (isopropyl acetate, 100 mL) and washed with 2X50 mL of water. The IPAc layer was concentrated in vacuum and chromatographed over silica gel to give 2-trimethylsilyl indole and indole.

### EXAMPLES 2 TO 8

According to the procedures of Example 2, starting with the appropriately substituted 2-iodo aniline, and the appropriate acyl silane, the following compounds are prepared:

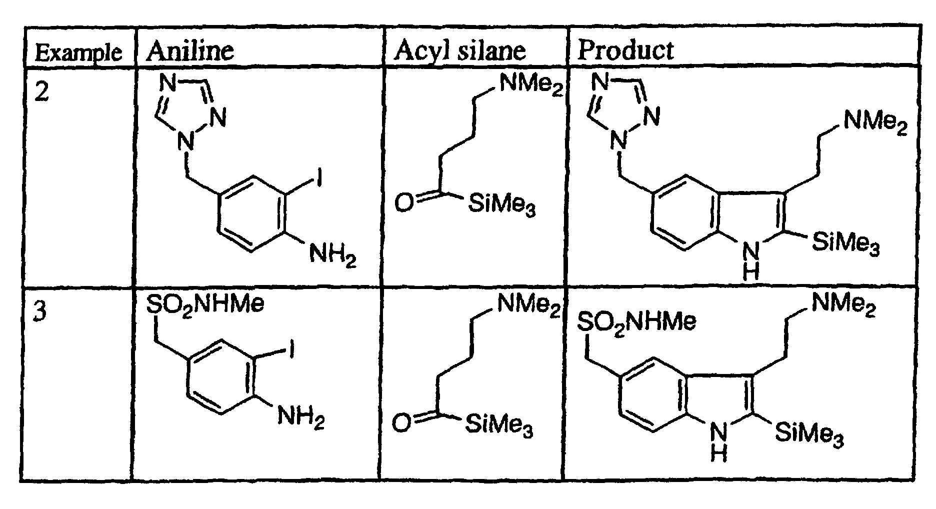

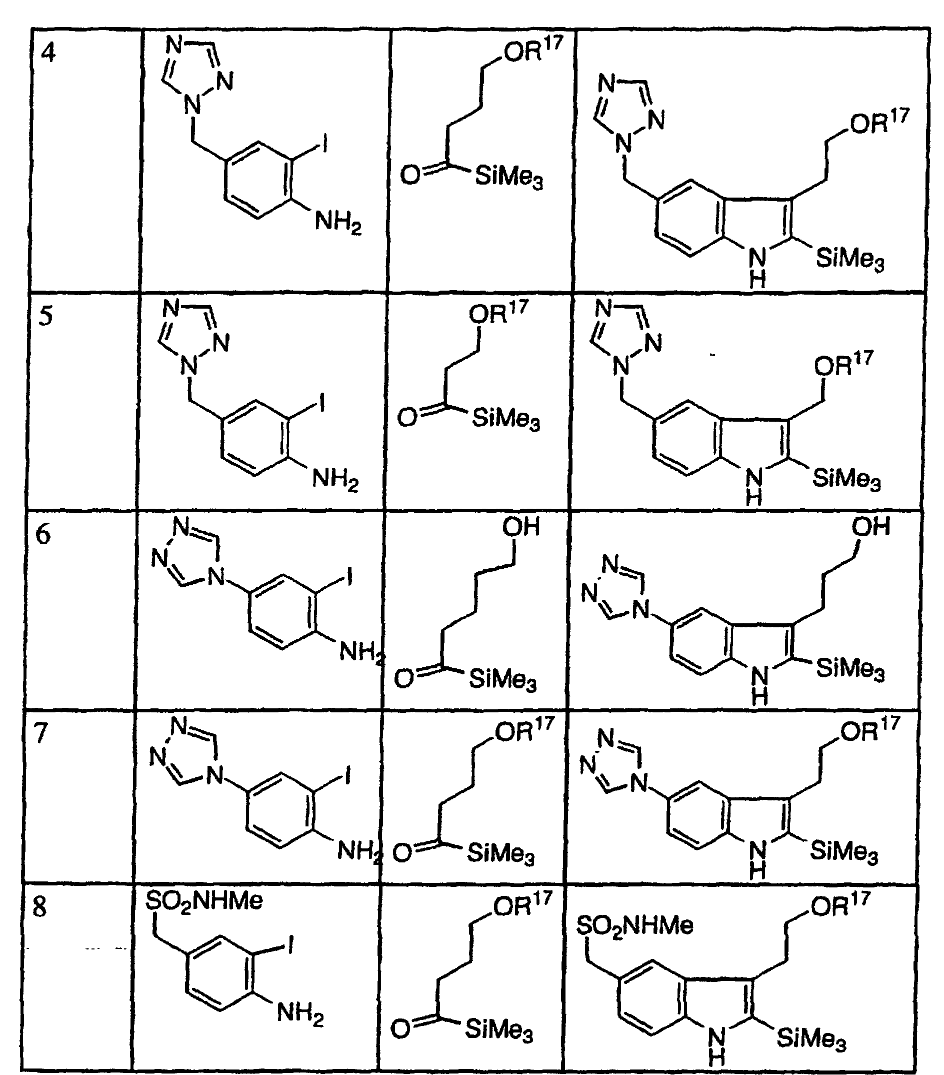

wherein R¹⁷ is a hydroxy protecting group that is removable under mild acid hydrolysis.

### EXAMPLE 9

### Preparation of Indole

A mixture of the 2-trimethylsilyl indole product of Example 1 (0.50 g, 2.6 mmol) in 5 mL methanol was treated with 2.5 N HCl (2.11 mL, 5.2 mmol) and the reaction mixture was aged at room temperature for 2 h. Isopropyl acetate (50 mL) and water (10 mL) were then added. The layers were separated and the organic layer was concentrated under vacuum. The residual oil was chromatographed over silica gel to afford the indole as a white solid.

### EXAMPLES 10-16

According to the procedure of Example 9 and employing the appropriate 2-trimethylsilyl indole product prepared according to the procedures of Examples 2 through 8, the following 2-unsubstituted indoles are prepared.

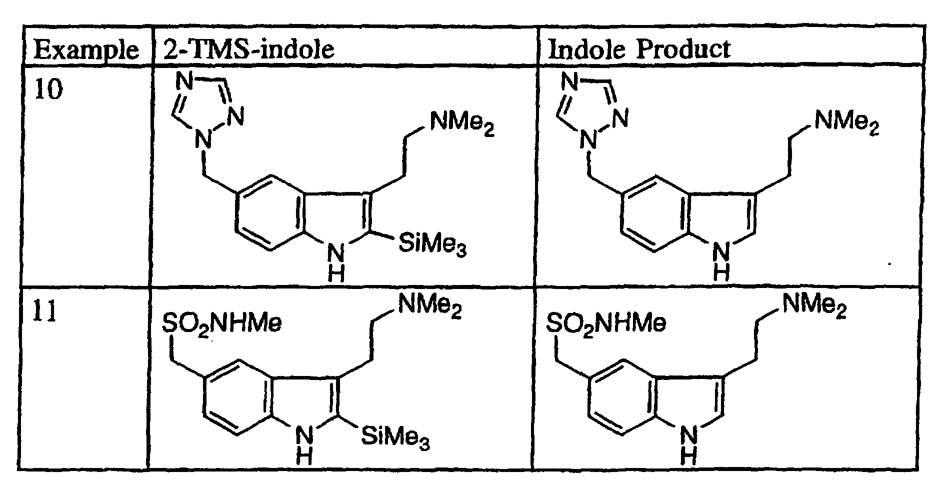

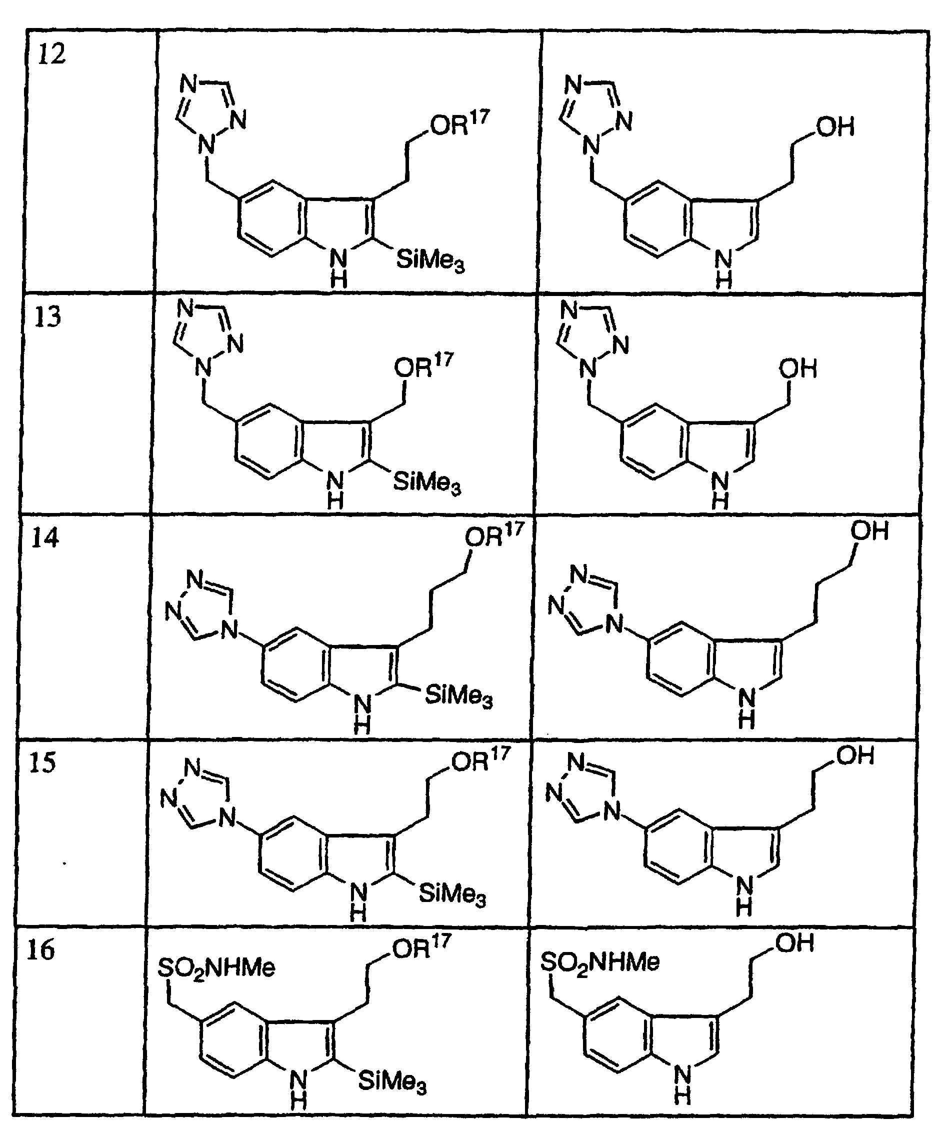

wherein R¹⁷ is a hydroxy protecting group that is removable under mild acid hydrolysis.

### REFERENCE EXAMPLE A

### Preparation of O-t-butyldimethylsilyl-2,2-dimethyl-2-sila-hexan-3-one-6-ol

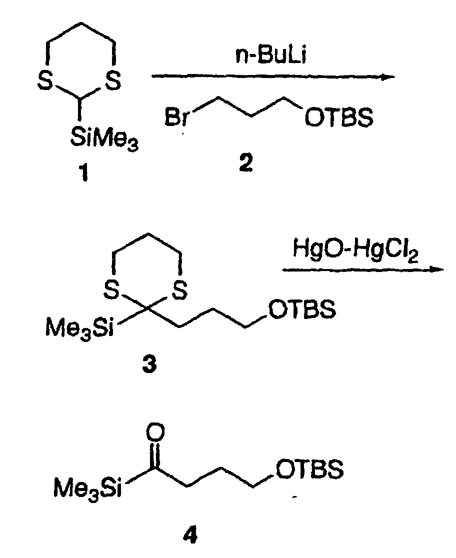

To 5 g (25.98 mmol) of TMS dithiane **1** in 70 mL dry THF at -78 °C was added n-BuLi (1.6 M, 16.25 mL, 26 mmol) dropwise. The mixture was warmed to -20 °C and aged at -20 °C for 0.5 h and cooled to -78 °C. Bromoether **2** in 5 mL of THF was added dropwise. The mixture was warmed to room temperature and aged for 12 h. It was partitioned between heptane (250 mL) and water (200 mL). The heptane layer was separated and concentrated under vacuum to give the dithiane **3** as a pale yellow oil. A portion of this material was directly used in the next step.

A mixture of dithiane **3** (4.0 g, 11 mmol), mercuric oxide (8.0 g) and mercuric chloride (8.0 g) in acetonitrile-H₂O (80:20, 30 mL) and ethyl acetate (10 mL) was aged at room temperature for 0.5 h. The solid was filtered and washed with ethyl acetate (40 mL). The filtrate and wash were combined and concentrated to an oil. This material was chromatographed over silica gel to give the acyl silane 4 as a pale yellow oil.

## Claims

1. A process for preparing a compound of structural formula III: 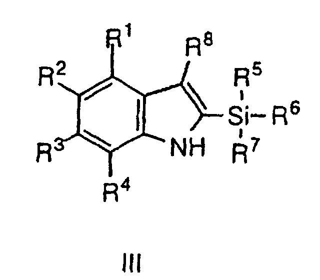 comprising reacting a compound of structural formula I with an acylsilane of structural formula II: 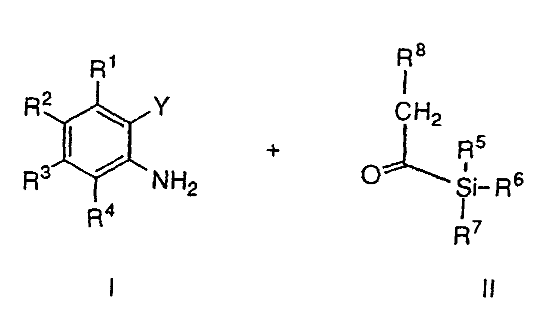 in the presence of a palladium catalyst and a proton acceptor,
wherein:
Y is selected from Br, I and triflate, and
R¹, R², R³ and R⁴ are each independently selected from:
(1) hydrogen;
(2) 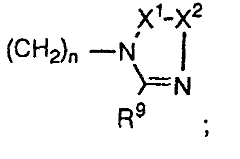
(3) C₁₋₆ alkyl;
(4) -(CH₂)ₙ-Z
wherein Z represents:
(a) hydrogen,
(b) halogen,
(c) cyano,
(d) nitro,
(e) trifluoromethyl,
(f) -OR¹⁰,
(g) -OCOR¹⁰,
(h) -OCONR¹⁰R¹¹,
(i) -OCH₂CN,
(j) -OCH₂CONR¹⁰R¹¹,
(k) -SR¹⁰,
(l) -SOR¹⁰,
(m) -SO₂R¹⁰,
(n) -SO₂NR¹⁰R¹¹,
(o) -NR¹⁰R¹¹,
(p) -NR¹⁰COR¹¹,
(q) -NR¹⁰CO₂R¹¹,
(r) -NR¹⁰SO₂R¹¹,
(s) -COR¹⁰,
(t) -CO₂R¹⁰,
(u) -CONR¹⁰R¹¹,
or a group of formula (Za), (Zb), (Zc), or (Zd): 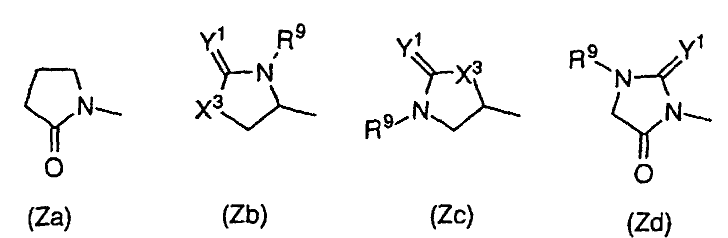 or Z represents an optionally substituted five-membered heteroaromatic ring selected from furan, thiophene, pyrrole, oxazole, thiazole, isoxazole, isothiazole, imidazole, pyrazole, oxadiazole, thiadiazole, triazole and tetrazole;
R⁵, R⁶, and R⁷ are each independently selected from:
(1) C₁₋₆ alkyl,
(2) -O-C₁₋₆ alkyl, and
(3) phenyl;
R⁸ is selected from:
(1) hydrogen,
(2) -R¹⁹-OH,
(3) -R¹⁹-O-R¹⁷, and
(4) -R¹⁹NR¹²R¹³, and
(5) -R¹⁹-Z¹
wherein: Z¹ is a 3 to 7 membered heterocyclic ring wherein the ring members are selected from 1 to 2 nitrogen atoms and wherein the heterocyclic ring may be subsituted by one or more R¹⁴;
R⁹ is selected from:
(1) hydrogen, and
(2) C₁₋₄ alkyl;
R¹⁰ and R¹¹ are each independently selected from:
(1) hydrogen,
(2) C₁₋₆ alkyl,
(3) trifluoromethyl,
(4) phenyl, optionally substituted with one or more R²⁰ substituents
(5) methylphenyl, optionally substituted with one or more R²⁰ substituents, and
(6) an arylC₁₋₆alkyl- or heteroaryl C₁₋₆alkyl- group. optionally substituted with one or more R²⁰ substituents, or
R¹⁰ and R¹¹ when linked through a nitrogen atom, together represent the residue of an optionally substituted azetidine, pyrrolidine, piperidine, morpholine or piperazine ring, optionally substituted with one or more R¹⁸ substituents;
R¹² and R¹³ are each independently selected from:
(1) C₁₋₄ alkyl,
(2) C₆aryl-C₁₋₄ alkyl- wherein aryl may be unsubstituted or substituted with one to three substituents selected from methyl, halo, and halomethyl,
R¹⁴ is selected from:
(1) aryl-C₁₋₆alkyl-, unsubstituted or substituted with one to three R²⁰ substitutents, and
(2) heteroaryl-C₁₋₆alkyl-, unsubstituted or substituted with one to three R²⁰ substitutents,
R¹⁵ and R¹⁶ are each independently selected from
(1) hydrogen,
(2) C₁₋₆alkyl,
(3) C₃₋₇cycloalkyl,
(4) C₃₋₇cycloalkylC₁₋₆alkyl-,
(5) indanyl,
(6) aryl,
(7) arylC₁₋₆alkyl-,
(8) C₃₋₇heterocycloalkyl-,
(9) C₃₋₇heterocycloalkylC₁₋₆alkyl-,
(10) heteroaryl, and
(11) heteroarylC₁₋₆alkyl-;
R¹⁷ is selected from a hydroxy protecting group that is removable under mild acid hydrolysis;
R¹⁸ is selected from:
(1) C₁₋₆alkyl-,
(2) arylC₁₋₆alkyl-,
(3) C₁₋₆alkoxy-,
(4) C₂₋₆alkyoxycarbonyl-, and
(5) C₁₋₆alkylaminocarbonyl-;
R¹⁹ is a straight or branched C₁₋₆alkyl chain;
R²⁰ is selected from:
(1) fluoro,
(2) cyano,
(3) trifluoromethyl,
(4) C₁₋₆alkyl,
(5) haloC₁₋₆alkyl-,
(6) aryl,
(7) triazolyl,
(8) tetrazolyl,
(9) tetrazolyl-C₁₋₆alkyl-,
(10) hydroxy,
(11) C₁₋₆alkoxy-,
(12) C₁₋₆alkylthio-,
(13) C₂₋₆alkoxycarbonyl-,
(14) C₂₋₆alkylcarbonyl-,
(15) C₁₋₆alkylsulphonyl-,
(16) arylsulfonyl-,
(17) C₂₋₆alkylcarbonylamino-,
(18) arylcarbonylamino-,
(19) C₂₋₆alkoxycarbonylamino-,
(20) N-C₁₋₆alkyl-N-C₂₋₆alkoxyamino-,
(21) carbonylamino-,
(22) mono- or diarylaminocarbonylamino-,
(23) pyrrolidinylcarbonylamino-,
(24) piperidinylcarbonylamino-,
(25) aminocarbonyl-,
(26) aminocarbonylamino-,
(27) C₁₋₆alkylaminocarbonyl-,
(28) C₁₋₆alkylaminocarbonylamino-,
(29) diC₁₋₆alkylaminocarbonyl-,
(30) diC₁₋₆alkylaminocarbonylamino-,
(31) pyrrolidinylcarbonylamino-,
(32) piperidinylcarbonylamino-,
(33) aminosulfonyl-,
(34) C₁₋₆alkylaminosulfonyl-,
(35) C₁₋₆alkylsulfonylamino-,
(36) C₁₋₆alkylsulfonylaminomethyl-,
(37) arylsulfonylamino-,
(38) diC₁₋₆alkylaminosulfonyl-,
(39) aminosulphonylmethyl-,
(40) C₁₋₆alkylaminosulfonylmethyl-,
(41) diC₁₋₆alkylaminosulfonylmethyl-,
(42) -(CH₂)ₘOR¹⁵,
(43) -(CH₂)ₘSR¹⁵,
(44) -(CH₂)ₘSOR¹⁵,
(45) -(CH₂)ₘSO₂R¹⁵,
(46) -(CH₂)ₘNR¹⁵R^{16,}
(47) =O, and
(48) 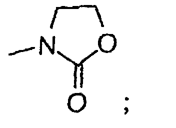
X¹ and X² are each independently selected from ring nitrogen or ring carbon atoms;
X³ is selected from the group consisting of oxygen, sulfur, -NH- or methylene;
Y¹ is oxygen or sulfur;
n is an integer independently selected at each occurrence from 0 and 1; and
m is an integer selected independently each occurrence from 0 to 4.

2. The process according to Claim 1 wherein R¹, R³, and R⁴ are each hydrogen.

3. The process according to Claim 1 wherein the palladium catalyst is selected from: a palladium alkanoate, a palladium acetonate, a palladium halide, a palladium halide complex, a palladium-benzylidine acetone complex and a triarylphosphine palladium complex.

4. The process according to Claim 3 wherein the palladium catalyst is selected from:
Pd(II) acetate, Pd(II) acetylacetonate, Pd(O)bis-dibenzylidene acetone ("dba"), Pd(II) bromide, Pd(II) chloride, Pd(II) iodide, Pd(II) sulfate, Pd(II)trifluoroacetate, Pd(II) Cl₂(CH₃CN)₂, Pd₂ (dba)₃, and Pd(II)Cl₂(PhCN)₂.

5. The process according to Claim 4 wherein the palladium catalyst is Pd(II) acetate.

6. The process according to Claim 1 wherein the proton acceptor does not interact with the palladium catalyst to inhibit its catalytic activity.

7. The process according to Claim 6 wherein the proton acceptor is selected from:
(a) an alkylamine,
(b) an aromatic amine,
(c) a heterocyclic amine, and
(d) a phosphate.

8. The process according to Claim 7 wherein the proton acceptor is an alkylamine.

9. The process according to Claim 8 wherein the alkylamine is selected from:
(a) 1,4-diazobicyclo[2.2.2]octane,
(b) quinuclidine,
(c) t-butylamine,
(d) 2,2,6,6-tetramethylpiperidine, and
(e) di-t-butylamine.

10. The process according to Claim 9 wherein the alkylamine is 1,4-diazobicyclo[2.2.2]octane.

11. The process according to Claim 1 wherein the reaction is carried out in a dry organic solvent inert for the starting materials.

12. The process according to Claim 11 wherein the solvent is selected from:
(a) DMSO,
(b) DMF,
(c) DMAC, and
(d) NMP.

13. The process according to Claim 12 wherein the solvent is DMF.

14. The process according to Claim 1 wherein the reaction is carried out at a temperature of 90 C° to 120 C°.

15. The process according to Claim 1 additionally comprising the step of deprotecting the compound of structural formula (III) to obtain the compound of structural formula (IV):

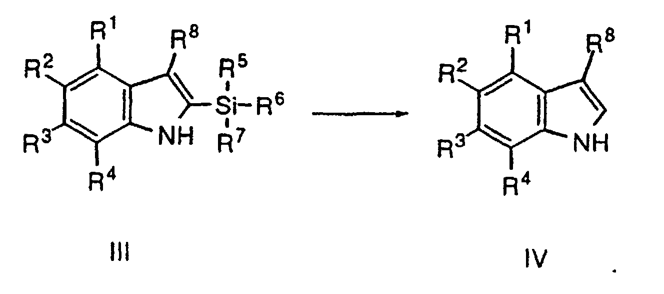

16. The process according to Claim 15 wherein the deprotection is a Lewis-acid catalyzed deprotection.

17. The process according to Claim 15 wherein:
R¹, R², R³ and R⁴ are each independently selected from:
(1) hydrogen;
(2) 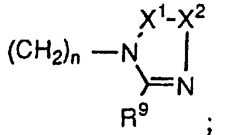
(3) C₁₋₆ alkyl;
(4) -(CH₂)ₙ-Z
wherein Z represents:
(a) hydrogen,
(b) halogen,
(c) cyano,
(d) nitro,
(e) trifluoromethyl,
(f) OR¹⁰,
(g) OCOR¹⁰,
(h) OCONR¹⁰R¹¹,
(i) OCH₂CN,
(j) OCH₂CONR¹⁰R¹¹,
(k) SR¹⁰, provided that R¹⁰ is not hydrogen,
(l) SOR¹⁰,
(m) SO₂R¹⁰,
(n) SO₂NR¹⁰R¹¹,
(o) NR¹⁰R¹¹,
(p) NR¹⁰COR¹¹,
(q) NR¹⁰CO₂R¹¹,
(r) NR¹⁰SO₂R¹¹,
(s) COR¹⁰,
(t) CO₂R¹⁰,
(u) CONR¹⁰R¹¹,
or Z is a group of formula (Za), (Zb), (Zc), or (Zd): 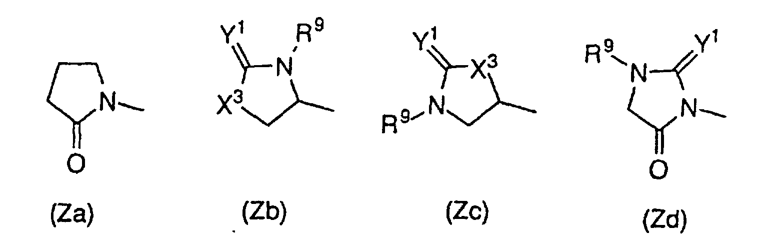
or Z represents an optionally substituted five-membered heteroaromatic ring selected from furan, thiophene, pyrrole, oxazole, thiazole, isoxazole, isothiazole, imidazole, pyrazole, oxadiazole, thiadiazole, triazole and tetrazole;
R⁵, R⁶, and R⁷ are each independently selected from:
(1) C₁₋₆ alkyl,
(2) -O-C₁₋₆ alkyl, and
(2) phenyl;
R⁸ is selected from:
(1) hydrogen,
(2) -R¹⁹-OH,
(3) -R¹⁹-O-R¹⁷, and
(4) -R¹⁹NR¹²R¹³, and
(5) -R¹⁹-Z¹
wherein: Z¹ is a 3 to 7 membered heterocyclic ring wherein the ring members are selected from 1 to 2 nitrogen atoms and wherein the heterocyclic ring may be subsituted by one or more R¹⁴;
R⁹ is selected from:
(1) hydrogen, and
(2) C₁₋₄ alkyl;
R¹⁰ and R¹¹ are each independently selected from:
(1) hydrogen,
(2) C₁₋₆ alkyl,
(3) trifluoromethyl,
(4) phenyl, optionally substituted with one or more R²⁰ substituents
(5) methylphenyl, optionally substituted with one or more R²⁰ substituents, and
(6) an arylC₁₋₆alkyl or heteroaryl C₁₋₆alkyl group. optionally substituted with one or more R²⁰ substituents, or
R¹⁰ and R¹¹ when linked through a nitrogen atom, together represent the residue of an optionally substituted azetidine, pyrrolidine, piperidine, morpholine or piperazine ring, optionally substituted with one or more R¹⁸ substituents;
R¹² and R¹³ are each independently selected from:
(1) C₁₋₄ alkyl,
(2) C₁₋₄ alkyl-C₆aryl wherein aryl may be unsubstituted or substituted with one to three substituents selected from methyl, halo, and halomethyl,
R¹⁴ is selected from:
(1) aryl-C₁₋₆alkyl, unsubstituted or substituted with one to three R²⁰ substitutents, and
(2) heteroaryl-C₁₋₆alkyl, unsubstituted or substituted with one to three R²⁰ substitutents,
R¹⁵ and R¹⁶ are each independently selected from
(1) hydrogen,
(2) C₁₋₆alkyl,
(3) C₃₋₇cycloalkyl,
(4) C₃₋₇cycloalkylC₁₋₆alkyl,
(5) indanyl,
(6) aryl,
(7) arylC₁₋₆alkyl,
(8) C₃₋₇heterocycloalkyl,
(9) C₃₋₇heterocycloalkylC₁₋₆alkyl,
(10) heteroaryl, and
(11) heteroarylC₁₋₆alkyl;
R¹⁷ is selected from a hydroxy protecting group that is removable under mild acid hydrolysis;
R¹⁸ is selected from:
(1) C₁₋₆alkyl,
(2) arylC₁₋₆alkyl,
(3) C₁₋₆alkoxy,
(4) C₂₋₆alkyoxycarbonyl, and
(5) C₁₋₆alkylaminocarbonyl;
R¹⁹ is a straight or branched C₁₋₆alkyl chain;
R²⁰ is selected from:
(1) fluoro,
(2) cyano,
(3) trifluoromethyl,
(4) C₁₋₆alkyl,
(5) haloC₁₋₆alkyl,
(6) aryl,
(7) triazolyl,
(8) tetrazolyl,
(9) C₁₋₆alkyl-tetrazolyl,
(10) hydroxy,
(11) C₁₋₆alkoxy,
(12) C₁₋₆alkylthio,
(13) C₂₋₆alkoxycarbonyl,
(14) C₂₋₆alkylcarbonyl,
(15) C₁₋₆alkylsulphonyl,
(16) arylsulfonyl,
(17) C₂₋₆alkylcarbonylamino,
(18) arylcarbonylamino,
(19) C₂₋₆alkoxycarbonylamino,
(20) N-C₁₋₆alkyl-N-C₂₋₆alkoxyamino,
(21) carbonylamino,
(22) mono- or diarylaminocarbonylamino,
(23) pyrrolidinylcarbonylamino,
(24) piperidinylcarbonylamino,
(25) aminocarbonyl,
(26) aminocarbonylamino,
(27) C₁₋₆alkylaminocarbonyl,
(28) C₁₋₆alkylaminocarbonylamino,
(29) diC₁₋₆alkylaminocarbonyl,
(30) diC₁₋₆alkylaminocarbonylamino,
(31) pyrrolidinylcarbonylamino,
(32) piperidinylcarbonylamino,
(33) aminosulfonyl,
(34) C₁₋₆alkylaminosulfonyl,
(35) C₁₋₆alkylsulfonylamino,
(36) C₁₋₆alkylsulfonylaminomethyl,
(37) arylsulfonylamino,
(38) diC₁₋₆alkylaminosulfonyl,
(39) aminosulphonylmethyl,
(40) C₁₋₆alkylaminosulfonylmethyl, and
(41) diC₁₋₆alkylaminosulfonylmethyl,
(42) (CH₂)ₘOR¹⁵,
(43) (CH₂)ₘSR¹⁵, provided that R¹⁵ is not hydrogen,
(44) (CH₂)ₘSOR¹⁵,
(45) (CH₂)ₘSO₂R¹⁵,
(46) (CH₂)ₘNR¹⁵R¹⁶,
(47) =O, and
(48) 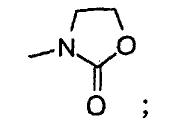
X¹ and X² are each independently selected from ring nitrogen or ring carbon atoms;
X³ is selected from the group consisting of oxygen, sulfur, -NH- or methylene;
Y¹ is oxygen or sulfur;
n is an integer independently selected at each occurrence from 0 and 1; and
m is an integer selected independently at each occurrence from 0 to 4.

18. The process according to Claim 17 wherein R¹, R³, and R⁴ are each hydrogen.

19. The process according to Claim 18 wherein the compound according to structural formula IV is selected from:
(1) 1-benzyl-4-[3-(5-(1,2,4-triazol-4-yl)-1H-indol-3-yl)propyl]piperazin-2-one;
(2) 1-(2-phenylethyl)-4-[3-(5-(1,2,4-triazol-4-yl)-1H-indol-3-yl)propyl]piperazin-2-one;
(3) 1-[2-(3-fluorophenyl)ethyl]-4-[3-(5-(1,2,4-triazol-4-yl)-1H-indol-3-yl)propyl]piperazin-2-one;
(4) (3*S*)-3-(*N*-benzyl)aminomethyl-1-[2-(5-(*N*-methyl)-aminosulphonylmethyl)-1*H*-indol-3-yl)ethyl]pyrrolidine;
(5) (3*S*)-3-(*N*-benzyl)aminomethyl-1-[2-(5-(aminosulphonylmethyl)-1*H*-indol-3-yl)ethyl]pyrrolidine;
(6) (3*S*)-3-(*N*-benzyl)aminomethyl-(*S*)-1-[2-(5-(2-oxo-1,3-oxazolidin-4-ylmethyl)-1*H*-indol-3-yl)ethyl]pyrrolidine;
(7) (3*S*)-3-[*N*-(*R*)-α-(hydroxymethyl)benzyl]aminomethyl-(*S*)-1-[2-(5-(2-oxo-1,3-oxazolidin-4-ylmethyl)-1*H*-indol-3-yl)ethyl]pyrrolidine;
(8) (3*S*)-3-[*N*-(*S*)-α-methylbenzyl]aminomethyl-(*S*)-1-[2-(5-(2-oxo-1,3-oxazolidin-4-ylmethyl)-1*H*-indol-3-yl)ethyl]pyrrolidine;
(9) 4-[*N*-(*R*)-α-(hydroxymethyl)benzyl]amino-(*S*)-1-[3-(5-(2-oxo-1,3-oxazolidin-4-ylmethyl)-1*H*-indol-3-yl)propyl]piperidine;
(10) (3*S*)-3-(*N*-benzyl-*N*-methyl)aminomethyl-(*S*)-1-[2-(5-(3-methyl-2-oxo-1,3-oxazolidin-4-ylmethyl)-1*H*-indol-3-yl )ethyl]pyrrolidine;
(11) (3R)-3-[*N*-(*S*)-α-methylbenzyl-*N*-methyl]aminomethyl-(*S*)-1-[2-(5-(2-oxo-1,3-oxazolidin-4-ylmethyl)-1*H*-indol-3-yl )ethyl]pyrrolidine;
(12) (3*R*)-3-[*N*-(*S*)-α-methylbenzyl-*N*-methyl]aminomethyl-(*S*)-1-[2-(5-(3-methyl-2-oxo-1,3-oxazolidin-4-ylmethyl)-1*H*-indol-3-yl)ethyl]pyrrolidine;
(13) (3*S*)-3-[*N*-(4-fluorobenzyl)-*N*-methyl]aminomethyl-(*S*)-1-[2-(5-(3-methyl-2-oxo-1,3-oxazolidin-4-ylmethyl)-1*H*-indol-3-yl )ethyl]pyrrolidine;
(14) 4-benzyl12-[2-fluoromethyl-3-(5-(1,2,4-triazol-4-yl)-1H-indol-3-yl)propyl]piperazine;
(15) 4-[2-(3-fluorophenyl)ethyl]-1-[2-fluoro-3-(5-(1,2,4-triazol-4-yl)-1H-indol-3-yl)propyl]piperazine;
(16) 4-benzyl-1-[2-fluoro-3-(5-(1,2,4-triazol-4-yl)-1H-indol-3-yl)propyl]piperidine;
(17) 4-(N-benzyl-N-methylamino)-1-[2-fluoro-3-(5-(1,2,4-triazol-4-yl)-1H-indol-3-yl)propyl]piperidine;
(18) 4-[(R)-2-hydroxy-1-(4-fluorophenyl)ethylamino]-1-[2-fluoro-3-(5-(1,2,4-triazol-4-yl)-1H-indol-3-yl)propyl]piperidine;
(19) 7-benzyl-2-[2-(5-(1,2,4-triazol-4-yl)-1H-indol-3-yl)ethyl]-cis-2,7-diazabicyclo[3.3.0]octane;
(20) 7-(3-furylmethyl-2-[2-(5-(1,2,4-triazol-4-yl)-1H-indol-3-yl)ethyl]-*cis*-2,7-diazabicyclo[3.3.0]octane;
(21) 7-(2-phenylethyl-2-[2-(5-(1,2,4-triazol-4-yl)-1H-indol-3-yl)ethyl]-*cis*-2,7-diazabicyclo[3.3.0]octane;
(22) 7-(4-fluorobenzyl-2-[2-(5-(1,2,4-triazol-4-yl)-1H-indol-3-yl)ethyl]-*cis*-2,7-diazabicyclo[3.3.0]octane;
(23) 7-(2,4-difluorobenzyl-2-[2-(5-(1,2,4-triazol-4-yl)-1H-indol-3-yl)ethyl]-*cis*-2,7-diazabicyclo[3.3.0]octane;
(24) 4-(2,2-difluoro-1-oxo-2-phenylethyl)-1-[3-(5-(1,2,4-triazol-4-yl)-lH-indol-3-yl)propyl]piperazine;
(25) 4-benzyl-3-methyl-1-[3-(5-(1,2,4-triazol-4-yl)-1H-indol-3-yl)propyl]-1,2,5,6-tetrahydropyridine;
(26) 4-benzyl-3-methoxymethyl-1-[3-(5-(1,2,4-triazol-4-yl)-1H-indol-3-yl)propyl]-1,2,5,6-tetrahydropyridine;
(27) 1-(2-hydroxy-1-phenylethyl)-4-[3-(5-(1,2,4-triazol-4-yl)-1*H*-indol-3-yl)propyl]piperidine;
(28) 1-[2-(2-fluorophenyl)ethyl]-4-[3-(5-(1,2,4-triazol-4-yl)-1*H*-indol-3-yl)propyl]piperidine;
(29) 1-benzyl-4-[3-(5-(1,2,4-triazol-4-yl)-1*H*-indol-3-yl)propyl]piperidine;
(30) 1-(3,3-dimethylbutyl)-4-[3-(5-(1,2,4-triazol-4-yl)-1*H*-indol-3-yl)propyl]piperidine;
(31) 1-(2-phenylethyl)-4-[3-(5-(1,2,4-triazol-4-yl)-1*H*-indol-3-yl)propyl]piperidine;
(32) 1-cyclohexylmethyl-4-[3-(5-(1,2,4-triazol-4-yl)-1*H*-indol-3-yl)propyl]piperidine;
(33) 1-(3-phenylpropyl)-4-[3-(5-( 1,2,4-triazol-4-yl)-1*H*-indol-3-yl)propyl]piperidirie
(34) 1-[2-(3-fluorophenyl)ethyl]-4-[3-(5-(1,2,4-triazol-4-yl)-1*H*-indol-3-yl)propyl]piperidine;
(35) 1-[2-(4-trifluoromethylphenyl)ethyl]-4-[3-(5-(1,2,4-triazol-4-yl)-1*H*-indol-3-yl)propyl]piperidine;
(36) 1-[2-(3,4-difluorophenyl)ethyl]-4-[3-(5-(1,2,4-triazol-4-yl)-1*H*-indol-3-yl)propyl]piperidine;
(37) N-methyl-2-phenyl-2-[4-(3-(5-(1,2,4-triazol-4-yl)-1*H*-indol-3-yl)propyl)piperidin-1-yl]acetamide;
(38) 1-(2-oxo-2-phenylethyl)-4-[3-(5-(1,2,4-triazol-4-yl)-1*H*-indol-3-yl)propyl]piperidine;
(39) 1-(2-phenylpropyl)-4-[3-(5-(1,2,4-triazol-4-yl)-1*H*-indol-3-yl)propyl]piperidine;
(40) 4-benzyl-4-fluoro-1-[3-(5-(1,2,4-triazol-4-yl)-1*H*-indol-3-yl)propyl]piperidine;
(41) 4-fluoro-4-[2-(3-fluorophenyl)ethyl]-1-[3-(5-(1,2,4-triazol-4-yl)-1*H*-indol-3-yl)propyl]piperidine;
(42) 4-fluoro-4-(3-fluorobenzyl)-1-[3-(5-(1,2,4-triazol-4-yl)-1*H*-indol-3-yl)propyl]piperidine;
(43) 4-fluoro-4-(2-fluorobenzyl)-1-[3-(5-(1,2,4-triazol-4-yl)-1*H*-indol-3-yl)propyl]piperidine;
(44) 4-benzyl-4-methoxy-1-[3-(5-(1,2,4-triazol-4-yl)-1*H*-indol-3-yl)propyl]piperidine;
(45) 4-benzyl-4-methoxy-1-[3-(5-(1,2,4-triazol-1-ylmethyl)-1*H*-indol-3-yl)propyl]piperidine;
(46) 4-(2-fluorobenzyl)-4-methoxy-1-[3-(5-(1,2,4-triazol-4-yl)-1*H*-indol-3-yl)propyl]piperidine;
(47) 4-(3-fluorobenzyl)-4-methoxy-1-[3-(5-(1,2,4-triazol-4-yl)-1*H*-indol-3-yl)propyl]piperidine;
(48) 4-(4-fluorobenzyl)-4-methoxy-1-[3-(5-(1,2,4-triazol-4-yl)-1*H*-indol-3-yl)propyl]piperidine;
(49) 1-[3-(5-(1,2,4-triazol-4-yl)-1*H*-indol-3-yl)propyl]-4-[2-(oxazol-2-on-3-yl)-1-phenylethyl]piperazine;
(50) 1-[3-(5-(1,2,4-triazol-4-yl)-1*H*-indol-3-yl)propyl]-4-[2-(oxazolidin-2-on-3-yl)-1-phenylethyl]piperazine;
(51) 1-[3-(5-(1,2,4-triazol-4-yl)-1*H*-indol-3-yl)propyl]-4-[1-(4-fluorophenyl)-2-(oxazolidin-2-on-3-yl)ethyl]piperazine;
(52) 1-[3-(5-(1,2,4-triazol-4-yl)-1*H*-indol-3-yl)propyl]-4-(3-hydroxy-1-phenylpropyl)piperazine;
(53) 1-[3-(5-(1,2,4-triazol-4-yl)-1*H*-indol-3-yl)propyl]-4-[2-(imidazol-1-yl)-1-phenylethyl]piperazine;
(54) 1-[3-(5-(1,2,4-triazol-4-yl)-1*H*-indol-3-yl)propyl]-4-[1-(4-fluorophenyl)-2-hydroxyethyl]piperazine;
(55) 1-[3-(5-(1,2,4-triazol-4-yl)-1*H*-indol-3-yl)propyl]-4-[1-(4-fluorophenyl)-2-methoxyethyl]piperazine;
(56) 1-[3-(5-(1,2,4-triazol-4-yl)-1*H*-indol-3-yl)propyl]-4-[3-(5-methyl-1,2,4-oxadiazol-3-yl)-1-phenylpropyl]piperazine;
(57) 1-[3-(5-(1,2,4-triazol-4-yl)-1*H*-indol-3-yl)propyl]-4-[2-benzyloxy-1-(4-fluorophenyl)ethyl]piperazine;
(58) 1-[3-(5-(1,2,4-triazol-4-yl)-1*H*-indol-3-yl)propyl]-4-[1-(4-fluorophenyl)-3-methoxypropyl]piperazine;
(59) 1-[3-(5-(1,2,4-triazol-4-yl)-1*H*-indol-3-yl)propyl]-4-[1-(4-fluorophenyl)-2-(imidazol-1-yl)ethyl]piperazine;
(60) 1-[3-(5-(1,2,4-triazol-4-yl)-1*H*-indol-3-yl)propyl]-4-[1-phenyl-2-(pyrrolidin-1-yl)ethyl]piperazine;
(61) 1-[3-(5-(1,2,4-triazol-4-yl)-1*H*-indol-3-yl)propyl]-4-[1-(4-fluorophenyl)-3-hydroxypropyl]piperazine;
(62) 1-[3-(5-(1,2,4-triazol-4-yl)-1*H*-indol-3-yl)propyl]-4-[3-(imidazol-1-yl)-1-phenylpropyl]piperazine;
(63) 1-[3-(5-(1,2,4-triazol-4-yl)-1*H*-indol-3-yl)propyl]-4-(3-hydroxy-2-phenylpropyl)piperazine;
(64) 1-[3-(5-(1,2,4-triazol-4-yl)-1*H*-indol-3-yl)propyl]-4-(3-methoxy-2-phenylpropyl)piperazine;
(65) 1-[3-(5-(1,2,4-triazol-4-yl)-1*H*-indol-3-yl)propyl]-4-[2-(4-fluorophenyl)-3-hydroxypropyl]piperazine;
(66) 1-[3-(5-(1,2,4-triazol-4-yl)-1*H*-indol-3-yl)propyl]-4-[1-(4-fluorophenyl)prop-2-yl]piperazine;
(67) 1-[3-(5-(1,2,4-triazol-4-yl)-1*H*-indol-3-yl)propyl]-4-[2-(4-fluorophenyl)propyl]piperazine;
(68) 1-[3-(5-(1,2,4-triazol-4-yl)-1*H*-indol-3-yl)propyl]-4-[1-(4-fluorophenyl)-3-hydroxyprop-2-yl]piperazine;
(69) 1-[3-(5-(1,2,4-triazol-4-yl)-1*H*-indol-3-yl)propyl]-4-[1-(4-fluorophenyl)-3-methoxyprop-2-yl]piperazine;
(70) 1-[3-(5-(1,2,4-triazol-4-yl)-1*H*-indol-3-yl)propyl]-4-[2-(3-fluorophenyl)-ethyl]piperazine;
(71) 1-[3-(5-(1,2,4-triazol-4-yl)-1*H*-indol-3-yl)propyl]-4-[2-(2-fluorophenyl)-ethyl]piperazine;
(72) 1-[3-(5-(1,2,4-triazol-4-yl)-1*H*-indol-3-yl)propyl]-4-[2-(4-fluorophenyl)-ethyl]piperazine;
(73) 1-[3-(5-(1,2,4-triazol-4-yl)-1*H*-indol-3-yl)propyl]-4-[2-(3-methoxyphenyl)-ethyl]piperazine;
(74) 1-[3-(5-(1,2,4-triazol-4-yl)-1*H*-indol-3-yl)propyl]-4-[2-(3-trifluoromethyl-phenyl)ethyl]piperazine;
(75) 1-[3-(5-(1,2,4-triazol-4-yl)-1*H*-indol-3-yl)propyl]-4-[2-(3,4-difluorophenyl)-ethyl]piperazine;
(76) 1-[3-(5-(1,2,4-triazol-4-yl)-1*H*-indol-3-yl)propyl]-4-[2-(2,4-difluorophenyl)-ethyl]piperazine;
(77) 1-[3-(5-(1,2,4-triazol-4-yl)-1*H*-indol-3-yl)propyl]-4-[2-(3,5-difluorophenyl)-ethyl]piperazine;
(78) 1-[3-(5-(1,2,4-triazol-4-yl)-1*H*-indol-3-yl)propyl]-4-[2-(3-(oxazolidin-2-on-3-yl)phenyl)ethyl]piperazine;
(79) N-methyl-3-[5-(1,2,4-triazol-4-yl)-1H-indol-3-yl]pyrrolidine;
(80) N-methyl-4-[5-(1,2,4-triazol-4-yl)-1H-indol-3-yl]piperidine;
(81) N,N-dimethyl-2-[5-(1,2,4-triazol-4-yl)-1H-indol-3-yl]ethylamine;
(82) 4-(1-phenylethyl)-1-[3-(5-(1,2,4-triazol-4-yl)-1*H*-indol-3-yl)propyl]-1,2,5,6-tetrahydropyridine;
(83) 4-(a-isopropyloxy)phenylmethyl-1-[3-(5-(1,2,4-triazol-4-yl)-1*H*-indol-3-yl)propyl]-1,2,5,6-tetrahydropyridine;
(84) 4-(a-methoxy)phenylmethyl-1-[3-(5-(1,2,4-triazol-4-yl)-1*H*-indol-3-yl)propyl]-1,2,5,6-tetrahydropyridine;
(85) 4-[a-(2-methoxyethyl)oxy]phenylmethyl-1-[3-(5-(1,2,4-triazol-4-yl)-1*H*-indol-3-yl)propyl]-1,2,5,6-tetrahydropyridine;
(86) 4-benzyl-1-[3-(2,3-dihydro-5-(1,2,4-triazol-4-yl)-1*H*-indol-3-yl)propyl]piperazine;
(87) 1-[3-(2,3-dihydro-5-(1,2,4-triazol-4-yl)-1*H*-indol-3-yl)propyl]-4-(pyridin-3-ylmethyl)piperazine;
(88) 1-[2-(5-(1,2,4-triazol-4-yl)-1*H*-indol-3-yl)propyl]-4-[2-(4-fluorophenyl)-4-methylpiperazin-1-yl]piperidine;
(89) 1-[2-(5-(1,2,4-triazol-1-ylmethyl)-1*H*-indol-3-yl)ethyl]-3(*R*)-(3(*R*)-phenylmorpholin-4-ylmethyl)pyrrolidine;
(90) 1-[3-(5-(1,2,4-triazol-4-yl)-1*H*-indol-3-yl)propyl]-4-(3-oxo-2-phenylpiperazin-1-yl)methylpiperidine;
(91) 1-[3-(5-(1,2,4-triazol-4-yl)-1*H*-indol-3-yl)propyl]-4-(4-methyl-2-phenylpiperazin-1-yl)piperidine;
(92) 1-[3-(5-(1,2,4-triazol-4-yl)-1*H*-indol-3-yl)propyl]-4-(benzimidazol-2-on-1-yl)piperidine;
(93) 1-[3-(5-(1,2,4-triazol-4-yl)-1*H*-indol-3-yl)propyl]-4-[6-(4-fluorophenyl)-4-methyl-3-oxopiperazin-1-yl]piperidine;
(94) [3-(3-(4-(2-(3,4-difluorophenyl)ethyl)piperazin-1-yl)propyl)-1*H*-indol-5-ylmethyl]oxazolidin-2-one;
(95) (*S*)-4-[3-(3-(4-(2-(3,4-difluorophenyl)ethyl)piperazin-1-yl)propyl)-1*H*-indol-5-ylmethyl]-3-methyloxazolidin-2-one;
(96) 1-[3-(5-(N-methylaminosulphonylmethyl)-1H-indol-3-yl)propyl)-4-[2-(4-(acetylamino)phenyl)ethyl)piperazine;
(97) 3-benzyl-7-[3-(5-(1,2,4-triazol-4-yl)-1*H*-indol-3-yl)propyl]-*cis*-3,7-diazabicyclo[3.3.0]octane;
(98) 3-(pyridin-3-yl)methyl-7-[3-(5-(1,2,4-triazol-4-yl)-1*H*-indol-3-yl)propyl]-*cis*-3,7-diazabicyclo[3.3.0]octane;
(99) 3-[2-(4-(acetylamino)phenyl)ethyl]-7-[3-(5-(1,2,4-triazol-4-yl)-1*H*-indol-3-yl)propyl]-*cis*-3,7-diazabicyclo[3.3.0]octane;
(100) 3-benzoyl-7-[3-(5-(1,2,4-triazol-4-yl)-1*H*-indol-3-yl)propyl]*-cis*-3,7-diazabicyclo[3.3.0]octane;
(101) (1*RS*,3*RS*,5*RS*)-7-benzyl-3-[5-(imidazol-1-yl)-1*H*-indol-3-ylmethyl]-2-methyl-2,7-diazabicyclo[3.3.0]octane;
(102) (1*RS*,3*RS*,5*RS*)-7-[2-(3-fluorophenyl)ethyl]-2-methyl-3-[5-(1,2,4-triazol-4-yl)-1*H*-indol-3-ylmethyl]-2,7-diazabicyclo[3.3.0]octane;
(103) (1*RS*,3*RS*,5*RS*)-7-(4-fluorobenzyl)-2-methyl-3-[5-(1,2,4-triazol-4-yl)-1*H*-indol-3-ylmethyl]-2,7-diazabicyclo[3.3.0]octane;
(104) 4-[1-(phenyl)-N,N-dimethylcarboxamidomethyl]-1-[3-(5-(1,2,4-triazol-4-yl)-1H-indol-3-yl)propyl]piperazine;
(105) 4-(2-methoxycarbonylamino-1-phenylethyl)-1-[3-(5-(1,2,4-triazol-4-yl)-1H-indol-3-yl)propyl]piperazine;
(106) 4-(2-dimethylamino-1-phenylethyl)-1-[3-(5-(1,2,4-triazol-4-yl)-1H-indol-3-yl)propyl]piperazine;
(107) 1-[3-(5-(1,2,4-triazol-4-yl)-1H-indol-3-yl)propyl]-4-(4-fluorophenyl)methylpiperazine;
(108) 4-[2-(N-methyl-N-methoxycarbonyl)amino-1-phenylethyl]-1-[3-(5-(1,2,4-triazol-4-yl)-1H-indol-3-yl)propyl]piperazine;
(109) 1-benzyl-4-[(R,S)-2-hydroxymethyl-3-(5-(1,2,4-triazol-4-yl)-1H-indol-3-yl)propyl]piperazine;
(110) 1-[3-(5-(1,2,4-triazol-4-yl)-1H-indol-3-yl)propyl]-4-[2-(1H-tetrazol-5-yl)phenyl]methylpiperazine;
(111) 1-[3-(5-(1,2,4-triazol-4-yl)-1H-indol-3-yl)propyl]-4-(2-phenylethyl)piperazine;
(112) 4-benzyl-1-[3-(5-(1,2,4-triazol-4-yl)-1H-indol-3-yl)propyl]piperazine;
(113) 4-[2-(2-methyltetrazol-5-yl)phenyl]methyl-1-[3-(5-(1,2,4-triazol-4-yl)-1H-indol-3-yl)propyl]piperazine;
(114) 4-[2-(1-methyltetrazol-5-yl)phenyl]methyl-1-[3-(5-(1,2,4-triazol-4-yl)-1H-indol-3-yl)propyl]piperazine;
(115) 4-[2-(N-methylcarboxamido)phenyl]methyl-1-[3-(5-(1,2,4-triazol-4-yl)-1H-indol-3-yl)propyl]piperazine;
(116) 4-[2-(N,N-dimethylaminomethyl)phenyl]methyl-1-[3-(5-(1,2,4-triazol-4-yl)-1H-indol-3-yl)propyl]piperazine;
(117) 4-(but-3-enyl)-1-[3-(5-(1,2,4-triazol-4-yl)-1H-indol-3-yl)propyl]piperazine;
(118) 4-(3-methylbut-2-enyl)-1-[3-(5-(1,2,4-triazol-4-yl)-1H-indol-3-yl)propyl]piperazine;
(119) 4-(prop-2-enyl)-1-[3-(5-(1,2,4-triazol-4-yl)-1H-indol-3-yl)propyl]piperazine;
(120) 4-(prop-2-ynyl)-1-[3-(5-(1,2,4-triazol-4-yl)-1H-indol-3-yl)propyl]piperazine;
(121) 4-[(R,S)-1-(phenyl)carboxamidomethyl]-1-[3-(5-(1,2,4-triazol-4-yl)-1H-indol-3-yl)propyl]piperazine;
(122) 4-[1-(phenyl)-N-methylcarboxamidomethyl]-1-[3-(5-(1,2,4-triazol-4-yl)-1H-indol-3-yl)propyl]piperazine;
(123) 1-[3-(5-(1,2,4-triazol-1-ylmethyl)-1H-indol-3-yl)propyl]-4-[2-(4-(acetylamino)phenyl)ethyl]piperazine;
(124) 1-[3-(5-(1,2,4-triazol-4-yl)-1H-indol-3-yl)propyl]-4-[2-(3-(acetylamino)phenyl)ethyl]piperazine;
(125) 1-[3-(5-(1,2,4-triazol-4-yl)-1H-indol-3-yl)propyl]-4-[4-(aminosulphonyl)phenyl]methylpiperazine;
(126) 1-[3-(5-(1,2,4-triazol-4-yl)-1H-indol-3-yl)propyl]-4-(furan-3-yl)methylpiperazine;
(127) 1-[3-(5-(1,2,4-triazol-4-yl)-1H-indol-3-yl)propyl]-4-(furan-2-yl)methylpiperazine;
(128) 1-[3-(5-(1,2,4-triazol-4-yl)-1H-indol-3-yl)propyl]-4-(thien-2-yl)methylpiperazine;
(129) 1-benzyl-4-[(R,S)-2-hydroxy-3-(5-(1,2,4-triazol-4-yl)-1H-indol-3-yl)propyl]piperazine;
(130) 1-[2-(4-(acetylamino)phenyl)ethyl]-4-[(R,S)-2-hydroxy-3-(5-(1,2,4-triazol-4-yl)-1H-indol-3-yl)propyl]piperazine;
(131) 1-[3-(5-(1,2,4-triazol-4-yl)-1H-indol-3-yl)propyl]-4-[2-(4-(aminocarbonylamino)phenyl)ethyl]piperazine;
(132) 1-[3-(5-(1,2,4-triazol-4-yl)-1H-indol-3-yl)propyl]-4-(4-cyanophenyl)methylpiperazine;
(133) 1-[3-(5-(1,2,4-triazol-4-yl)-1H-indol-3-yl)propyl]-4-[2-(4-cyanophenyl)ethyl]piperazine;
(134) 1-[3-(5-(1,2,4-triazol-4-yl)-1H-indol-3-yl)propyl]-4-[2-(4-(1,2,4-triazol-4-yl)phenyl)ethyl]piperazine;
(135) 1-[3-(5-(1,2,4-triazol-1-yl)-1H-indol-3-yl)propyl]-4-[2-(4-(acetylamino)phenyl)ethyl]piperazine;
(136) 1-[3-(5-(1,2,4-triazol-1-yl)-1H-indol-3-yl)propyl]-4-benzylpiperazine;
(137) 1-[3-(5-(1,2,4-triazol-1-ylmethyl)-1H-indol-3-yl)propyl]-4-benzylpiperazine;
(138) 4-(4-acetylaminophenyl)methyl-1-[3-(5-(1,2,4-triazol-4-yl)-1H-indol-3-yl)propyl]piperidine;
(139) 4-benzyl-1-[3-(5-(1,2,4-triazol-4-yl)-1H-indol-3-yl)propyl]-1,2,5,6-tetrahydropyridine;
(140) 1-[3-(5-(1,2,4-triazol-4-yl)-1H-indol-3-yl)propyl]-4-(2-aminopyridin-5-yl)methylpiperazine;
(141) 1-[3-(5-(1,2,4-triazol-4-yl)-1H-indol-3-yl)propyl]-4-(4-aminophenyl)methylpiperazine;
(142) 1-[4-(5-(1,2,4-triazol-4-yl)-1H-indol-3-yl)butyl]-4-benzylpiperazine;
(143) 1-[4-(5-(1,2,4-triazol-4-yl)-1H-indol-3-yl)butyl]-4-(pyridin-2-yl)methylpiperazine;
(144) 1-[4-(5-(1,2,4-triazol-4-yl)-1H-indol-3-yl)butyl]-4-(pyridin-3-yl)methylpiperazine;
(145) 1-[3-(5-(1,2,4-triazol-4-yl)-1H-indol-3-yl)propyl]-4-[2-(4-aminophenyl)ethyl]piperazine;
(146) 1-[3-(5-(1,2,4-triazol-4-yl)-1H-indol-3-yl)propyl]-4-[2-(4-(acetylamino)phenyl)ethyl]piperazine;
(147) 1-[3-(5-(1,2,4-triazol-4-yl)-1H-indol-3-yl)propyl]-4-(imidazol-2-yl)methylpiperazine;
(148) 1-[3-(5-(1,2,4-triazol-4-yl)-1H-indol-3-yl)propyl]-4-[3-(acetylamino)phenyl]methylpiperazine;
(149) 1-[3-(5-(1,2,4-triazol-4-yl)-1H-indol-3-yl)propyl]-4-[4-(acetylamino)phenyl]methylpiperazine;
(150) 1-[4-(5-(1,2,4-triazol-4-yl)-1H-indol-3-yl)butyl]-4-[4-(acetylamino)phenyl]methylpiperazine;
(151) 1-[3-(5-(1,2,4-triazol-4-yl)-1H-indol-3-yl)propyl]-4-(2-methoxyphenyl)methylpiperazine;
(152) 1-[3-(5-(1,2,4-triazol-4-yl)-1H-indol-3-yl)propyl]-4-benzylpiperazine; .
(153) 1-[3-(5-(1,2,4-triazol-4-yl)-1H-indol-3-yl)propyl]-4-(pyridin-3-yl)methylpiperazine;
(154) 1-[3-(5-(1,2,4-triazol-4-yl)-1H-indol-3-yl)propyl]-4-(pyridin-2-yl)methylpiperazine;
(155) 1-[3-(5-(1,2,4-triazol-4-yl)-1H-indol-3-yl)propyl]-4-(pyridin-4-yl)methylpiperazine;
(156) (3*R*)-3-benzyloxymethyl-1-[2-(5-(1,2,4-triazol-4-yl)-1*H*-indol-3-yl)ethyl]pyrrolidine;
(157) (3*S*)-3-(*N*-benzyl-*N*-methyl)aminomethyl-1-[2-(5-(1,2,4-triazol-4-yl)-1*H*-indol-3-yl)ethyl)pyrrolidine;
(158) (2*S*)-2-(*N*-benzyl-*N*-methyl)aminomethyl-1-[2-(5-(1,2,4-triazol-4-yl)-1*H*-indol-3-yl)ethyl]pyrrolidine;
(159) (3S)-3-(N-benzyl)aminomethyl-1-[2-(5-(1,2,4-triazol-4-yl)-1*H*-indol-3-yl)ethyl]pyrrolidine;
(160) 4-(4-acetylaminophenyl)methylamino-1-[3-(5-(1,2,4-triazol-4-yl)-1*H*-indol-3-yl)propyl]piperidine;
(161) 1-[3-(5-(imidazol-1-yl)-1*H*-indol-3-yl)propyl]-4-[(*R*)-α-(methoxymethyl)benzylamino]piperidine;
(162) 1-[3-(5-(1,2,4-triazol-1-ylmethyl)-1*H*-indol-3-yl)propyl]-4-[(*R*)-1-(4-fluorophenyl)-2-methoxyethylamino]piperidine;
(163) 1-[3-(5-(1,2,4-triazol-1-ylmethyl)-1*H*-indol-3-yl)propyl]-4-[*N*-(4-fluorobenzyl)-*N*-methylamino]piperidine;
(164) 1-[3-(5-(1,2,4-triazol-4-yl)-1*H*-indol-3-yl)propyl]-4-(2-phenylpiperidin-1-yl)piperidine;
(165) 1-[3-(5-(1,2,4-triazol-4-yl)-1*H*-indol-3-yl)propyl]-4-[(*R*)-1-(4-fluorophenyl)-2-methoxyethylamino]piperidine;
(166) 1-[3-(5-(1,2,4-triazol-4-yl)-1*H*-indol-3-yl)propyl]-4-(indan-1-ylaminomethyl)piperidine;
(167) 1-[3-(5-(1,2,4-triazol-4-yl)-1*H*-indol-3-yl)propyl]-4-[*N*-(*R*)-α-(hydroxymethyl)benzyl-*N*-methylaminomethyl]piperidine;
(168) (3R)-3-(benzylthio)methyl-1-[2-(5-(1,2,4-triazol-4-yl)-1*H*-indol-3-yl)ethyl]pyrrolidine;
(169) (±)-1-[3-(5-(1,2,4-triazol-4-yl)-1*H*-indol-3-yl)propyl]-4-(1-benzylamino-2-hydroxyethyl)piperidine;
(170) 1-[3-(5-(1,2,4-triazol-1-yl)-1*H*-indol-3-yl)propyl]-4-[(*R*)-α-(hydroxymethyl)benzylamino]piperidine;
(171) 1-[3-(5-(imidazol-1-yl)-1*H*-indol-3-yl)propyl]-4-[(*R*)-α-(methyl)benzylamino]piperidine;
(172) 1-[3-(5-(imidazol-1-yl)-1*H*-indol-3-yl)propyl]-4-[(*R*)-α-(hydroxymethyl)benzylamino]piperidine;
(173) 1-[3-(5-(1,2,4-triazol-1-ylmethyl)-1*H*-indol-3-yl)propyl]-4-[(*R*)-a-(hydroxymethyl)benzylamino]piperidine;
(174) 1-[3-(5-(1,2,4-triazol-4-yl)-1*H*-indol-3-yl)propyl]-4-[(*R*)-α-(methoxymethyl)benzylamino]piperidine;
(175) 1-[3-(5-(1,2,4-triazol-4-yl)-1*H*-indol-3-yl)propyl]-4-[*N*-(*R*)-α-(methoxymethyl)benzyl-*N*-methylamino]piperidine;
(176) (3*R*)-3-benzyloxy-1-[2-(5-(1,2,4-triazol-4-yl)-1*H*-indol-3-yl)ethyl]pyrrolidine;
(177) (3*R*)-3-(4-methoxyphenyl)methoxy-1-[2-(5-(1,2,4-triazol-4-yl)-1*H*-indol-3-yl)ethyl]pyrrolidine;
(178) (3R)-3-(pyridin-3-yl)methoxy-1-[2-(5-(1,2,4-triazol-4-yl)-1*H*-indol-3-yl)ethyl]pyrrolidine;
(179) (3*R*)-3-benzyloxymethyl-1-[2-(5-(1,2,4-triazol-4-yl)-1*H*-indol-3-yl)ethyl]pyrrolidine;
(180) (3S)-3-(*N*-benzyl-*N*-methyl)aminomethyl-1-[2-(5-(1,2,4-triazol-4-yl)-1*H*-indol-3-yl)ethyl]pyrrolidine;
(181) (2S)-2-(*N*-benzyl-*N*-methyl)aminomethyl-1-[2-(5-(1,2,4-triazol-4-yl)-1*H*-indol-3-yl)ethyl]pyrrolidine;
(182) (3*S*)-3-(*N*-benzyl)aminomethyl-1-[2-(5-(1,2,4-triazol-4-yl)-1*H*-indol-3-yl)ethyl]pyrrolidine;
(183) 4-(4-acetylaminophenyl)methylamino-1-[3-(5-(1,2,4-triazol-4-yl)-1*H*-indol-3-yl)propyl]piperidine;
(184) 4-benzylamino-1-[3-(5-(1,2,4-triazol-4-yl)-1*H*-indol-3-yl)propyl]piperidine;
(185) 4-(*N*-benzyl-*N*-methyl)amino-1-[3-(5-(1,2,4-triazol-4-yl)-1*H*-indol-3-yl)propyl]piperidine;
(186) 4-(*N*-benzyl-*N*-methyl)aminomethyl-1-[3-(5-(1,2,4-triazol-4-yl)-1*H*-indol-3-yl)propyl]piperidine;
(187) 1-[3-(5-(1,2,4-triazol-4-yl)-1*H*-indol-3-yl)propyl]-4-[α-(methyl)benzylamino]piperidine;
(188) 1-[3-(5-(1,2,4-triazol-4-yl)-1*H*-indol-3-yl)propyl]-4-[α-(hydroxymethyl)benzylamino]piperidine;
(189) 1-[3-(5-(1,2,4-triazol-4-yl)-1*H*-indol-3-yl)propyl]-4-[(1-hydroxymethyl-2-phenyl)ethylamino]piperidine;
(190) 4-(*N*-benzyl)aminomethyl-1-[3-(5-(1,2,4-triazol-4-yl)-1*H*-indol-3-yl)propyl]piperidine;
(191) 1-[3-(5-(1,2,4-triazol-4-yl)-1*H*-indol-3-yl)propyl]-4-[(2-hydroxy-1-methyl-2-phenyl)ethylamino]piperidine;
(192) 1-[3-(5-(1,2,4-triazol-4-yl)-1*H*-indol-3-yl)propyl]-4-[2-(4-acetylaminophenyl)ethylamino]piperidine;
(193) 1-[3-(5-(1,2,4-triazol-4-yl)-1*H*-indol-3-yl)propyl]-4-[α-(methyl)benzylamino]methylpiperidine;
(194) 1-[3-(5-(1,2,4-triazol-4-yl)-1*H*-indol-3-yl)propyl]-4-[1-(4-acetylaminophenyl)ethylamino]methylpiperidine;
(195) 1-[3-(5-(1,2,4-triazol-4-yl)-1*H*-indol-3-yl)propyl]-4-[*N*-[α-(hydroxymethyl)benzyl]-*N*-methylamino]piperidine;
(196) 1-[3-(5-(1,2,4-triazol-4-yl)-1*H*-indol-3-yl)propyl]-4-[*N*-(2-(4-acetylaminophenyl)ethyl)-*N*-methylamino]piperidine;
(197) 1-[3-(5-(1,2,4-triazol-4-yl)-1*H*-indol-3-yl)propyl]-4-[*N*-(4-acetylaminobenzyl)-*N*-methylamino]methylpiperidine;
(198) 1-[3-(5-(1,2,4-triazol-4-yl)-1*H*-indol-3-yl)propyl]-4-[*N*-(thien-2-yl)methyl-N-methylamino]piperidine;
(199) 1-[3-(5-(1,2,4-triazol-4-yl)-1*H*-indol-3-yl)propyl]-4-[(*R*)-α-(hydroxymethyl)benzylamino]methylpiperidine;
(200) 3-(4-acetylaminobenzyl)aminomethyl-1-[2-(5-(1,2,4-triazol-4-yl)-1*H*-indol-3-yl)ethyl]pyrrolidine;
(201) (3*R*)-3-(N-benzyl)aminomethyl-1-[2-(5-(1,2,4-triazol-4-yl)-1*H*-indol-3-yl)ethyl]pyrrolidine;
(202) (3*S*)-3-(pyridin-4-ylmethyl)aminomethyl-1-[2-(5-(1,2,4-triazol-4-yl)-1*H*-indol-3-yl)ethyl]pyrrolidine;
(203) 3-(*N*-benzyl)aminomethyl-1-[2-(5-(1,2,4-triazol-4-yl)-1*H*-indol-3-yl)ethyl]azetidine;
(204) 4-benzyl-4-hydroxy-1-[3-(5-(1,2,4-triazol-4-yl)-1*H*-indol-3-yl)propyl]piperidine;
(205) 3-(*N*-benzyl)aminomethyl-1-[3-(5-(1,2,4-triazol-4-yl)-1*H*-indol-3-yl)propyl]azetidine;
(206) 4-(*N*-benzyl)aminomethyl-4-hydroxy-1-[3-(5-(1,2,4-triazol-4-yl)-1*H*-indol-3-yl)propyl]piperidine;
(207) 4-(*N*-benzyl-*N*-methyl)aminomethyl-4-hydroxy-1-[3-(5-(1,2,4-triazol-4-yl)-1*H*-indol-3-yl)propyl]piperidine;
(208) 3-(*N*-benzyl-*N*-methyl)aminomethyl-1-[2-(5-(1,2,4-triazol-4-yl)-1*H*-indol-3-yl)ethyl]azetidine;
(209) (3*S*)-3-[*N*-α-(methyl)benzyl]aminomethyl-1-[2-(5-(1,2,4-triazol-4-yl)-1*H*-indol-3-yl)ethyl]pyrrolidine;
(210) (3*S*)-3-[*N*-(furan-3-ylmethyl)amino]methyl-1-[2-(5-(1,2,4-triazol-4-yl)-1*H*-indol-3-yl)ethyl]pyrrolidine;
(211) (3*S*)-3-[*N*-(furan-2-ylmethyl)amino]methyl-1-[2-(5-(1,2,4-triazol-4-yl)-1*H*-indol-3-yl)ethyl]pyrrolidine;
(212) (3*S*)-3-[*N*-α-(hydroxymethyl)benzyl)aminomethyl-1-[2-(5-(1,2,4-triazol-4-yl)-1*H*-indol-3-yl)ethyl]pyrrolidine;
(213) (3S)-3-[*N*-benzyl-*N*-(2-hydroxy)ethyl]aminomethyl-1-[2-(5-(1,2,4-triazol-4-yl)-1*H*-indol-3-yl)ethyl]pyrrolidine;
(214) (3*S*)-3-[*N*-(2-phenylethyl)amino]methyl-1-[2-(5-(1,2,4-triazol-4-yl)-1*H*-indol-3-yl)ethyl]pyrrolidine;
(215) (3*S*)-3-[*N*-(2-phenylethyl)-*N*-methylamino]methyl-1-[2-(5-(1,2,4-triazol-4-yl)-1*H*-indol-3-yl)ethyl]pyrrolidine;
(216) (3*S*)-3-(*N*-α-dimethylbenzyl)aminomethyl-1-[2-(5-(1,2,4-triazol-4-yl)-1*H*-indol-3-yl)ethyl]pyrrolidine;
(217) (3*S*)-3-[*N*-(*S*)-α-methylbenzyl]aminomethyl-1-[2-(5-(1,2,4-triazol-1-yl)-1*H*-indol-3-yl)ethyl]pyrrolidine;
(218) (3*S*)-3-[*N*-(*R*)-α-(hydroxymethyl)benzyl]aminomethyl-1-[2-(5-(1,2,4-triazol-1-yl)-1*H*-indol-3-yl)ethyl]pyrrolidine;
(219) (3*S*)-3-(*N*-benzyl)aminomethyl-1-[2-(5-(1,2,4-triazol-1-ylmethyl)-1*H*-indol-3-yl)ethyl]pyrrolidine;
(220) (3*S*)-3-[*N*-(*S*)-α-methylbenzyl]aminomethyl-1-[2-(5-(1,2,4-triazol-1-ylmethyl)-1*H*-indol-3-yl)ethyl]pyrrolidine;
(221) (3*S*)-3-[*N*-(*R*)-α-(hydroxymethyl)benzyl]aminomethyl-1-[2-(5-(1,2,4-triazol-1-ylmethyl)-1*H*-indol-3-yl)ethyl]pyrrolidine;
(222) (3*S*)-3-(*N*-benzyl-*N*-methyl)aminomethyl-1-[2-(5-(imidazol-1-yl)-1*H*-indol-3-yl)ethyl]pyrrolidine;
(223) (3*S*)-3-(*N*-benzyl-*N*-methyl)aminomethyl-1-[2-(5-(1,2,4-triazol- 1-ylmethyl)-1*H*-indol-3-yl)ethyl]pyrrolidine;
(224) (3*R*)-3-[*N*-methyl-*N*-(*S*)-α-methylbenzyl]aminomethyl-1-[2-(5-(1,2,4-triazol-1-ylmethyl)-1*H*-indol-3-yl)ethyl]pyrrolidine;
(225) (3*R*)-3-[*N*-methyl-*N*-(*R*)-α-hydroxymethylbenzyl]aminomethyl-1-[2-(5-(1,2,4-triazol-1-ylmethyl)-1*H*-indol-3-yl)ethyl]pyrrolidine;
(226) (3*R*)-3-[*N*-methyl-*N*-(*S*)-α-methylcyclohexylmethyl]aminomethyl-1-[2-(5-(1,2,4-triazol-1-ylmethyl)-1*H*-indol-3-yl)ethyl]pyrrolidine;
(227) (3*R*)-3-[3-(*R*)-hydroxy-2-(*R*)-phenylpiperidin-1-yl]methyl-1-[2-(5-(1,2,4-triazol-1-ylmethyl)-1*H*-indol-3-yl)ethyl]pyrrolidine;
(228) (3*R*)-3-[3-(*R*)-hydroxy-2-(*R*)-phenylpiperidin-1-yl]methyl-1-[2-(5-(1,2,4-triazol-1-yl)-1*H*-indol-3-yl)ethyl]pyrrolidine;
(229) 4-hydroxy-4-(phenylsulfinyl)methyl-1-[3-(5-( 1,2,4-triazol-4-yl)-1*H*-indol-3-yl)propyl]piperidine;
(230) (3*R*)-3-[2-(*R,S*)-phenylpiperidin-1-yl]methyl-1-[2-(5-(1,2,4-triazol-1-ylmethyl)-1*H*-indol-3-yl)ethyl]pyrrolidine;
(231) 4-(3,3-dimethylpiperidin-1-yl)methyl-4-hydroxy-1-[3-(5-(1,2,4-triazol-4-yl)-1*H*-indol-3-yl)propyl]piperidine;
(232) 4-hydroxy-4-(1,2,3,4-tetrahydroisoquinolin-2-yl)methyl-1-[3-(5-(1,2,4-triazol-4-yl)-1*H*-indol-3-yl)propyl]piperidine;
(233) 4-hydroxy-4-(*N*-isobutyl-*N*-methyl)aminomethyl-1-[3-(5-(1,2,4-triazol-4-yl)-1*H*-indol-3-yl)propyl]piperidine;
(234) 4-[*N*-benzyl-*N*-(2-hydroxyethyl)amino]methyl-4-hydroxy-1-[3-(5-(1,2,4-triazol-4-yl)-1*H*-indol-3-yl)propyl]piperidine;
(235) 4-[*N*-(2,2-dimethylpropyl)-*N*-methylamino]methyl-4-hydroxy-1-[3-(5-(1,2,4-triazol-4-yl)-1*H*-indol-3-yl)propyl]piperidine;
(236) 4-[*N*-(*R*)-α-hydroxymethylbenzyl-*N*-methylamino]methyl-4-hydroxy-1-[3-(5-(1,2,4-triazol-4-yl)-1*H*-indol-3-yl)propyl]piperidine;
(237) 4-hydroxy-4-(2-pyridylmethyl)aminomethyl-1-[3-(5-(1,2,4-triazol-4-yl)-1*H*-indol-3-yl)propyl]piperidine;
(238) 4-hydroxy-4-(2-methylphenylmethyl)aminomethyl-1-[3-(5-(1,2,4-triazol-4-yl)-1*H*-indol-3-yl)propyl]piperidine;
(239) 4-hydroxy-4-[*N*-(2-methylphenylmethyl)-N-methylamino]methyl-1-[3-(5-(1,2,4-triazol-4-yl)-1*H*-indol-3-yl)propyl]piperidine;
(240) 3-(benzylamino)methyl-3-hydroxy-1-[3-(5-(1,2,4-triazol-4-yl)-1*H*-indol-3-yl)propyl]pyrrolidine;
(241) 3-(benzylamino)methyl-3-hydroxy-1-[2-(5-(1,2,4-triazol-4-yl)-1*H*-indol-3-yl)ethyl]pyrrolidine;
(242) 1-[3-(5-(1,2,4-triazol-4-yl)-1*H*-indol-3-yl)propyl]-4-[(*R*)-α-(carbamoyl-oxymethyl)benzylamino]piperidine;
(243) 1-[3-(5-(1,2,4-triazol-4-yl)-1*H*-indol-3-yl)propyl]-4-[(1*R*,2*S*)-2-hydroxy-1-phenylpropylamino]piperidine;
(244) 1-[3-(5-(1,2,4-triazol-4-yl)-1*H*-indol-3-yl)propyl]-4-[(1*R*,2*R*)-2-hydroxy-1-phenylpropylamino]piperidine;
(245) 1-[3-(5-(1,2,4-triazol-4-yl)-1*H*-indol-3-yl)propyl]-4-[(*R,S*)-1-hydroxy-2-phenylprop-2-ylamino]piperidine;
(246) 1-[3-(5-(1,2,4-triazol-4-yl)-1*H*-indol-3-yl)propyl]-4-[(*R*)-2-hydroxy-1-(4-fluorophenyl)ethylamino]piperidine;
(247) 1-[3-(5-(1,2,4-triazol-4-yl)-1*H*-indol-3-yl)propyl]-4-[(1*R*,2*R*)-2-hydroxyindan-1-ylamino)piperidine;
(248) 1-[3-(5-(1,2,4-triazol-4-yl)-1*H*-indol-3-yl)propyl]-4-[(R,S)-indan-1-ylamino]piperidine;
(249) 1-[3-(5-(1,2,4-triazol-4-yl)-1*H*-indol-3-yl)propyl]-4-[(*R,S*)-1-(4-fluorophenyl)ethylamino]piperidine;
(250) 1-[3-(5-(1,2,4-triazol-4-yl)-1*H*-indol-3-yl)propyl]-4-[(*R*)-1-phenylprop-2-ylamino]piperidine;
(251) 1-[3-(5-(1,2,4-triazol-4-yl)-1*H*-indol-3-yl)propyl]-4-[*N*-(thien-3-ylmethyl)-N-methylamino)piperidine;
(252) 1-[3-(5-(1,2,4-triazol-4-yl)-1*H*-indol-3-yl)propyl]-4-[*N*-(furan-3-ylmethyl)-*N*-methylamino]piperidine;
(253) 1-[3-(5-(1,2,4-triazol-4-yl)-1*H*-indol-3-yl)propyl]-4-(furan-3-ylmethyl)aminopiperidine;
(254) 1-[3-(5-(1,2,4-triazol-4-yl)-1*H*-indol-3-yl)propyl]-4-[*N,N*-di(furan-3-ylmethyl)amino]piperidine;
(255) 1-[3-(5-(1,2,4-triazol-4-yl)-1*H*-indol-3-yl)propyl]-4-[*N*-(3,3-dimethylallyl)-*N*-methylamino]piperidine;
(256) 1-[3-(5-(1,2,4-triazol-4-yl)-1*H*-indol-3-yl)propyl]-4-(*N*-allyl-*N*-methylamino)piperidine;
(257) N,N-Dimethyl-2-[5-(1,2,4-triazol-1-ylmethyl)-1H-indol-3-yl]ethylamine;
(258) N,N-Dimethyl-2-[5-(1,3-imidazol-1-ylmethyl)-1H-indol-3-yl]ethylamine;
(259) N,N-Dimethyl-2-[5-(5-methyl-1,2,3,4-triazol-1-ylmethyl)-1H-indol-3-yl]ethylamine;
(260) N,N-Dimethyl-2-[5-(1,3,4-triazol-1-ylmethyl)-1H-indol-3-yl]ethylamine;
(261) N,N-Dimethyl-2-[5-(1,3,4-triazol-1-yl)-1H-indol-3-yl]ethylamine;
(262) N,N-Diethyl-2-[5-(1,2,4-triazol-1-ylmethyl)-1H-indol-3-yl]ethylamine;
(263) N,N-Diethyl-2-[5-(1,3-imidazol-1-ylmethyl)-1H-indol-3-yl]ethylamine;
(264) N,N-Diethyl-2-[5-(5-methyl-1,2,3,4-tetrazol-1-ylmethyl)-1H-indol-3-yl]ethylamine;
(265) N,N-Diethyl-2-[5-(1,3,4-triazol-1-ylmethyl)-1H-indol-3-yl]ethylamine;
(266) N,N-Diethyl-2-[5-(1,3,4-triazol-1-yl)-1H-indol-3-yl]ethylamine;
(267) N,N-Dimethyl-[5-(1,2,4-triazol-1-ylmethyl)-1H-indol-3-yl]methylamine;
(268) N,N-Dimethyl-[5-(1,3-imidazol-1-ylmethyl)-1H-indol-3-yl]methylamine;
(269) N,N-Dimethyl-[5-(5-methyl-1,2,3,4-tetrazol-1-ylmethyl)-1H-indol-3-yl]methylamine;
(270) N,N-Dimethyl-[5-(1,3,4-triazol-1-ylmethyl)-1H-indol-3-yl]methylamine;
(271) N,N-Dimethyl-[5-(1,3,4-triazol-1-yl)-1H-indol-3-yl]methylamine;
(272) N,N-Diethyl-3-[5-(1,2,4-triazol-1-ylmethyl)-1H-indol-3-yl]propylamine;
(273) N,N-Dimethyl-3-[5-(1,3-imidazol-1-yl)-1H-indol-3-yl]propylamine;
(274) N,N-Diethyl-3-[5-(5-methyl-1,2,3,4-tetrazol-1-ylmethyl)-1H-indol-3-yl]propylamine;
(275) N,N-Dimethyl-3-[5-(1,3,4-triazol-1-ylmethyl)-1H-indol-3-yl]propyl-amine;
(276) N,N-Diethyl-3-[5-(1,3,4-triazol-1-yl)-1H-indol-3-yl]propylamine;
(277) N,N-Dimethyl-4-[5-(3-methyl-1,2,4,5-tetrazol-1-ylmethyl)-1H-indol-3-yl]butylamine;
(278) N,N-Dimethyl-4-[5-(2-ethyl-1,3-ethyl-imidazol-1-ylmethyl)-1H-indol-3-yl]butylamine;
(279) N,N-Dimethyl-4-[5-(5-ethyl-1,2,3,4-triazol-1-ylmethyl)-1H-indol-3-yl]butylamine;
(280) N,N-Dimethyl-4-[5-(2-methyl-1,3,4-triazol-1-ylmethyl)1H-indol-3-yl]butylamine;
(281) N,N-Dimethyl-4-[5-(2-ethyl-1,3,4-triazol-1-yl)-1H-indol-3-yl]butylamine;
(282) 2-[5-(1,2,4-triazol-1-ylmethyl)-1H-indol-3-yl]ethylalcohol;
(283) 2-[5-(1,3-imidazol-1-ylmethyl)-1H-indol-3-yl]ethylalcohol;
(284) 2-[5-(5-methyl-1,2,3,4-tetrazol-1-ylmethyl)-1H-indol-3-yl]ethylalcohol;
(285) 2-[5-(1,3,4-triazol-1-ylmethyl)-1H-indol-3-yl]ethylalcohol;
(286) 2-[5-(1,3,4-triazol-1-yl)-1H-indol-3-yl]ethylalcohol;
(287) [5-(1,2,4-triazol-1-ylmethyl)-1H-indol-3-yl]-methylalcohol;
(288) 3-[5-(1,3-imidazol-1-ylmethyl)-1H-indol-3-yl]propylalcohol;
(289) 4-[5-(5-methyl-1,2,3,4-tetrazol-1-ylmethyl)-1H-indol-3-yl]butylalcohol;
(290) 2-[5-(2-methyl-1,3,4-triazol-1-ylmethyl)-1H-indol-3-yl]ethylalcohol; and
(291) 2-[5-(5-methyl-1,3,4-triazol-1-yl)-1H-indol-3-yl]ethylalcohol.

20. The process according to Claim 18 wherein the compound of structural formula IV is selected from: 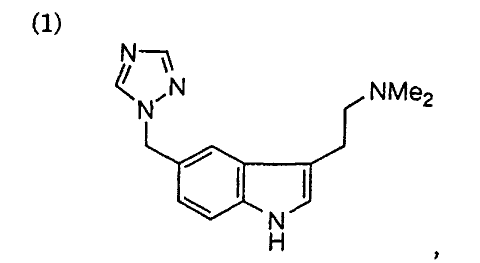 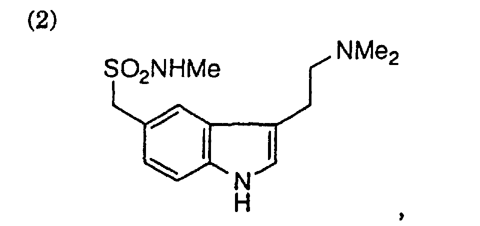 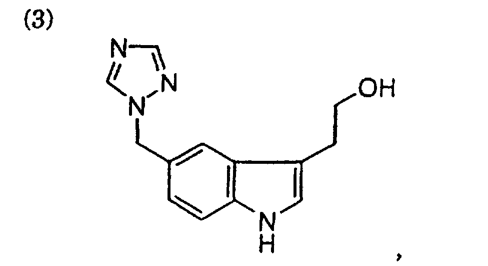 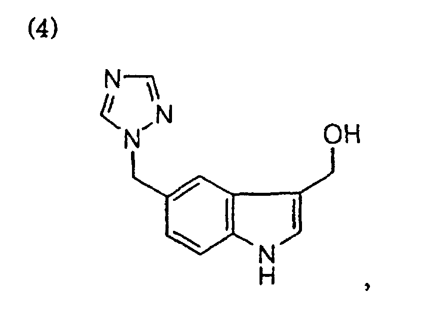 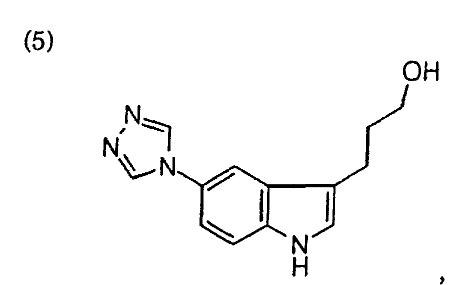 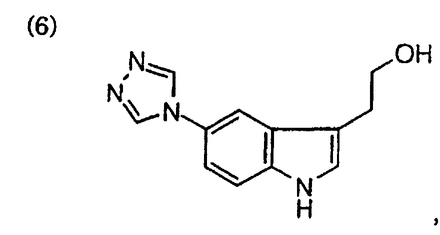 and

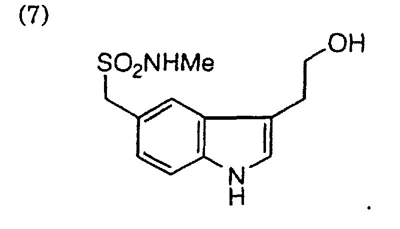

21. The compounds of structural formulae (V) and (VI): 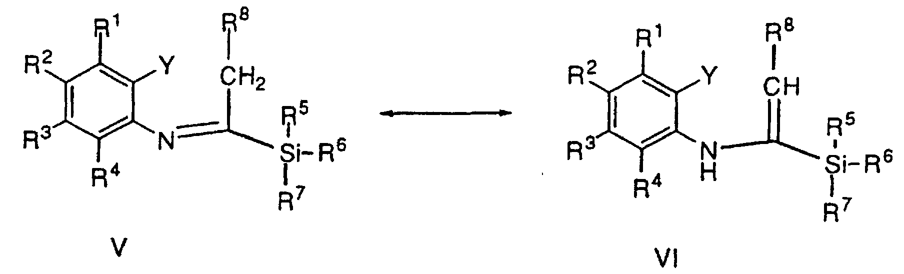 wherein:
Y is selected from Br, I and triflate;
R¹, R², R³ and R⁴ are each independently selected from:
(1) hydrogen;
(2) 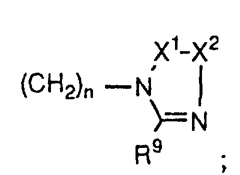
(3) C₁₋₆ alkyl;
(4) -(CH₂)ₙ-Z
wherein Z represents:
(a) hydrogen,
(b) halogen,
(c) cyano,
(d) nitro,
(e) trifluoromethyl,
(f) OR¹⁰,
(g) OCOR¹⁰,
(h) OCONR¹⁰R¹¹,
(i) OCH₂CN,
(j) OCH₂CONR¹⁰R¹¹,
(k) SR¹⁰,
(l) SOR¹⁰,
(m) SO₂R¹⁰, provided that R¹⁰ is not hydrogen,
(n) SO₂NR¹⁰R¹¹,
(o) NR¹⁰R¹¹,
(p) NR¹⁰COR¹¹,
(q) NR¹⁰CO₂R¹¹,
(r) NR¹⁰SO₂R¹¹,
(s) COR¹⁰,
(t) CO₂R¹⁰,
(u) CONR¹⁰R¹¹,
or Z is a group of formula (Za), (Zb), (Zc), or (Zd): 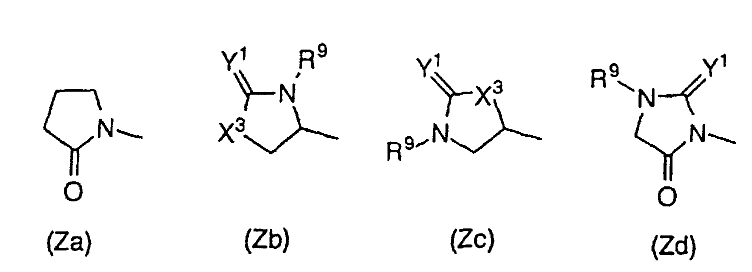
or Z represents an optionally substituted five-membered heteroaromatic ring selected from furan, thiophene, pyrrole, oxazole, thiazole, isoxazole, isothiazole, imidazole, pyrazole, oxadiazole, thiadiazole, triazole and tetrazole;
R⁵, R⁶, and R⁷ are each independently selected from:
(1) C₁₋₆ alkyl,
(2) -O-C₁₋₆ alkyl, and
(3) phenyl;
R⁸ is selected from:
(1) hydrogen,
(2) -R¹⁹-OH,
(3) -R¹⁹-O-R¹⁷, and
(4) -R¹⁹NR¹²R¹³, and
(5) -R¹⁹-Z¹
wherein: Z¹ is a 3 to 7 membered heterocyclic ring wherein the ring members are selected from 1 to 2 nitrogen atoms and wherein the heterocyclic ring may be subsituted by one or more R¹⁴;
R⁹ is selected from:
(1) hydrogen, and
(2) C₁₋₄ alkyl;
R¹⁰ and R¹¹ are each independently selected from:
(1) hydrogen,
(2) C₁₋₆ alkyl,
(3) trifluoromethyl,
(4) phenyl, optionally substituted with one or more R²⁰ substituents
(5) methylphenyl, optionally substituted with one or more R²⁰ substituents, and
(6) an arylC₁₋₆alkyl or heteroaryl C₁₋₆alkyl group. optionally substituted with one or more R²⁰ substituents, or
R¹⁰ and R¹¹ when linked through a nitrogen atom, together represent the residue of an optionally substituted azetidine, pyrrolidine, piperidine, morpholine or piperazine ring, optionally substituted with one or more R¹⁸ substituents;
R¹² and R¹³ are each independently selected from:
(1) C₁₋₄ alkyl,
(2) C₁₋₄ alkyl-C₆aryl wherein aryl may be unsubstituted or substituted with one to three substituents selected from methyl, halo, and halomethyl,
R¹⁴ is selected from:
(1) aryl-C₁₋₆alkyl, unsubstituted or substituted with one to three R²⁰ substitutents, and
(2) heteroaryl-C₁₋₆alkyl, unsubstituted or substituted with one to three R²⁰ substitutents,
R¹⁵ and R¹⁶ are each independently selected from
(1) hydrogen,
(2) C₁₋₆alkyl,
(3) C₃₋₇cycloalkyl,
(4) C₃₋₇cycloalkylC₁₋₆alkyl,
(5) indanyl,
(6) aryl,
(7) arylC₁₋₆alkyl,
(8) C₃₋₇heterocycloalkyl,
(9) C₃₋₇heterocycloalkylC₁₋₆alkyl,
(10) heteroaryl, and
(11) heteroarylC₁₋₆alkyl;
R¹⁷ is selected from a hydroxy protecting group that is removable under mild acid hydrolysis;
R¹⁸ is selected from:
(1) C₁₋₆alkyl,
(2) arylC₁₋₆alkyl,
(3) C₁₋₆alkoxy,
(4) C₂₋₆alkyoxycarbonyl, and
(5) C₁₋₆alkylaminocarbonyl;
R¹⁹ is a straight or branched C₁₋₆alkyl chain;
R²⁰ is selected from:
(1) fluoro,
(2) cyano,
(3) trifluoromethyl,
(4) C₁₋₆alkyl,
(5) haloC₁₋₆alkyl,
(6) aryl,
(7) triazolyl,
(8) tetrazolyl,
(9) C₁₋₆alkyl-tetrazolyl,
(10) hydroxy,
(11) C₁₋₆alkoxy,
(12) C₁₋₆alkylthio,
(13) C₂₋₆alkoxycarbonyl,
(14) C₂₋₆alkylcarbonyl,
(15) C₁₋₆alkylsulphonyl,
(16) arylsulfonyl,
(17) C₂₋₆alkylcarbonylamino,
(18) arylcarbonylamino,
(19) C₂₋₆alkoxycarbonylamino,
(20) N-C₁₋₆alkyl-N-C₂₋₆alkoxyamino,
(21) carbonylamino,
(22) mono- or diarylaminocarbonylamino,
(23) pyrrolidinylcarbonylamino,
(24) piperidinylcarbonylamino,
(25) aminocarbonyl,
(26) aminocarbonylamino,
(27) C₁₋₆alkylaminocarbonyl,
(28) C₁₋₆alkylaminocarbonylamino,
(29) diC₁₋₆alkylaminocarbonyl,
(30) diC₁₋₆alkylaminocarbonylamino,
(31) pyrrolidinylcarbonylamino,
(32) piperidinylcarbonylamino,
(33) aminosulfonyl,
(34) C₁₋₆alkylaminosulfonyl,
(35) C₁₋₆alkylsulfonylamino,
(36) C₁₋₆alkylsulfonylaminomethyl,
(37) arylsulfonylamino,
(38) diC₁₋₆alkylaminosulfonyl,
(39) aminosulphonylmethyl,
(40) C₁₋₆alkylaminosulfonylmethyl, and
(41) diC₁₋₆alkylaminosulfonylmethyl,
(42) (CH₂)ₘOR¹⁵,
(43) (CH₂)ₘSR¹⁵, provided that R¹⁵ is not hydrogen,
(44) (CH₂)ₘSOR¹⁵,
(45) (CH₂)ₘSO₂R¹⁵,
(46) (CH₂)ₘNR¹⁵R¹⁶,
(47) =O, and
(48) 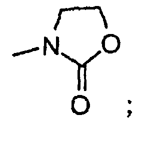
X¹ and X² are each independently selected from ring nitrogen or ring carbon atoms;
X³ is selected from the group consisting of oxygen, sulfur, -NH- or methylene;
Y¹ is oxygen or sulfur;
n is an integer independently selected at each occurrence from 0 and 1; and
m is an integer selected independently at each occurrence from 0 to 4.

## Patentansprüche

1. Ein Verfahren zur Herstellung einer Verbindung der Strukturformel III: 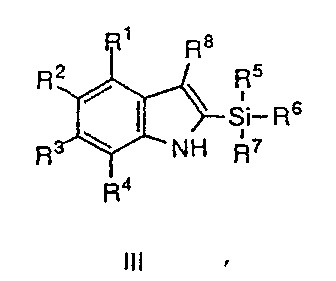 umfassend die Umsetzung einer Verbindung der Strukturformel I mit einem. Acylsilan der Strukturformel II: 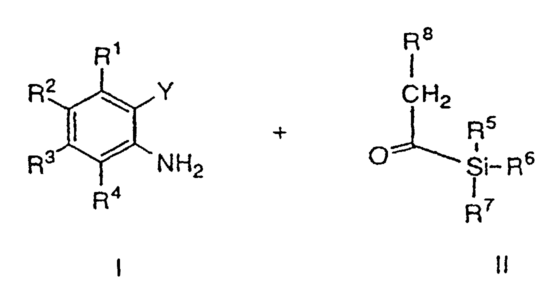 in Gegenwart eines Palladiumkatalysators und eines Protonenakzeptors,
wobei:
Y ausgewählt ist aus Br, I und Triflat und
R¹, R², R³ und R⁴ jeweils unabhängig ausgewählt sind aus:
(1) Wasserstoff,
(2) 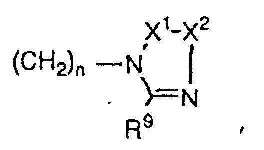
(3) C₁₋₆-Alkyl,
(4) -(CH₂)ₙ-Z,
wobei Z bedeutet:
(a) Wasserstoff,
(b) Halogen,
(c) Cyano,
(d) Nitro,
(e) Trifluormethyl,
(f) -OR¹⁰,
(g) -OCOR¹⁰,
(h) -OCONR¹⁰R¹¹,
(i) -OCH₂CN,
( j ) -OCH₂CONR¹⁰R¹¹,
(k) -SR¹⁰,
(l) -SOR¹⁰,
(m) -SO₂R¹⁰,
(n) -SO₂NR¹⁰R¹¹,
(o) -NR¹⁰R¹¹,
(p) -NR¹⁰COR¹¹,
(q) -NR¹⁰CO₂R¹¹,
(r) -NR¹⁰SO₂R¹¹,
(s) -COR¹⁰,
(t) -CO₂R¹⁰,
(u) -CONR¹⁰R¹¹,
oder eine Gruppe der Formel (Za), (Zb), (Zc) oder (Zd): 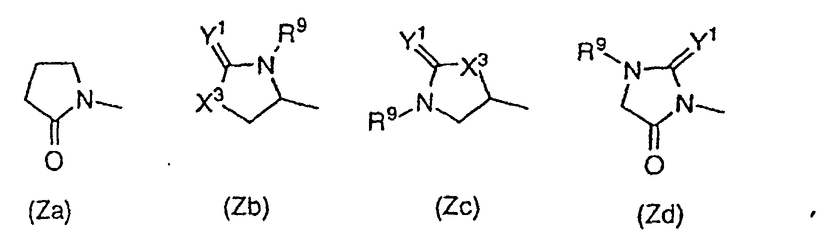 oder wobei Z einen gegebenenfalls substituierten fünfgliedrigen heteroaromatischen Ring, ausgewählt aus Furan, Thiophen, Pyrrol, Oxazol, Thiazol, Isoxazol, Isothiazol, Imidazol, Pyrazol, Oxadiazol, Thiadiazol, Triazol und Tetrazol, bedeutet,
R⁵, R⁶ und R⁷ jeweils unabhängig ausgewählt sind aus:
(1) C₁₋₆-Alkyl,
(2) -O-C₁₋₆-Alkyl und
(3) Phenyl,
R⁸ ausgewählt ist aus:
(1) Wasserstoff,
(2) -R¹⁹-OH,
(3) -R¹⁹-O-R¹⁷ und
(4) -R¹⁹NR¹²R¹³ und
(5) -R¹⁹-Z¹,
wobei: Z¹ ein 3- bis 7gliedriger heterocyclischer Ring ist, wobei die Ringelemente ausgewählt sind aus 1 bis 2 Stickstoffatomen, und wobei der heterocyclische Ring durch einen oder mehrere Reste R¹⁴ substituiert sein kann,
R⁹ ausgewählt ist aus:
(1) Wasserstoff und
(2) C₁₋₄-Alkyl,
R¹⁰ und R¹¹ jeweils unabhängig ausgewählt sind aus:
(1) Wasserstoff,
(2) C₁₋₆-Alkyl,
(3) Trifluormethyl,
(4) Phenyl, gegebenenfalls substituiert mit einem oder mehreren R²⁰-Substituenten,
(5) Methylphenyl, gegebenenfalls substituiert mit einem oder mehreren R²⁰-Substituenten, und
(6) einer Aryl-C₁₋₆-alkyl- oder Heteroaryl-C₁₋₆-alkylgruppe, gegebenenfalls substituiert mit einem oder mehreren R²⁰-Substituenten, oder
R¹⁰ und R¹¹, wenn sie durch ein Stickstoffatom verbunden sind, zusammen den Rest eines gegebenenfalls substituierten Azetidin-, Pyrrolidin-, Piperidin-, Morpholin- oder Piperazinrings bedeuten, der gegebenenfalls mit einem oder mehreren R¹⁸-Substituenten substituiert ist,
R¹² und R¹³ jeweils unabhängig ausgewählt sind aus:
(1) C₁₋₄-Alkyl,
(2) C₆-Aryl-C₁₋₄-alkyl-, wobei das Aryl unsubstituiert oder mit einem bis drei Substituenten, ausgewählt aus Methyl, Halogen und Halogenmethyl, substituiert sein kann,
R¹⁴ ausgewählt ist aus:
(1) Aryl-C₁₋₆-alkyl-, unsubstituiert oder substituiert mit einem bis drei R²⁰-Substituenten, und
(2) Heteroaryl-C₁₋₆-alkyl-, unsubstituiert oder substituiert mit einem bis drei R²⁰-Substituenten,
R¹⁵ und R¹⁶ jeweils unabhängig ausgewählt sind aus
(1) Wasserstoff,
(2) C₁₋₆-Alkyl,
(3) C₃₋₇-Cycloalkyl,
(4) C₃₋₇-Cycloalkyl-C₁₋₆-alkyl-,
(5) Indanyl,
(6) Aryl,
(7) Aryl-C₁₋₆-alkyl-,
(8) C₃₋₇-Heterocycloalkyl-,
(9) C₃₋₇-Heterocycloalkyl-C₁₋₆-alkyl-,
(10) Heteroaryl und
(11) Heteroaryl-C₁₋₆-alkyl-,
R¹⁷ ausgewählt ist aus einer Hydroxyschutzgruppe, die unter milder Säurehydrolyse entfernt werden kann,
R¹⁸ ausgewählt ist aus:
(1) C₁₋₆-Alkyl-,
(2) Aryl-C₁₋₆-alkyl-,
(3) C₁₋₆-Alkoxy-,
(4) C₂₋₆-Alkyloxycarbonyl- und
(5) C₁₋₆-Alkylaminocarbonyl-,
R¹⁹ eine gerade oder verzweigte C₁₋₆-Alkylkette ist,
R²⁰ ausgewählt ist aus:
(1) Fluor,
(2) Cyano,
(3) Trifluormethyl,
(4) C₁₋₆-Alkyl,
(5) Halogen-C₁₋₆-alkyl-,
(6) Aryl,
(7) Triazolyl,
(8) Tetrazolyl,
(9) Tetrazolyl-C₁₋₆-alkyl-,
(10) Hydroxy,
(11) C₁₋₆-Alkoxy-,
(12) C₁₋₆-Alkylthio-,
(13) C₂₋₆-Alkoxycarbonyl-,
(14) C₂₋₆-Alkylcarbonyl-,
(15) C₁₋₆-Alkylsulfonyl-,
(16) Arylsulfonyl-,
(17) C₂₋₆-Alkylcarbonylamino-,
(18) Arylcarbonylamino-,
(19) C₂₋₆-Alkoxycarbonylamino-,
(20) N-C₁₋₆-Alkyl-N-C₂₋₆-alkoxyamino-,
(21) Carbonylamino-,
(22) Mono- oder Diarylaminocarbonylamino-,
(23) Pyrrolidinylcarbonylamino-,
(24) Piperidinylcarbonylamino-,
(25) Aminocarbonyl-,
(26) Aminocarbonylamino-,
(27) C₁₋₆-Alkylaminocarbonyl-,
(28) C₁₋₆-Alkylaminocarbonylamino-,
(29) Di-C₁₋₆-alkylaminocarbonyl-,
(30) Di-C₁₋₆-alkylaminocarbonylamino-,
(31) Pyrrolidinylcarbonylamino-,
(32) Piperidinylcarbonylamino-,
(33) Aminosulfonyl-,
(34) C₁₋₆-Alkylaminosulfonyl-,
(35) C₁₋₆-Alkylsulfonylamino-,
(36) C₁₋₆-Alkylsulfonylaminomethyl-,
(37) Arylsulfonylamino-,
(38) Di-C₁₋₆-alkylaminosulfonyl-,
(39) Aminosulfonylmethyl-,
(40) C₁₋₆-Alkylaminosulfonylmethyl-,
(41) Di-C₁₋₆-alkylaminosulfonylmethyl-,
(42) -(CH₂)ₘOR¹⁵,
(43) -(CH₂)ₘSR¹⁵,
(44) -(CH₂)ₘSOR¹⁵,
(45) -(CH₂)ₘSO₂R¹⁵,
(46) -(CH₂)ₘNR¹⁵R¹⁶,
(47) =O und
(48) 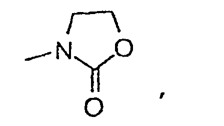
X¹ und X² jeweils unabhängig ausgewählt sind aus Ring-Stickstoff- oder Ring-Kohlenstoffatomen,
X³ ausgewählt ist aus der Gruppe, bestehend aus Sauerstoff, Schwefel, -NHoder Methylen,
Y¹ Sauerstoff oder Schwefel ist,
n eine ganze Zahl ist, die,bei jedem Vorkommen unabhängig ausgewählt ist aus 0 und 1, und
m eine ganze Zahl ist, die bei jedem Vorkommen unabhängig ausgewählt ist aus 0 bis 4.

2. Das Verfahren gemäß Anspruch 1, bei dem R¹, R³ und R⁴ jeweils Wasserstoff sind.

3. Das Verfahren gemäß Anspruch 1, bei dem der Palladiumkatalysator ausgewählt ist aus: einem Palladiumalkanoat, einem Palladiumacetonat, einem Palladiumhalogenid, einem Palladiumhalogenidkomplex, einem Palladiumbenzylidenacetonkomplex und einem Triarylphosphinpalladiumkomplex.

4. Das Verfahren gemäß Anspruch 3, bei dem der Palladiumkatalysator ausgewählt ist aus:
Pd(II)acetat, Pd(II)acetylacetonat, Pd(O)bisdibenzylidenaceton ("dba"), Pd(II)bromid, Pd(II)chlorid, Pd(II)iodid, Pd(II)sulfat, Pd(II)trifluoracetat, Pd(II)Cl₂(CH₃CN)₂, Pd₂(dba)₃ und Pd(II)Cl₂(PhCN)₂.

5. Das Verfahren gemäß Anspruch 4, bei dem der Palladiumkatalysator Pd(II)acetat ist.

6. Das Verfahren gemäß Anspruch 1, bei dem der Protonenakzeptor nicht mit dem Palladiumkatalysator in Wechselwirkung tritt, um dessen katalytische Wirkung zu inhibieren.

7. Der Verfahren gemäß Anspruch 6, bei dem der Protonenakzeptor ausgewählt ist aus:
(a) einem Alkylamin,
(b) einem aromatischen Amin,
(c) einem heterocyclischen Amin und
(d) einem Phosphat.

8. Das Verfahren gemäß Anspruch 7, bei dem der Protonenakzeptor ein Alkylamin ist.

9. Das Verfahren gemäß Anspruch 8, bei dem das Alkylamin ausgewählt ist aus:
(a) l,4-Diazobicyclo[2.2.2]octan,
(b) Chinuclidin,
(c) t-Butylamin,
(d) 2,2,6,6-Tetramethylpiperidin und
(e) Di-t-butylamin.

10. Das Verfahren gemäß Anspruch 9, bei dem das Alkylamin 1,4-Diazobicyclo[2.2.2]octan ist.

11. Das Verfahren gemäß Anspruch 1, bei dem die Reaktion in einem trockenen organischen Lösungsmittel durchgeführt wird, das gegenüber den Ausgangsmaterialien inert ist.

12. Das Verfahren gemäß Anspruch 11, bei dem das Lösungsmittel ausgewählt ist aus:
(a) DMSO,
(b) DMF,
(c) DMAC und
(d) NMP.

13. Das Verfahren gemäß Anspruch 12, bei dem das Lösungsmittel DMF ist.

14. Das Verfahren gemäß Anspruch 1, bei dem die Reaktion bei einer Temperatur von 90°C bis 120°C durchgeführt wird.

15. Das Verfahren gemäß Anspruch 1, das zusätzlich den Schritt der Entfernung der Schutzgruppe von der Verbindung der Strukturformel (III) umfaßt, um die Verbindung der Strukturformel (IV) zu erhalten:

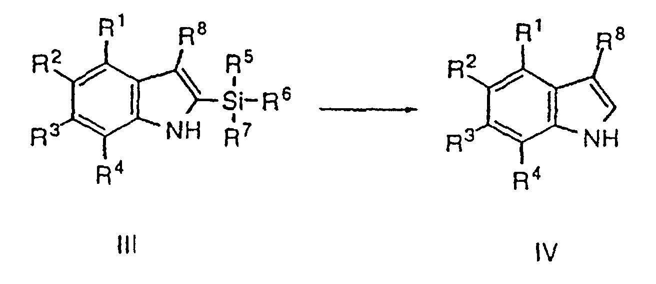

16. Das Verfahren gemäß Anspruch 15, bei dem die Entfernung der Schutzgruppe eine lewissäurekatalysierte Entfernung der Schutzgruppe ist.

17. Das Verfahren gemäß Anspruch 15, bei dem:
R¹, R², R³ und R⁴ jeweils unabhängig ausgewählt sind aus:
(1) Wasserstoff,
(2) 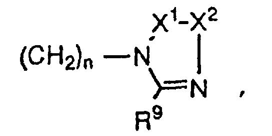
(3) C₁₋₆-Alkyl,
(4) -(CH₂)ₙ-Z,
wobei Z bedeutet:
(a) Wasserstoff,
(b) Halogen,
(c) Cyano,
(d) Nitro,
(e) Trifluormethyl,
(f) OR¹⁰,
(g) OCOR¹⁰,
(h) OCONR¹⁰R¹¹,
(i) OCH₂CN,
(j) OCH₂CONR¹⁰R¹¹,
(k) SR¹⁰, mit der Maßgabe, daß R¹⁰ nicht Wasserstoff ist,
(1) SOR¹⁰,
(m) SO₂R¹⁰,
(n) SO₂NR¹⁰R¹¹,
(o) NR¹⁰R¹¹,
(p) NR¹⁰COR¹¹,
(q) NR¹⁰CO₂R¹¹,
(r) NR¹⁰SO₂R¹¹,
(s) COR¹⁰,
(t) CO₂R¹⁰,
(u) CONR¹⁰R¹¹,
oder Z eine Gruppe der Formel (Za), (Zb), (Zc) oder (Zd) ist: 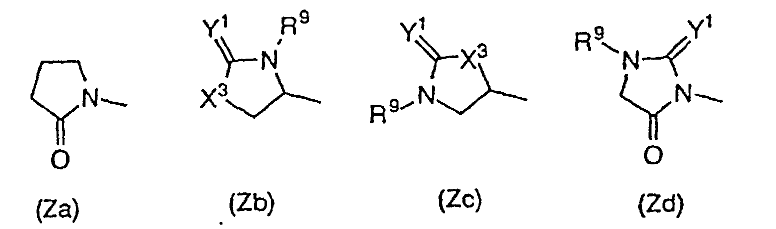
oder Z einen gegebenenfalls substituierten fünfgliedrigen heteroaromatischen Ring bedeutet, ausgewählt aus Furan, Thiophen, Pyrrol, Oxazol, Thiazol, Isoxazol, Isothiazol, Imidazol, Pyrazol, Oxadiazol, Thiadiazol, Triazol und Tetrazol,
R⁵, R⁶ und R⁷ jeweils unabhängig ausgewählt sind aus:
(1) C₁₋₆-Alkyl,
(2) -O-C₁₋₆-Alkyl und
(3) Phenyl,
R⁸ ausgewählt ist aus:
(1) Wasserstoff,
(2) -R¹⁹-OH,
(3) -R¹⁹-O-R¹⁷ und
(4) -R¹⁹NR¹²R¹³ und
(5) -R¹⁹-Z¹,
wobei: Z¹ ein 3- bis 7gliedriger heterocyclischer Ring ist, wobei die Ringelemente ausgewählt sind aus 1 bis 2 Stickstoffatomen und der heterocyclische Ring durch einen oder mehrere Reste R¹⁴ substituiert sein kann,
R⁹ ausgewählt ist aus:
(1) Wasserstoff und
(2) C₁₋₄-Alkyl,
R¹⁰ und R¹¹ jeweils unabhängig ausgewählt sind aus:
(1) Wasserstoff,
(2) C₁₋₆-Alkyl,
(3) Trifluormethyl,
(4) Phenyl, gegebenenfalls substituiert mit einem oder mehreren R²⁰-Substituenten,
(5) Methylphenyl, gegebenenfalls substituiert mit einem oder mehreren R²⁰-Substituenten, und
(6) einer Aryl-C₁₋₆-alkyl- oder Heteroaryl-C₁₋₆-alkylgruppe, gegebenenfalls substituiert mit einem oder mehreren R²⁰-Substituenten, oder
R¹⁰ und R¹¹, wenn sie durch ein Stickstoffatom verbunden sind, zusammen den Rest eines gegebenenfalls substituierten Azetidin-, Pyrrolidin-, Piperidin-, Morpholin- oder Piperazinrings bedeuten, der gegebenenfalls mit einem oder mehreren R¹⁸-Substituenten substituiert ist,
R¹² und R¹³ jeweils unabhängig ausgewählt sind aus:
(1) C₁₋₄-Alkyl,
(2) C₁₋₄-Alkyl-C₆-aryl, wobei das Aryl unsubstituiert oder mit einem bis drei Substituenten, ausgewählt aus Methyl, Halogen und Halogenmethyl, substituiert sein kann,
R¹⁴ ausgewählt ist aus:
(1) Aryl-C₁₋₆-alkyl, unsubstituiert oder substituiert mit einem bis drei R²⁰-Substituenten, und
(2) Heteroaryl-C₁₋₆-alkyl, unsubstituiert oder substituiert mit einem bis drei R²⁰-Substituenten,
R¹⁵ und R¹⁶ jeweils unabhängig ausgewählt sind aus
(1) Wasserstoff,
(2) C₁₋₆-Alkyl,
(3) C₃₋₇-Cycloalkyl,
(4) C₃₋₇-Cycloalkyl-C₁₋₆-alkyl,
(5) Indanyl,
(6) Aryl,
(7) Aryl-C₁₋₆-alkyl,
(8) C₃₋₇-Heterocycloalkyl,
(9) C₃₋₇-Heterocycloalkyl-C₁₋₆-alkyl,
(10) Heteroaryl und
(11) Heteroaryl-C₁₋₆-alkyl,
R¹⁷ ausgewählt ist aus einer Hydroxyschutzgruppe, die unter milder Säurehydrolyse entfernt werden kann,
R¹⁸ ausgewählt ist aus:
(1) C₁₋₆-Alkyl,
(2) Aryl-C₁₋₆-alkyl,
(3) C₁₋₆-Alkoxy,
(4) C₂₋₆-Alkyloxycarbonyl und
(5) C₁₋₆-Alkylaminocarbonyl,
R¹⁹ eine gerade oder verzweigte C₁₋₆-Alkylkette ist,
R²⁰ ausgewählt ist aus:
(1) Fluor,
(2) Cyano,
(3) Trifluormethyl,
(4) C₁₋₆-Alkyl,
(5) Halogen-C₁₋₆-alkyl,
(6) Aryl,
(7) Triazolyl,
(8) Tetrazolyl,
(9) C₁₋₆-Alkyltetrazolyl,
(10) Hydroxy,
(11) C₁₋₆-Alkoxy,
(12) C₁₋₆-Alkylthio,
(13) C₂₋₆-Alkoxycarbonyl,
(14) C₂₋₆-Alkylcarbonyl,
(15) C₁₋₆-Alkylsulfonyl,
(16) Arylsulfonyl,
(17) C₂₋₆-Alkylcarbonylamino,
(18) Arylcarbonylamino,
(19) C₂₋₆-Alkoxycarbonylamino,
(20) N-C₁₋₆-Alkyl-N-C₂₋₆-alkoxyamino,
(21) Carbonylamino,
(22) Mono- oder Diarylaminocarbonylamino,
(23) Pyrrolidinylcarbonylamino,
(24) Piperidinylcarbonylamino,
(25) Aminocarbonyl,
(26) Aminocarbonylamino,
(27) C₁₋₆-Alkylaminocarbonyl,
(28) C₁₋₆-Alkylaminocarbonylamino,
(29) Di-C₁₋₆-alkylaminocarbonyl,
(30) Di-C₁₋₆-alkylaminocarbonylamino,
(31) Pyrrolidinylcarbonylamino,
(32) Piperidinylcarbonylamino,
(33) Aminosulfonyl,
(34) C₁₋₆-Alkylaminosulfonyl,
(35) C₁₋₆-Alkylsulfonylamino,
(36) C₁₋₆-Alkylsulfonylaminomethyl,
(37) Arylsulfonylamino,
(38) Di-C₁₋₆-alkylaminosulfonyl,
(39) Aminosulfonylmethyl,
(40) C₁₋₆-Alkylaminosulfonylmethyl, und
(41) Di-C₁₋₆-alkylaminosulfonylmethyl,
(42) (CH₂)ₘOR¹⁵,
(43) (CH₂)ₘSR¹⁵, mit der Maßgabe, daß R¹⁵ nicht Wasserstoff ist,
(44) (CH₂)ₘSOR¹⁵,
(45) (CH₂)ₘSO₂R¹⁵,
(46) (CH₂)ₘNR¹⁵R¹⁶,
(47) =O und
(48) 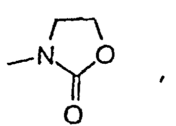
X¹ und X² jeweils unabhängig ausgewählt sind aus Ring-Stickstoff- oder Ring-Kohlenstoffatomen,
X³ ausgewählt ist aus der Gruppe, bestehend aus Sauerstoff, Schwefel, -NHoder Methylen,
Y¹ Sauerstoff oder Schwefel ist,
n eine ganze Zahl ist, die bei jedem Vorkommen unabhängig ausgewählt ist aus 0 und 1, und
m eine ganze Zahl ist, die bei jedem Vorkommen unabhängig ausgewählt ist aus 0 bis 4.

18. Das Verfahren gemäß Anspruch 17, bei dem R¹, R³ und R⁴ jeweils Wasserstoff sind.

19. Das Verfahren gemäß Anspruch 18, bei dem die Verbindung gemäß der Strukturformel IV ausgewählt ist aus:
(1) 1-Benzyl-4-[3-(5-(1,2,4-triazol-4-yl)-1H-indol-3-yl)propyl]-piperazin-2-on,
(2) 1-(2-Phenylethyl)-4-[3-(5-(1,2,4-triazol-4-yl)-1H-indol-3-yl)propyl]piperazin-2-on,
(3) 1-[2-(3-Fluorphenyl)ethyl]-4-[3-(5-(1,2,4-triazol-4-yl)-1Hindol-3-yl)propyl]piperazin-2-on,
(4) (3S)-3-(N-Benzyl)aminomethyl-1-[2-(5-(N-methyl)aminosulfonylmethyl)-1H-indol-3-yl)ethyl]pyrrolidin,
(5) (3S)-3-(N-Benzyl)aminomethyl-1-[2-(5-(aminosulfonylmethyl)-1Hindol-3-yl)ethyl]pyrrolidin,
(6) (3S)-3-(N-Benzyl)aminomethyl-(S)-1-[2-(5-(2-oxo-1,3-oxazolidin-4-ylmethyl)-1H-indol-3-yl)ethyl)pyrrolidin,
(7) (3S)-3-[N-(R)-α-(Hydroxymethyl)benzyl]aminomethyl-(S)-1-[2-(5-(2-oxo-1,3-oxazolidin-4-ylmethyl)-1H-indol-3-yl)ethyl]-pyrrolidin,
(8) (3S)-3-[N-(S)-α-Methylbenzyl]aminomethyl-(S)-1-[2-(5-(2-oxo-1,3-oxazolidin-4-ylmethyl)-1H-indol-3-yl)ethyl]pyrrolidin,
(9) 4-[N-(R)-α-(Hydroxymethyl)benzyl]amino-(S)-1-[3-(5-(2-oxo-1,3-oxazolidin-4-ylmethyl)-1H-indol-3-yl)propyl]piperidin,
(10) (3S)-3-(N-Benzyl-N-methyl)aminomethyl-(S)-1-[2-(5-(3-methyl-2-oxo-1,3-oxazolidin-4-ylmethyl)-1H-indol-3-yl)ethyl]pyrrolidin,
(11) (3R)-3-[N-(S)-α-Methylbenzyl-N-methyl]aminomethyl-(S)-1-[2-(5-(2-oxo-1,3-oxazolidin-4-ylmethyl)-1H-indol-3-yl)ethyl]-pyrrolidin,
(12) (3R)-3-[N-(S)-α-Methylbenzyl-N-methyl]aminomethyl-(S)-1-[2-(5-(3-methyl-2-oxo-1,3-oxazolidin-4-ylmethyl)-1H-indol-3-yl)-ethyl]pyrrolidin,
(13) (3S)-3-[N-(4-Fluorbenzyl)-N-methyl]aminomethyl-(S)-1-[2-(5-(3-methyl-2-oxo-1,3-oxazolidin-4-ylmethyl)-1H-indol-3-yl)ethyl]-pyrrolidin,
(14) 4-Benzyl-12-(2-fluormethyl-3-(5-(1,2,4-triazol-4-yl)-1H-indol-3-yl)propyl]piperazin,
(15) 4-[2-(3-Fluorphenyl)ethyl]-1-[2-fluor-3-(5-(1,2,4-triazol-4-yl)-1H-indol-3-yl)propyl]piperazin,
(16) 4-Benzyl-1-[2-fluor-3-(5-(1,2,4-triazol-4-yl)-1H-indol-3-yl)propyl]piperidin,
(17) 4-(N-Benzyl-N-methylamino)-1-[2-fluor-3-(5-(1,2,4-triazol-4-yl)-1H-indol-3-yl)propyl]piperidin,
(18) 4-[(R)-2-Hydroxy-1-(4-fluorphenyl)ethylamino]-1-[2-fluor-3-(5-(1,2,4-triazol-4-yl)-1H-indol-3-yl)propyl]piperidin,
(19) 7-Benzyl-2-[2-(5-(1,2,4-triazol-4-yl)-1H-indol-3-yl)ethyl]-cis-2,7-diazabicyclo[3.3.0]octan,
(20) 7-(3-Furylmethyl-2-[2-(5-(1,2,4-triazol-4-yl)-1H-indol-3-yl)ethyl]-cis-2,7-diazabicyclo[3.3.0]octan,
(21) 7-(2-Phenylethyl-2-[2-(5-(1,2,4-triazol-4-yl)-1H-indol-3-yl)ethyl]-cis-2,7-diazabicyclo[3.3.0]octan,
(22) 7-(4-Fluorbenzyl-2-[2-(5-(1,2,4-triazol-4-yl)-1H-indol-3-yl)ethyl]-cis-2,7-diazabicyclo[3.3.0]octan,
(23) 7-(2,4-Difluorbenzyl-2-[2-(5-(1,2,4-triazol-4-yl)-1H-indol-3-yl)ethyl]-cis-2,7-diazabicyclo[3.3.0]octan,
(24) 4-(2,2-Difluor-1-oxo-2-phenylethyl)-1-[3-(5-(1,2,4-triazol-4-yl)-1H-indol-3-yl)propyl]piperazin,
(25) 4-Benzyl-3-methyl-1-[3-(5-(1,2,4-triazol-4-yl)-1H-indol-3-yl)propyl]-1,2,5,6-tetrahydropyridin,
(26) 4-Benzyl-3-methoxymethyl-1-[3-(5-(1,2,4-triazol-4-yl)-1Hindol-3-yl)propyl]-1,2,5,6-tetrahydropyridin,
(27) 1-(2-Hydroxy-1-phenylethyl)-4-[3-(5-(1,2,4-triazol-4-yl)-1Hindol-3-yl)propyl]piperidin,
(28) 1-[2-(2-Fluorphenyl)ethyl]-4-[3-(5-(1,2,4-triazol-4-yl)-1Hindol-3-yl)propyl]piperidin,
(29) 1-Benzyl-4-[3-(5-(1,2,4-triazol-4-yl)-1H-indol-3-yl)propyl]-piperidin,
(30) 1-(3,3-Dimethylbutyl)-4-[3-(5-(1,2,4-triazol-4-yl)-1H-indol-3-yl) propyl]piperidin,
(31) 1-(2-Phenylethyl)-4-[3-(5-(1,2,4-triazol-4-yl)-1H-indol-3-yl)propyl]piperidin,
(32) 1-Cyclohexylmethyl-4-[3-(5-(1,2,4-triazol-4-yl)-1H-indol-3-yl)propyl]piperidin,
(33) 1-(3-Phenylpropyl)-4-[3-(5-(1,2,4-triazol-4-yl)-1H-indol-3-yl)propyl]piperidin,
(34) 1-[2-(3-Fluorphenyl)ethyl]-4-[3-(5-(1,2,4-triazol-4-yl)-1Hindol-3-yl)propyl]piperidin,
(35) 1-[2-(4-Trifluormethylphenyl)ethyl]-4-[3-(5-(1,2,4-triazol-4-yl)-1H-indol-3-yl)propyl]piperidin,
(36) 1-[2-(3,4-Difluorphenyl)ethyl]-4-[3-(5-(1,2,4-triazol-4-yl)-1H-indol-3-yl)propyl]piperidin,
(37) N-Methyl-2-phenyl-2-[4-(3-(5-(1,2,4-triazol-4-yl)-1H-indol-3-yl)propyl)piperidin-1-yl]acetamid,
(38) 1-(2-Oxo-2-phenylethyl)-4-[3-(5-(1,2,4-triazol-4-yl)-1H-indol-3-yl)propyl]piperidin,
(39) 1-(2-Phenylpropyl)-4-[3-(5-(1,2,4-triazol-4-yl)-1H-indol-3-yl)propyl]piperidin,
(40) 4-Benzyl-4-fluor-1-[3-(5-(1,2,4-triazol-4-yl)-1H-indol-3-yl)propyl]piperidin,
(41) 4-Fluor-4-[2-(3-fluorphenyl)ethyl]-1-[3-(5-(1,2,4-triazol-4-yl)-1H-indol-3-yl)propyl]piperidin,
(42) 4-Fluor-4-(3-fluorbenzyl)-1-[3-(5-(1,2,4-triazol-4-yl)-1Hindol-3-yl)propyl]piperidin,
(43) 4-Fluor-4-(2-fluorbenzyl)-1-[3-(5-(1,2,4-triazol-4-yl)-1Hindol-3-yl)propyl]piperidin,
(44) 4-Benzyl-4-methoxy-1-[3-(5-(1,2,4-triazol-4-yl)-1H-indol-3-yl)propyl]piperidin,
(45) 4-Benzyl-4-methoxy-1-[3-(5-(1,2,4-triazol-1-ylmethyl)-1Hindol-3-yl)propyl]piperidin,
(46) 4-(2-Fluorbenzyl)-4-methoxy-1-[3-(5-(1,2,4-triazol-4-yl)-1Hindol-3-yl)propyl]piperidin,
(47) 4-(3-Fluorbenzyl)-4-methoxy-1-[3-(5-(1,2,4-triazol-4-yl)-1Hindol-3-yl)propyl]piperidin,
(48) 4-(4-Fluorbenzyl)-4-methoxy-1-[3-(5-(1,2,4-triazol-4-yl)-1Hindol-3-yl)propyl]piperidin,
(49) 1-[3-(5-(1,2,4-Triazol-4-yl)-1H-indol-3-yl)propyl]-4-[2-(oxazol-2-on-3-yl)-1-phenylethyl]piperazin,
(50) 1-[3-(5-(1,2,4-Triazol-4-yl)-1H-indol-3-yl)propyl]-4-[2-(oxazolidin-2-on-3-yl)-1-phenylethyl]piperazin,
(51) 1-[3-(5-(1,2,4-Triazol-4-yl)-1H-indol-3-yl)propyl]-4-[1-(4-fluorphenyl)-2-(oxazolidin-2-on-3-yl)ethyl]piperazin,
(52) 1-[3-(5-(1,2,4-Triazol-4-yl)-1H-indol-3-yl)propyl]-4-(3-hydroxy-1-phenylpropyl)piperazin,
(53) 1-[3-(5-(1,2,4-Triazol-4-yl)-1H-indol-3-yl)propyl]-4-[2-(imidazol-1-yl)-1-phenylethyl]piperazin,
(54) 1-[3-(5-(1,2,4-Triazol-4-yl)-1H-indol-3-yl)propyl]-4-[1-(4-fluorphenyl)-2-hydroxyethyl]piperazin,
(55) 1-[3-(5-(1,2,4-Triazol-4-yl)-1H-indol-3-yl)propyl]-4-[1-(4-fluorphenyl)-2-methoxyethyl]piperazin,
(56) 1-[3-(5-(1,2,4-Triazol-4-yl)-1H-indol-3-yl)propyl]-4-[3-(5-methyl-1,2,4-oxadiazol-3-yl)-1-phenylpropyl]piperazin,
(57) 1-[3-(5-(1,2,4-Triazol-4-yl)-1H-indol-3-yl)propyl]-4-[2-benzyloxy-1-(4-fluorphenyl)ethyl]piperazin,
(58) 1-[3-(5-(1,2,4-Triazol-4-yl)-1H-indol-3-yl)propyl]-4-[1-(4-fluorphenyl)-3-methoxypropyl]piperazin,
(59) 1-[3-(5-(1,2,4-triazol-4-yl)-1H-indol-3-yl)propyl]-4-[1-(4-fluorphenyl)-2-(imidazol-1-yl)ethyl]piperazin,
(60) 1-[3-(5-(1,2,4-triazol-4-yl)-1H-indol-3-yl)propyl]-4-[1-phenyl-2-(pyrrolidin-1-yl)ethyl]piperazin,
(61) 1-[3-(5-(1,2,4-Triazol-4-yl)-1H-indol-3-yl)propyl]-4-[1-(4-fluorphenyl)-3-hydroxypropyl]piperazin,
(62) 1-[3-(5-(1,2,4-Triazol-4-yl)-1H-indol-3-yl)propyl]-4-[3-(imidazol-1-yl)-1-phenylpropyl]piperazin,
(63) 1-[3-(5-(1,2,4-Triazol-4-yl)-1H-indol-3-yl)propyl]-4-(3-hydroxy-2-phenylpropyl)piperazin,
(64) 1-[3-(5-(1,2,4-Triazol-4-yl)-1H-indol-3-yl)propyl]-4-(3-methoxy-2-phenylpropyl)piperazin,
(65) 1-[3-(5-(1,2,4-Triazol-4-yl)-1H-indol-3-yl)propyl]-4-[2-(4-fluorphenyl)-3-hydroxypropyl]piperazin,
(66) 1-[3-(5-(1,2,4-Triazol-4-yl)-1H-indol-3-yl)propyl]-4-[1-(4-fluorphenyl)prop-2-yl]piperazin,
(67) 1-[3-(5-(1,2,4-Triazol-4-yl)-1H-indol-3-yl)propyl]-4-[2-(4-fluorphenyl)propyl]piperazin,
(68) 1-[3-(5-(1,2,4-Triazol-4-yl)-1H-indol-3-yl)propyl]-4-[1-(4-fluorphenyl)-3-hydroxyprop-2-yl]piperazin,
(69) 1-[3-(5-(1,2,4-Triazol-4-yl)-1H-indol-3-yl)propyl]-4-[1-(4-fluorphenyl)-3-methoxyprop-2-yl]piperazin,
(70) 1-[3-(5-(1,2,4-Triazol-4-yl)-1H-indol-3-yl)propyl]-4-[2-(3-fluorphenyl)ethyl]piperazin,
(71) 1-[3-(5-(1,2,4-Triazol-4-yl)-1H-indol-3-yl)propyl]-4-[2-(2-fluorphenyl)ethyl]piperazin,
(72) 1-[3-(5-(1,2,4-Triazol-4-yl)-1H-indol-3-yl)propyl]-4-[2-(4-fluorphenyl)ethyl]piperazin,
(73) 1-[3-(5-(1,2,4-Triazol-4-yl)-1H-indol-3-yl)propyl]-4-[2-(3-methoxyphenyl)ethyl]piperazin,
(74) 1-[3-(5-(1,2,4-Triazol-4-yl)-1H-indol-3-yl)propyl]-4-[2-(3-trifluormethylphenyl)ethyl]piperazin,
(75) 1-[3-(5-(1,2,4-Triazol-4-yl)-1H-indol-3-yl)propyl]-4-[2-(3,4-difluorphenyl)ethyl]piperazin,
(76) 1-[3-(5-(1,2,4-Triazol-4-yl)-1H-indol-3-yl)propyl]-4-[2-(2,4-difluorphenyl)ethyl]piperazin,
(77) 1-[3-(5-(1,2,4-Triazol-4-yl)-1H-indol-3-yl)propyl]-4-[2-(3,5-difluorphenyl)ethyl]piperazin,
(78) 1-[3-(5-(1,2,4-Triazol-4-yl)-1H-indol-3-yl)propyl]-4-[2-(3-(oxazolidin-2-on-3-yl)phenyl)ethyl]piperazin,
(79) N-Methyl-3-[5-(1,2,4-triazol-4-yl)-1H-indol-3-yl]pyrrolidin,
(80) N-Methyl-4-[5-(1,2,4-triazol-4-yl)-1H-indol-3-yl]piperidin,
(81) N,N-Dimethyl-2-[5-(1,2,4-triazol-4-yl)-1H-indol-3-yl]ethylamin,
(82) 4-(1-Phenylethyl)-1-[3-(5-(1,2,4-triazol-4-yl)-1H-indol-3-yl)propyl]-1,2,5,6-tetrahydropyridin,
(83) 4-(α-Isopropyloxy)phenylmethyl-1-[3-(5-(1,2,4-triazol-4-yl)-1H-indol-3-yl)propyl]-1,2,5,6-tetrahydropyridin,
(84) 4-(α-Methoxy)phenylmethyl-1-[3-(5-(1,2,4-triazol-4-yl)-1Hindol-3-yl)propyl]-1,2,5,6-tetrahydropyridin,
(85) 4-[α-(2-Methoxyethyl)oxy]phenylmethyl-1-[3-(5-(1,2,4-triazol-4-yl)-1H-indol-3-yl)propyl]-1,2,5,6-tetrahydropyridin,
(86) 4-Benzyl-1-[3-(2,3-dihydro-5-(1,2,4-triazol-4-yl)-1H-indol-3-yl)propyl]piperazin,
(87) 1-[3-(2,3-Dihydro-5-(1,2,4-triazol-4-yl)-1H-indol-3-yl)propyl]-4-(pyridin-3-ylmethyl)piperazin,
(88) 1-[2-(5-(1,2,4-Triazol-4-yl)-1H-indol-3-yl)propyl]-4-[2-(4-fluorphenyl)-4-methylpiperazin-1-yl]piperidin,
(89) 1-[2-(5-(1,2,4-Triazol-1-ylmethyl)-1H-indol-3-yl)ethyl]-3(R)-(3(R)-phenylmorpholin-4-ylmethyl)pyrrolidin,
(90) 1-[3-(5-(1,2,4-Triazol-4-yl)-1H-indol-3-yl)propyl]-4-(3-oxo-2-phenylpiperazin-1-yl)methylpiperidin,
(91) 1-[3-(5-(1,2,4-Triazol-4-yl)-1H-indol-3-yl)propyl]-4-(4-methyl-2-phenylpiperazin-1-yl)piperidin,
(92) 1-[3-(5-(1,2,4-Triazol-4-yl)-1H-indol-3-yl)propyl]-4-(benzimidazol-2-on-1-yl)piperidin,
(93) 1-[3-(5-(1,2,4-Triazol-4-yl)-1H-indol-3-yl)propyl]-4-[6-(4-fluorphenyl)-4-methyl-3-oxopiperazin-1-yl]piperidin,
(94) [3-(3-(4-(2-(3,4-Difluorphenyl)ethyl)piperazin-1-yl)propyl)-1H-indol-5-ylmethyl]oxazolidin-2-on,
(95) (S)-4-[3-(3-(4-(2-(3,4-Difluorphenyl)ethyl)piperazin-1-yl)-propyl)-1H-indol-5-ylmethyl]-3-methyloxazolidin-2-on,
(96) 1-[3-(5-(N-Methylaminosulfonylmethyl)-1H-indol-3-yl)propyl]-4-[2-(4-(acetylamino)phenyl)ethyl]piperazin,
(97) 3-Benzyl-7-[3-(5-(1,2,4-triazol-4-yl)-1H-indol-3-yl)propyl]-cis-3,7-diazabicyclo[3.3.0]octan,
(98) 3-(Pyridin-3-yl)methyl-7-[3-(5-(1,2,4-triazol-4-yl)-1H-indol-3-yl)propyl]-cis-3,7-diazabicyclo[3.3.0]octan,
(99) 3-[2-(4-(Acetylamino)phenyl)ethyl]-7-[3-(5-(1,2,4-triazol-4-yl)-1H-indol-3-yl)propyl]-cis-3,7-diazabicyclo[3.3.0]octan,
(100) 3-Benzoyl-7-[3-(5-(1,2,4-Triazol-4-yl)-1H-indol-3-yl)propyl]-cis-3,7-diazabicyclo[3.3.0]octan,
(101) (1RS,3RS,5RS)-7-Benzyl-3-[5-(imidazol-1-yl)-1H-indol-3-ylmethyl]-2-methyl-2,7-diazabicyclo[3.3.0]octan,
(102) (1RS,3RS,5RS)-7-[2-(3-Fluorphenyl)ethyl]-2-methyl-3-[5-(1,2,4-triazol-4-yl)-1H-indol-3-ylmethyl]-2,7-diazabicyclo[3.3.0]-octan,
(103) (1RS,3RS,5RS)-7-(4-Fluorbenzyl)-2-methyl-3-[5-(1,2,4-triazol-4-yl)-1H-indol-3-ylmethyl]-2,7-diazabicyclo[3.3.0]octan,
(104) 4-[1-(Phenyl)-N,N-dimethylcarboxamidomethyl]-1-[3-(5-(1,2,4-triazol-4-yl)-1H-indol-3-yl)propyl]piperazin,
(105) 4-(2-Methoxycarbonylamino-1-phenylethyl)-1-[3-(5-(1,2,4-triazol-4-yl)-1H-indol-3-yl)propyl]piperazin,
(106) 4-(2-Dimethylamino-1-phenylethyl)-1-[3-(5-(1,2,4-triazol-4-yl)-1H-indol-3-yl)propyl]piperazin,
(107) 1-[3-(5-(1,2,4-Triazol-4-yl)-1H-indol-3-yl)propyl]-4-(4-fluorphenyl)methylpiperazin,
(108) 4-[2-(N-Methyl-N-methoxycarbonyl)amino-1-phenylethyl]-1-[3-(5-(1,2,4-triazol-4-yl)-1H-indol-3-yl)propyl]piperazin,
(109) 1-Benzyl-4-[(R,S)-2-hydroxymethyl-3-(5-(1,2,4-triazol-4-yl)-1H-indol-3-yl)propyl]piperazin,
(110) 1-[3-(5-(1,2,4-Triazol-4-yl)-1H-indol-3-yl)propyl]-4-[2-(1Htetrazol-5-yl)phenyl] methylpiperazin,
(111) 1-[3-(5-(1,2,4-Triazol-4-yl)-1H-indol-3-yl)propyl]-4-(2-phenylethyl)piperazin,
(112) 4-Benzyl-1-[3-(5-(1,2,4-triazol-4-yl)-1H-indol-3-yl)propyl]-piperazin,
(113) 4-[2-(2-Methyltetrazol-5-yl)phenyl]methyl-1-[3-(5-(1,2,4-triazol-4-yl)-1H-indol-3-yl)propyl]piperazin,
(114) 4-[2-(1-Methyltetrazol-5-yl)phenyl]methyl-1-[3-(5-(1,2,4-triazol-4-yl)-1H-indol-3-yl)propyl]piperazin,
(115) 4-[2-(N-Methylcarboxamido)phenyl]methyl-1-[3-(5-(1,2,4-triazol-4-yl)-1H-indol-3-yl)propyl]piperazin,
(116) 4-[2-(N,N-Dimethylaminomethyl)phenyl]methyl-1-[3-(5-(1,2,4-triazol-4-yl)-1H-indol-3-yl)propyl]piperazin,
(117) 4-(But-3-enyl)-1-[3-(5-(1,2,4-triazol-4-yl)-1H-indol-3-yl)-propyl]piperazin,
(118) 4-(3-Methylbut-2-enyl)-1-[3-(5-(1,2,4-triazol-4-yl)-1H-indol-3-yl)propyl]piperazin,
(119) 4-(Prop-2-enyl)-1-[3-(5-(1,2,4-triazol-4-yl)-1H-indol-3-yl)-propyl]piperazin,
(120) 4-(Prop-2-inyl)-1-[3-(5-(1,2,4-triazol-4-yl)-1H-indol-3-yl)propyl]piperazin,
(121) 4-[(R,S)-1-(Phenyl)carboxamidomethyl]-1-[3-(5-(1,2,4-triazol-4-yl)-1H-indol-3-yl)propyl]piperazin,
(122) 4-[1-(Phenyl)-N-methylcarboxamidomethyl]-1-[3-(5-(1,2,4-triazol-4-yl)-1H-indol-3-yl)propyl]piperazin,
(123) 1-[3-(5-(1,2,4-Triazol-1-ylmethyl)-1H-indol-3-yl)propyl]-4-[2-(4-(acetylamino)phenyl)ethyl]piperazin,
(124) 1-[3-(5-(1,2,4-Triazol-4-yl)-1H-indol-3-yl)propyl]-4-[2-(3-(acetylamino)phenyl)ethyl]piperazin,
(125) 1-[3-(5-(1,2,4-Triazol-4-yl)-1H-indol-3-yl)propyl]-4-[4-(aminosulfonyl)phenyl]methylpiperazin,
(126) 1-[3-(5-1,2,4-Triazol-4-yl)-1H-indol-3-yl)propyl]-4-(furan-3-yl)methylpiperazin,
(127) 1-[3-(5-(1,2,4-Triazol-4-yl)-1H-indol-3-yl)propyl]-4-(furan-2-yl)methylpiperazin,
(128) 1-[3-(5-(1,2,4-Triazol-4-yl)-1H-indol-3-yl)propyl]-4-(thien-2-yl)methylpiperazin,
(129) 1-Benzyl-4-[(R,S)-2-hydroxy-3-(5-(1,2,4-triazol-4-yl)-1Hindol-3-yl)propyl]piperazin,
(130) 1-[2-(4-(Acetylamino)phenyl)ethyl]-4-[(R,S)-2-hydroxy-3-(5-(1,2,4-triazol-4-yl)-1H-indol-3-yl)propyl]piperazin,
(131) 1-[3-(5-(1,2,4-Triazol-4-yl)-1H-indol-3-yl)propyl]-4-[2-(4-(aminocarbonylamino)phenyl)ethyl]piperazin,
(132) 1-[3-(5-(1,2,4-Triazol-4-yl)-1H-indol-3-yl)propyl]-4-(4-cyanophenyl)methylpiperazin,
(133) 1-[3-(5-(1,2,4-Triazol-4-yl)-1H-indol-3-yl)propyl]-4-[2-(4-cyanophenyl)ethyl]piperazin,
(134) 1-[3-(5-(1,2,4-Triazol-4-yl)-1H-indol-3-yl)propyl]-4-[2-(4-(1,2,4-triazol-4-yl)phenyl)ethyl]piperazin,
(135) 1-[3-(5-(1,2,4-Triazol-1-yl)-1H-indol-3-yl)propyl]-4-[2-(4-(acetylamino)phenyl)ethyl]piperazin,
(136) 1-[3-(5-(1,2,4-Triazol-1-yl)-1H-indol-3-yl)propyl]-4-benzylpiperazin,
(137) 1-[3-(5-(1,2,4-Triazol-1-ylmethyl)-1H-indol-3-yl)propyl]-4-benzylpiperazin,
(138) 4-(4-Acetylaminophenyl)methyl-1-[3-(5-(1,2,4-triazol-4-yl)-1Hindol-3-yl)propyl]piperidin,
(139) 4-Benzyl-1-[3-(5-(1,2,4-triazol-4-yl)-1H-indol-3-yl)propyl]-1,2,5,6-tetrahydropyridin,
(140) 1-[3-(5-(1,2,4-Triazol-4-yl)-1H-indol-3-yl)propyl]-4-(2-aminopyridin-5-yl)methylpiperazin,
(141) 1-[3-(5-(1,2,4-Triazol-4-yl)-1H-indol-3-yl)propyl]-4-(4-aminophenyl)methylpiperazin,
(142) 1-[4-(5-(1,2,4-Triazol-4-yl)-1H-indol-3-yl)butyl]-4-benzylpiperazin,
(143) 1-[4-(5-(1,2,4-Triazol-4-yl)-1H-indol-3-yl)butyl]-4-(pyridin-2-yl)methylpiperazin,
(144) 1-[4-(5-(1,2,4-Triazol-4-yl)-1H-indol-3-yl)butyl]-4-(pyridin-3-yl)methylpiperazin,
(145) 1-[3-(5-(1,2,4-Triazol-4-yl)-1H-indol-3-yl)propyl]-4-[2-(4-aminophenyl)ethyl]piperazin,
(146) 1-[3-(5-(1,2,4-Triazol-4-yl)-1H-indol-3-yl)propyl]-4-[2-(4-(acetylamino)phenyl)ethyl]piperazin,
(147) 1-[3-(5-(1,2,4-Triazol-4-yl)-1H-indol-3-yl)propyl]-4-(imidazol-2-yl)methylpiperazin,
(148) 1-[3-(5-(1,2,4-Triazol-4-yl)-1H-indol-3-yl)propyl]-4-[3-(acetylamino)phenyl]methylpiperazin,
(149) 1-[3-(5-(1,2,4-Triazol-4-yl)-1H-indol-3-yl)propyl]-4-[4-(acetylamino)phenyl]methylpiperazin,
(150) 1-[4-(5-(1,2,4-Triazol-4-yl)-1H-indol-3-yl)butyl]-4-[4-(acetylamino)phenyl]methylpiperazin,
(151) 1-[3-(5-(1,2,4-Triazol-4-yl)-1H-indol-3-yl)propyl]-4-(2-methoxyphenyl)methylpiperazin,
(152) 1-[3-(5-(1,2,4-Triazol-4-yl)-1H-indol-3-yl)propyl]-4-benzylpiperazin,
(153) 1-[3-(5-(1,2,4-Triazol-4-yl)-1H-indol-3-yl)propyl]-4-(pyridin-3-yl)methylpiperazin,
(154) 1-[3-(5-(1,2,4-Triazol-4-yl)-1H-indol-3-yl)propyl]-4-(pyridin-2-yl)methylpiperazin,
(155) 1-[3-(5-(1,2,4-Triazol-4-yl)-1H-indol-3-yl)propyl]-4-(pyridin-4-yl)methylpiperazin,
(156) (3R)-3-Benzyloxymethyl-1-[2-(5-(1,2,4-triazol-4-yl)-1H-indol-3-yl)ethyl]pyrrolidin,
(157) (3S)-3-(N-Benzyl-N-methyl)aminomethyl-1-[2-(5-(1,2,4-triazol-4-yl)-1H-indol-3-yl)ethyl]pyrrolidin,
(158) (2S)-2-(N-Benzyl-N-methyl)aminomethyl-1-[2-(5-(1,2,4-triazol-4-yl)-1H-indol-3-yl)ethyl]pyrrolidin,
(159) (3S)-3-(N-Benzyl)aminomethyl-1-[2-(5-(1,2,4-triazol-4-yl)-1Hindol-3-yl)ethyl]pyrrolidin,
(160) 4-(4-Acetylaminophenyl)methylamino-1-[3-(5-(1,2,4-triazol-4-yl)-1H-indol-3-yl)propyl]piperidin,
(161) 1-[3-(5-(Imidazol-1-yl)-1H-indol-3-yl)propyl]-4-[(R)-α-(methoxymethyl)benzylamino]piperidin,
(162) 1-[3-(5-(1,2,4-Triazol-1-ylmethyl)-1H-indol-3-yl)propyl]-4-[(R)-1-(4-fluorphenyl)-2-methoxyethylamino]piperidin,
(163) 1-[3-(5-(1,2,4-Triazol-1-ylmethyl)-1H-indol-3-yl)propyl]-4-[N-(4-fluorbenzyl)-N-methylamino]piperidin,
(164) 1-[3-(5-(1,2,4-Triazol-4-yl)-1H-indol-3-yl)propyl]-4-(2-phenylpiperidin-1-yl)piperidin,
(165) 1-[3-(5-(1,2,4-Triazol-4-yl)-1H-indol-3-yl)propyl]-4-[(R)-1-(4-fluorphenyl)-2-methoxyethylamino]piperidin,
(166) 1-[3-(5-(1,2,4-Triazol-4-yl)-1H-indol-3-yl)propyl]-4-(indan-1-ylaminomethyl)piperidin,
(167) 1-[3-(5-(1,2,4-Triazol-4-yl)-1H-indol-3-yl)propyl]-4-[N-(R)-α-(hydroxymethyl)benzyl-N-methylaminomethyl]piperidin,
(168) (3R)-3-(Benzylthio)methyl-1-[2-(5-(1,2,4-triazol-4-yl)-1Hindol-3-yl)ethyl]pyrrolidin,
(169) (±)-1-[3-(5-(1,2,4-Triazol-4-yl)-1H-indol-3-yl)propyl]-4-(1-benzylamino-2-hydroxyethyl)piperidin,
(170) 1-[3-(5-(1,2,4-Triazol-1-yl)-1H-indol-3-yl)propyl]-4-[(R)-α-(hydroxymethyl)benzylamino]piperidin,
(171) 1-[3-(5-(Imidazol-1-yl)-1H-indol-3-yl)propyl]-4-[(R)-α-(methyl)benzylamino]piperidin,
(172) 1-[3-(5-(Imidazol-1-yl)-1H-indol-3-yl)propyl]-4-[(R)-α-(hydroxymethyl)benzylamino]piperidin,
(173) 1-[3-(5- (1,2,4-Triazol-1-ylmethyl)-1H-indol-3-yl)propyl]-4-[(R)-α-(hydroxymethyl)benzylamino]piperidin,
(174) 1-[3-(5-(1,2,4-Triazol-4-yl)-1H-indol-3-yl)propyl]-4-([R)-α-(methoxymethyl)benzylamino]piperidin,
(175) 1-[3-(5-(1,2,4-Triazol-4-yl)-1H-indol-3-yl)propyl]-4-[N-(R)-α-(methoxymethyl)benzyl-N-methylamino]piperidin,
(176) (3R)-3-Benzyloxy-1-[2-(5-(1,2,4-triazol-4-yl)-1H-indol-3-yl)ethyl]pyrrolidin,
(177) (3R)-3-(4-Methoxyphenyl)methoxy-1-[2-(5-(1,2,4-triazol-4-yl)-1H-indol-3-yl)ethyl]pyrrolidin,
(178) (3R)-3-(Pyridin-3-yl)methoxy-1-[2-(5-(1,2,4-triazol-4-yl)-1Hindol-3-yl)ethyl]pyrrolidin,
(179) (3R)-3-Benzyloxymethyl-1-[2-(5-(1,2,4-triazol-4-yl)-1H-indol-3-yl)ethyl]pyrrolidin,
(180) (3S)-3-(N-Benzyl-N-methyl)aminomethyl-1-[2-(5-(1,2,4-triazol-4-yl)-1H-indol-3-yl)ethyl]pyrrolidin,
(181) (2S)-2-(N-Benzyl-N-methyl)aminomethyl-1-[2-(5-(1,2,4-triazol-4-yl)-1H-indol-3-yl)ethyl]pyrrolidin,
(182) (3S)-3-(N-Benzyl)aminomethyl-1-[2-(5-(1,2,4-triazol-4-yl)-1Hindol-3-yl)ethyl]pyrrolidin,
(183) 4-(4-Acetylaminophenyl)methylamino-1-[3-(5-(1,2,4-triazol-4-yl)-1H-indol-3-yl)propyl]piperidin,
(184) 4-Benzylamino-1-[3-(5-(1,2,4-triazol-4-yl)-1H-indol-3-yl)-propyl]piperidin,
(185) 4-(N-Benzyl-N-methyl)amino-1-[3-(5-(1,2,4-triazol-4-yl)-1Hindol-3-yl)propyl]piperidin,
(186) 4-(N-Benzyl-N-methyl)aminomethyl-1-[3-(5-(1,2,4-triazol-4-yl)-1H-indol-3-yl)propyl]piperidin,
(187) 1-[3-(5-(1,2,4-Triazol-4-yl)-1H-indol-3-yl)propyl]-4-[α-(methyl)benzylamino]piperidin,
(188) 1-[3-(5-(1,2,4-Triazol-4-yl)-1H-indol-3-yl)propyl]-4-[α-(hydroxymethyl)benzylamino]piperidin,
(189) 1-[3-(5-(1,2,4-Triazol-4-yl)-1H-indol-3-yl)propyl]-4-[(1-hydroxymethyl-2-phenyl)ethylamino]piperidin,
(190) 4-(N-Benzyl)aminomethyl-1-[3-(5-(1,2,4-triazol-4-yl)-1H-indol-3-yl)propyl]piperidin,
(191) 1-[3-(5-(1,2,4-Triazol-4-yl)-1H-indol-3-yl)propyl]-4-[(2-hydroxy-1-methyl-2-phenyl)ethylamino]piperidin,
(192) 1-[3-(5-(1,2,4-Triazol-4-yl)-1H-indol-3-yl)propyl]-4-[2-(4-acetylaminophenyl)ethylamino]piperidin,
(193) 1-[3-(5-(1,2,4-Triazol-4-yl)-1H-indol-3-yl)propyl]-4-[α-(methyl)benzylamino]methylpiperidin,
(194) 1-[3-(5-(1,2,4-Triazol-4-yl)-1H-indol-3-yl)propyl]-4-[1-(4-acetylaminophenyl)ethylamino]methylpiperidin,
(195) 1-[3-(5-(1,2,4-Triazol-4-yl)-1H-indol-3-yl)propyl]-4-[N-[α-(hydroxymethyl)benzyl]-N-methylamino]piperidin,
(196) 1-[3-(5-(1,2,4-Triazol-4-yl)-1H-indol-3-yl)propyl]-4-[N-(2-(4-acetylaminophenyl)ethyl-N-methylamino]piperidin,
(197) 1-[3-(5-(1,2,4-Triazol-4-yl)-1H-indol-3-yl)propyl]-4-[N-(4-acetylaminobenzyl)-N-methylamino]methylpiperidin,
(198) 1-[3-(5-(1,2,4-Triazol-4-yl)-1H-indol-3-yl)propyl]-4-[N-(thien-2-yl)methyl-N-methylamino]piperidin,
(199) 1-[3-(5-(1,2,4-Triazol-4-yl)-1H-indol-3-yl)propyl]-4-[(R)-α-(hydroxymethyl)benzylamino]methylpiperidin,
(200) 3-(4-Acetylaminobenzyl)aminomethyl-1-[2-(5-(1,2,4-triazol-4-yl)-1H-indol-3-yl)ethyl]pyrrolidin,
(201) (3R)-3-(N-Benzyl)aminomethyl-1-[2-(5-(1,2,4-triazol-4-yl)-1Hindol-3-yl)ethyl]pyrrolidin,
(202) (3S)-3-(Pyridin-4-ylmethyl)aminomethyl-1-[2-(5-(1,2,4-triazol-4-yl)-1H-indol-3-yl)ethyl]pyrrolidin,
(203) 3-(N-Benzyl)aminomethyl-1-[2-(5-(1,2,4-triazol-4-yl)-1H-indol-3-yl)ethyl] azetidin,
(204) 4-Benzyl-4-hydroxy-1-[3-(5-(1,2,4-triazol-4-yl)-1H-indol-3-yl)propyl]piperidin,
(205) 3-(N-Benzyl)aminomethyl-1-[3-(5-(1,2,4-triazol-4-yl)-1H-indol-3-yl)propyl]azetidin,
(206) 4-(N-Benzyl)aminomethyl-4-hydroxy-1-[3-(5-(1,2,4-triazol-4-yl)-1H-indol-3-yl)propyl]piperidin,
(207) 4-(N-Benzyl-N-methyl)aminomethyl-4-hydroxy-1-[3-(5-(1,2,4-triazol-4-yl)-1H-indol-3-yl)propyl]piperidin,
(208) 3-(N-Benzyl-N-methyl)aminomethyl-1-[2-(5-(1,2,4-triazol-4-yl)-lH-indol-3-yl)ethyl]azetidin,
(209) (3S)-3-[N-α-(Methyl)benzyl]aminomethyl-1-[2-(5-(1,2,4-triazol-4-yl)-1H-indol-3-yl)ethyl]pyrrolidin,
(210) (3S)-3-[N-(Furan-3-ylmethyl)amino]methyl-1-[2-(5-(1,2,4-triazol-4-yl)-1H-indol-3-yl)ethyl]pyrrolidin,
(211) (3S)-3-[N-(Furan-2-ylmethyl)amino]methyl-1-[2-(5-(1,2,4-triazol-4-yl)-1H-indol-3-yl)ethyl]pyrrolidin,
(212) (3S)-3-[N-α-(Hydroxymethyl)benzyl]aminomethyl-1-[2-(5-(1,2,4-triazol-4-yl)-1H-indol-3-yl)ethyl]pyrrolidin,
(213) (3S)-3-[N-Benzyl-N-(2-hydroxy)ethyl]aminomethyl-1-[2-(5-(1,2,4-triazol-4-yl)-1H-indol-3-yl)ethyl]pyrrolidin,
(214) (3S)-3-[N-(2-Phenylethyl)amino]methyl-1-[2-(5-(1,2,4-triazol-4-yl)-1H-indol-3-yl)ethyl]pyrrolidin,
(215) (3S)-3-[N-(2-Phenylethyl)-N-methylamino]methyl-1-[2-(5-(1,2,4-triazol-4-yl)-1H-indol-3-yl)ethyl]pyrrolidin,
(216) (3S)-3-(N-α-Dimethylbenzyl)aminomethyl-1-[2-(5-(1,2,4-triazol-4-yl)-1H-indol-3-yl)ethyl]pyrrolidin,
(217) (3S)-3-[N-(S)-α-Methylbenzyl]aminomethyl-1-[2-(5-(1,2,4-triazol-1-yl)-1H-indol-3-yl)ethyl]pyrrolidin,
(218) (3S)-3-[N-(R)-α-(Hydroxymethyl)benzyl]aminomethyl-1-[2-(5-(1,2,4-triazol-1-yl)-1H-indol-3-yl)ethyl]pyrrolidin,
(219) (3S)-3-(N-Benzyl)aminomethyl-1-[2-(5-(1,2,4-triazol-1-ylmethyl)-1H-indol-3-yl)ethyl]pyrrolidin,
(220) (3S)-3-[N-(S)-α-Methylbenzyl]aminomethyl-1-[2-(5-(1,2,4-triazol-1-ylmethyl)-1H-indol-3-yl)ethyl]pyrrolidin,
(221) (3S)-3-[N-(R)-α-(Hydroxymethyl)benzyl]aminomethyl-1-[2-(5-(1,2,4-triazol-1-ylmethyl)-1H-indol-3-yl)ethyl]pyrrolidin,
(222) (3S)-3-(N-Benzyl-N-methyl)aminomethyl-1-[2-(5-(imidazol-1-yl)-1H-indol-3-yl)ethyl]pyrrolidin,
(223) (3S)-3-(N-Benzyl-N-methyl)aminomethyl-1-[2-(1,2,4-triazol-1-ylmethyl)-1H-indol-3-yl)ethyl]pyrrolidin,
(224) (3R)-3-[N-Methyl-N-(S)-α-methylbenzyl]aminomethyl-1-[2-(5-(1,2,4-triazol-1-ylmethyl)-1H-indol-3-yl)ethyl]pyrrolidin,
(225) (3R)-3-[N-Methyl-N-(R)-α-hydroxymethylbenzyl]aminomethyl-1-[2-(5-(1,2,4-triazol-1-ylmethyl)-1H-indol-3-yl)ethyl)pyrrolidin,
(226) (3R)-3-[N-Methyl-N-(S)-α-Methylcyclohexylmethyl]aminomethyl-1-[2-(5-(1,2,4-triazol-1-ylmethyl)-1H-indol-3-yl)ethyl]-pyrrolidin,
(227) (3R)-3-[3-(R)-Hydroxy-2-(R)-phenylpiperidin-1-yl]methyl-1-[2-(5-(1,2,4-triazol-1-ylmethyl)-1H-indol-3-yl)ethyl]pyrrolidin,
(228) (3R)-3-[3-(R)-Hydroxy-2-(R)-phenylpiperidin-1-yl]methyl-1-[2-(5-(1,2,4-triazol-1-yl)-1H-indol-3-yl)ethyl]pyrrolidin,
(229) 4-Hydroxy-4-(phenylsulfinyl)methyl-1-[3-(5-(1,2,4-triazol-4-yl)-1H-indol-3-yl)propyl]piperidin,
(230) (3R)-3-[2-(R,S)-Phenylpiperidin-1-yl]methyl-1-[2-(5-(1,2,4-triazol-1-ylmethyl)-1H-indol-3-yl)ethyl]pyrrolidin,
(231) 4-(3,3-Dimethylpiperidin-1-yl)methyl-4-hydroxy-1-[3-(5-(1,2,4-triazol-4-yl)-1H-indol-3-yl)propyl]piperidin,
(232) 4-Hydroxy-4-(1,2,3,4-tetrahydroisochinolin-2-yl)methyl-1-[3-(5-(1,2,4-triazol-4-yl)-1H-indol-3-yl)propyl]piperidin,
(233) 4-Hydroxy-4-(N-isobutyl-N-methyl)aminomethyl-1-[3-(5-(1,2,4-triazol-4-yl)-1H-indol-3-yl)propyl]piperidin,
(234) 4-[N-Benzyl-N-(2-hydroxyethyl)amino]methyl-4-hydroxy-1-[3-(5-(1,2,4-triazol-4-yl)-1H-indol-3-yl)propyl]piperidin,
(235) 4-[N-(2,2-Dimethylpropyl)-N-methylamino]methyl-4-hydroxy-1-[3-(5-(1,2,4-triazol-4-yl)-1H-indol-3-yl)propyl]piperidin,
(236) 4-[N-(R)-α-Hydroxymethylbenzyl-N-methylamino]methyl-4-hydroxy-1-[3-(5-(1,2,4-triazol-4-yl)-1H-indol-3-yl)propyl]piperidin,
(237) 4-Hydroxy-4-(2-pyridylmethyl)aminomethyl-1-[3-(5-(1,2,4-triazol-4-yl)-1H-indol-3-yl)propyl]piperidin,
(238) 4-Hydroxy-4-(2-methylphenylmethyl)aminomethyl-1-[3-(5-(1,2,4-triazol-4-yl)-1H-indol-3-yl)propyl]piperidin,
(239) 4-Hydroxy-4-[N-(2-methylphenylmethyl)-N-methylamino]methyl-1-[3-(5-(1,2,4-triazol-4-yl)-1H-indol-3-yl)propyl]piperidin,
(240) 3-(Benzylamino)methyl-3-hydroxy-1-[3-(5-(1,2,4-triazol-4-yl)-1H-indol-3-yl)propyl]pyrrolidin,
(241) 3-(Benzylamino)methyl-3-hydroxy-1-[2-(5-(1,2,4-triazol-4-yl)-1H-indol-3-yl)ethyl]pyrrolidin,
(242) 1-[3-(5-(1,2,4-Triazol-4-yl)-1H-indol-3-yl)propyl]-4-[(R)-α-(carbamoyloxymethyl)benzylamino]piperidin,
(243) 1-[3-(5-(1,2,4-Triazol-4-yl)-1H-indol-3-yl)propyl)-4-[(1R,2S)-2-hydroxy-1-phenylpropylamino]piperidin,
(244) 1-[3-(5-(1,2,4-Triazol-4-yl)-1H-indol-3-yl)propyl]-4-[(1R,2R)-2-hydroxy-1-phenylpropylamino]piperidin,
(245) 1-[3-(5-(1,2,4-Triazol-4-yl)-1H-indol-3-yl)propyl]-4-[(R,S)-1-hydroxy-2-phenylprop-2-ylamino]piperidin,
(246) 1-[3-(5-(1,2,4-Triazol-4-yl)-1H-indol-3-yl)propyl]-4-[(R)-2-hydroxy-1-(4-fluorphenyl)ethylamino]piperidin,
(247) 1-[3-(5-(1,2,4-Triazol-4-yl)-1H-indol-3-yl)propyl]-4-[(1R,2R)-2-hydroxyindan-1-ylamino)piperidin,
(248) 1-[3-(5-(1,2,4-Triazol-4-yl)-1H-indol-3-yl)propyl]-4-[(R,S)-indan-1-ylamino]piperidin,
(249) 1-[3-(5-(1,2,4-Triazol-4-yl)-1H-indol-3-yl)propyl]-4-[(R,S)-1-(4-fluorphenyl)ethylamino]piperidin,
(250) 1-[3-(5-(1,2,4-Triazol-4-yl)-1H-indol-3-yl)propyl]-4-[(R)-1-phenylprop-2-ylamino]piperidin,
(251) 1-[3-(5-(1,2,4-Triazol-4-yl)-1H-indol-3-yl)propyl]-4-[N-(thien-3-ylmethyl)-N-methylamino]piperidin,
(252) 1-[3-(5-(1,2,4-Triazol-4-yl)-1H-indol-3-yl)propyl]-4-[N-(furan-3-ylmethyl)-N-methylamino]piperidin,
(253) 1-[3-(5-(1,2,4-Triazol-4-yl)-1H-indol-3-yl)propyl]-4-(furan-3-ylmethyl)aminopiperidin,
(254) 1-[3-(5-(1,2,4-Triazol-4-yl)-1H-indol-3-yl)propyl]-4-[N,N-di(furan-3-ylmethyl)amino]piperidin,
(255) 1-[3-(5-(1,2,4-Triazol-4-yl)-1H-indol-3-yl)propyl]-4-[N-(3,3-dimethylallyl)-N-methylamino]piperidin,
(256) 1-[3-(5-(1,2,4-Triazol-4-yl)-1H-indol-3-yl)propyl]-4-(N-allyl-N-methylamino)piperidin,
(257) N,N-Dimethyl-2-[5-(1,2,4-triazol-1-ylmethyl)-1H-indol-3-yl]ethylamin,
(258) N,N-Dimethyl-2-[5-(1,3-imidazol-1-ylmethyl)-1H-indol-3-yl]-ethylamin,
(259) N,N-Dimethyl-2-[5-(5-methyl-1,2,3,4-triazol-1-ylmethyl)-1Hindol-3-yl]ethylamin,
(260) N,N-Dimethyl-2-[5-<1,3,4-triazol-1-ylmethyl)-1H-indol-3-yl]ethylamin,
(261) N,N-Dimethyl-2-[5-(1,3,4-triazol-1-yl)-1H-indol-3-yl]ethylamin,
(262) N,N-Diethyl-2-[5-(1,2,4-triazol-1-ylmethyl)-1H-indol-3-yl]-ethylamin,
(263) N,N-Diethyl-2-[5-(1,3-imidazol-1-ylmethyl)-1H-indol-3-yl]-ethylamin,
(264) N,N-Diethyl-2-[5-(5-methyl-1,2,3,4-tetrazol-1-ylmethyl)-1Hindol-3-yl]ethylamin,
(265) N,N-Diethyl-2-[5-(1,3,4-triazol-1-ylmethyl)-1H-indol-3-yl]-ethylamin,
(266) N,N-Diethyl-2-[5-(1,3,4-triazol-1-yl)-1H-indol-3-yl]ethylamin,
(267) N,N-Dimethyl-[5-(1,2,4-triazol-1-ylmethyl)-1H-indol-3-yl]-methylamin,
(268) N,N-Dimethyl-[5-(1,3-imidazol-1-ylmethyl)-1H-indol-3-yl]-methylamin,
(269) N,N-Dimethyl-[5-(5-methyl-1,2,3,4-tetrazol-1-ylmethyl)-1Hindol-3-yl]methylamin,
(270) N,N-Dimethyl-[5-(1,3,4-triazol-1-ylmethyl)-1H-indol-3-yl]-methylamin,
(271) N,N-Dimethyl-[5-(1,3,4-triazol-1-yl)-1H-indol-3-yl]methylamin,
(272) N,N-Diethyl-3-[5-(1,2,4-triazol-1-ylmethyl)-1H-indol-3-yl]-propylamin,
(273) N,N-Dimethyl-3-[5-(1,3-imidazol-1-yl)-1H-indol-3-yl]propylamin,
(274) N,N-Diethyl-3-[5-(5-methyl-1,2,3,4-tetrazol-1-ylmethyl)-1Hindol-3-yl]propylamin,
(275) N,N-Dimethyl-3-[5-(1,3,4-triazol-1-ylmethyl)-1H-indol-3-yl]propylamin,
(276) N,N-Diethyl-3-[5-(1,3,4-triazol-1-yl)-1H-indol-3-yl]propylamin,
(277) N,N-Dimethyl-4-[5-(3-methyl-1,2,4,5-tetrazol-1-ylmethyl)-1Hindol-3-yl]butylamin,
(278) N,N-Dimethyl-4-[5-(2-ethyl-1,3-ethylimidazol-1-ylmethyl)-1Hindol-3-yl]butylamin,
(279) N,N-Dimethyl-4-[5-(5-ethyl-1,2,3,4-triazol-1-ylmethyl)-1Hindol-3-yl]butylamin,
(280) N,N-Dimethyl-4-[5-(2-methyl-1,3,4-triazol-1-ylmethyl)-1Hindol-3-yl]butylamin,
(281) N,N-Dimethyl-4-[5-(2-ethyl-1,3,4-triazol-1-yl)-1H-indol-3-yl]butylamin,
(282) 2-[5-(1,2,4-Triazol-1-ylmethyl)-1H-indol-3-yl]ethylalkohol,
(283) 2-[5-(1,3-Imidazol-1-ylmethyl)-1H-indol-3-yl]ethylalkohol,
(284) 2-[5-(5-Methyl-1,2,3,4-tetrazol-1-ylmethyl)-1H-indol-3-yl]-ethylalkohol,
(285) 2-[5-(1,3,4-Triazol-1-ylmethyl)-1H-indol-3-yl]ethylalkohol,
(286) 2-[5-(1,3,4-Triazol-1-yl)-1H-indol-3-yl]ethylalkohol,
(287) [5-(1,2,4-Triazol-1-ylmethyl)-1H-indol-3-yl]methylalkohol,
(288) 3-[5-(1,3-Imidazol-1-ylmethyl)-1H-indol-3-yl)propylalkohol,
(289) 4-[5-(5-Methyl-1,2,3,4-tetrazol-1-ylmethyl)-1H-indol-3-yl]-butylalkohol,
(290) 2-[5-(2-Methyl-1,3,4-triazol-1-ylmethyl)-1H-indol-3-yl]ethylalkohol und
(291) 2-[5-(5-Methyl-1,3,4-triazol-1-yl)-1H-indol-3-yl]ethylalkohol.

20. Das Verfahren gemäß Anspruch 18, bei dem die Verbindung der Strukturformel IV ausgewählt ist aus: 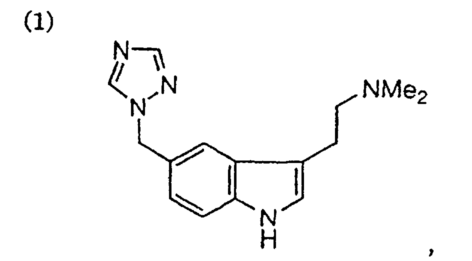 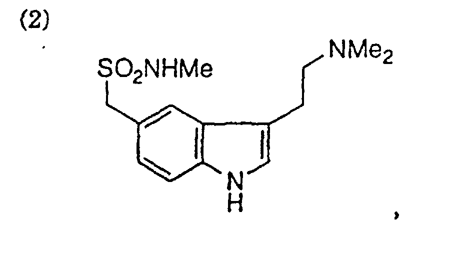 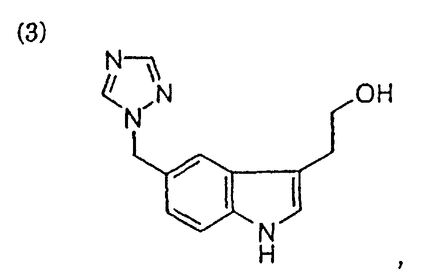 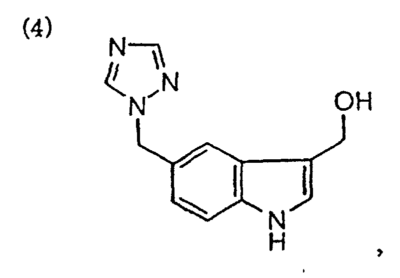 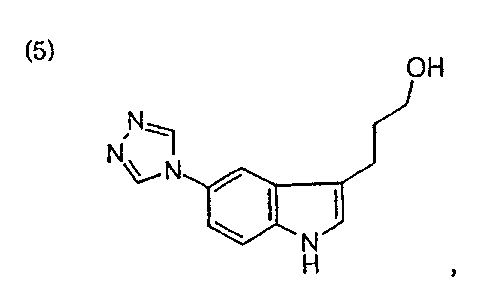 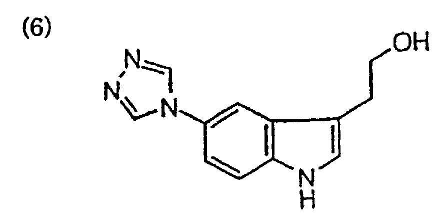 und

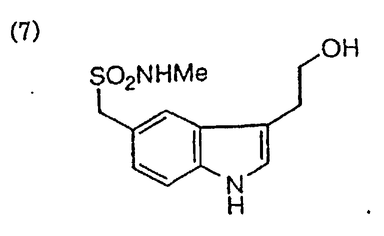

21. Die Verbindungen der Strukturformeln (V) und (VI): 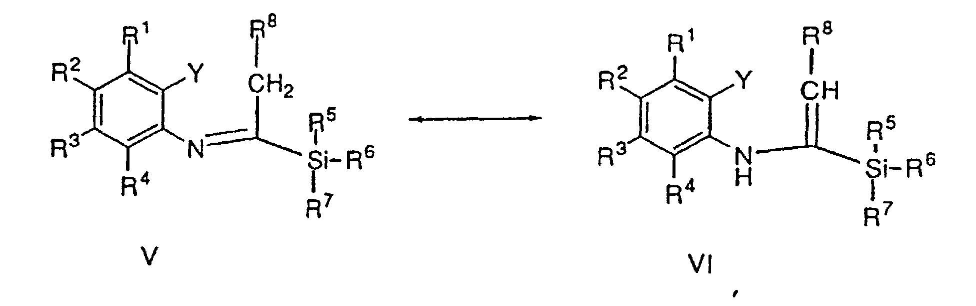 wobei:
Y ausgewählt ist aus Br, I und Triflat,
R¹, R², R³ und R⁴ jeweils unabhängig ausgewählt sind aus:
(1) Wasserstoff,
(2) 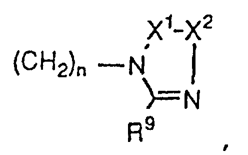
(3) C₁₋₆-Alkyl,
(4) -(CH₂)ₙ-Z,
wobei Z bedeutet:
(a) Wasserstoff,
(b) Halogen,
(c) Cyano,
(d) Nitro,
(e) Trifluormethyl,
(f) OR¹⁰,
(g) OCOR¹⁰,
(h) OCONR¹⁰R¹¹,
(i) OCH₂CN,
(j) OCH₂CONR¹⁰R¹¹,
(k) SR¹⁰,
(l) SOR¹⁰,
(m) SO₂R¹⁰, mit der Maßgabe, daß R¹⁰ nicht Wasserstoff ist,
(n) SO₂NR¹⁰R¹¹,
(o) NR¹⁰R¹¹,
(p) NR¹⁰COR¹¹,
(q) NR¹⁰CO₂R¹¹,
(r) NR¹⁰SO₂R¹¹,
(s) COR¹⁰,
(t) CO₂R¹⁰,
(u) CONR¹⁰R¹¹,
oder Z eine Gruppe der Formel (Za), (Zb), (Zc) oder (Zd) ist: 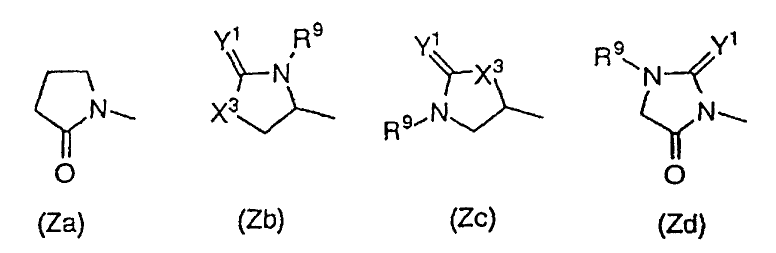
oder Z einen gegebenenfalls substituierten fünfgliedrigen heteroaromatischen Ring, ausgewählt aus Furan, Thiophen, Pyrrol, Oxazol, Thiazol, Isoxazol, Isothiazol, Imidazol, Pyrazol, Oxadiazol, Thiadiazol, Triazol und Tetrazol, bedeutet,
R⁵, R⁶ und R⁷ jeweils unabhängig ausgewählt sind aus:
(1) C₁₋₆-Alkyl,
(2) -O-C₁₋₆-Alkyl und
(3) Phenyl,
R⁸ ausgewählt ist aus:
(1) Wasserstoff,
(2) -R¹⁹-OH,
(3) -R¹⁹-O-R¹⁷ und
(4) -R¹⁹NR¹²R¹³ und
(5) -R¹⁹-Z¹,
wobei: Z¹ ein 3- bis 7gliedriger heterocyclischer Ring ist, wobei die Ringelemente ausgewählt sind aus 1 bis 2 Stickstoffatomen, und wobei der heterocyclische Ring durch einen oder mehrere Reste R¹⁴ substituiert sein kann,
R⁹ ausgewählt ist aus:
(1) Wasserstoff und
(2) C₁₋₄-Alkyl,
R¹⁰ und R¹¹ jeweils unabhängig ausgewählt sind aus:
(1) Wasserstoff,
(2) C₁₋₆-Alkyl,
(3) Trifluormethyl,
(4) Phenyl, gegebenenfalls substituiert mit einem oder mehreren R²⁰-Substituenten,
(5) Methylphenyl, gegebenenfalls substituiert mit einem oder mehreren R²⁰-Substituenten, und
(6) einer Aryl-C₁₋₆-alkyl- oder Heteroaryl-C₁₋₆-alkylgruppe, gegebenenfalls substituiert mit einem oder mehreren R²⁰-Substituenten, oder
R¹⁰ und R¹¹, wenn sie durch ein Stickstoffatom verbunden sind, zusammen den Rest eines gegebenenfalls substituierten Azetidin-, Pyrrolidin-, Piperidin-, Morpholin- oder Piperazinrings bedeuten, der gegebenenfalls mit einem oder mehreren R¹⁸-Substituenten substituiert ist,
R¹² und R¹³ jeweils unabhängig ausgewählt sind aus:
(1) C₁₋₄-Alkyl,
(2) C₁₋₄-Alkyl-C₆-aryl, wobei das Aryl unsubstituiert oder mit einem bis drei Substituenten, ausgewählt aus Methyl, Halogen und Halogenmethyl, substituiert sein kann,
R¹⁴ ausgewählt ist aus:
(1) Aryl-C₁₋₆-alkyl, unsubstituiert oder substituiert mit einem bis drei R²⁰-Substituenten, und
(2) Heteroaryl-C₁₋₆-alkyl, unsubstituiert oder substituiert mit einem bis drei R²⁰-Substituenten,
R¹⁵ und R¹⁶ jeweils unabhängig ausgewählt sind aus
(1) Wasserstoff,
(2) C₁₋₆-Alkyl,
(3) C₃₋₇-Cycloalkyl,
(4) C₃₋₇-Cycloalkyl-C₁₋₆-alkyl,
(5) Indanyl,
(6) Aryl,
(7) Aryl-C₁₋₆-alkyl,
(8) C₃₋₇-Heterocycloalkyl,
(9) C₃₋₇-Heterocycloalkyl-C₁₋₆-alkyl,
(10) Heteroaryl und
(11) Heteroaryl-C₁₋₆-alkyl,
R¹⁷ ausgewählt ist aus einer Hydroxyschutzgruppe, die unter milder Säurehydrolyse entfernt werden kann,
R¹⁸ ausgewählt ist aus:
(1) C₁₋₆-Alkyl,
(2) Aryl-C₁₋₆-alkyl,
(3) C₁₋₆-Alkoxy,
(4) C₂₋₆-Alkyloxycarbonyl und
(5) C₁₋₆-Alkylaminocarbonyl,
R¹⁹ eine gerade oder verzweigte C₁₋₆-Alkylkette ist,
R²⁰ ausgewählt ist aus:
(1) Fluor,
(2) Cyano,
(3) Trifluormethyl,
(4) C₁₋₆-Alkyl,
(5) Halogen-C₁₋₆-alkyl,
(6) Aryl,
(7) Triazolyl,
(8) Tetrazolyl,
(9) C₁₋₆-Alkyltetrazolyl,
(10) Hydroxy,
(11) C₁₋₆-Alkoxy,
(12) C₁₋₆-Alkylthio,
(13) C₂₋₆-Alkoxycarbonyl,
(14) C₂₋₆-Alkylcarbonyl,
(15) C₁₋₆-Alkylsulfonyl,
(16) Arylsulfonyl,
(17) C₂₋₆-Alkylcarbonylamino,
(18) Arylcarbonylamino,
(19) C₂₋₆-Alkoxycarbonylamino,
(20) N-C₁₋₆-Alkyl-N-C₂₋₆-alkoxyamino,
(21) Carbonylamino,
(22) Mono- oder Diarylaminocarbonylamino,
(23) Pyrrolidinylcarbonylamino,
(24) Piperidinylcarbonylamino,
(25) Aminocarbonyl,
(26) Aminocarbonylamino,
(27) C₁₋₆-Alkylaminocarbonyl,
(28) C₁₋₆-Alkylaminocarbonylamino,
(29) Di-C₁₋₆-alkylaminocarbonyl,
(30) Di-C₁₋₆-alkylaminocarbonylamino,
(31) Pyrrolidinylcarbonylamino,
(32) Piperidinylcarbonylamino,
(33) Aminosulfonyl,
(34) C₁₋₆-Alkylaminosulfonyl,
(35) C₁₋₆-Alkylsulfonylamino,
(36) C₁₋₆-Alkylsulfonylaminomethyl,
(37) Arylsulfonylamino,
(38) Di-C₁₋₆-alkylaminosulfonyl,
(39) Aminosulfonylmethyl,
(40) C₁₋₆-Alkylaminosulfonylmethyl und
(41) Di-C₁₋₆-alkylaminosulfonylmethyl,
(42) (CH₂)ₘOR¹⁵,
(43) (CH₂)ₘSR¹⁵, mit der Maßgabe, daß R¹⁵ nicht Wasserstoff ist,
(44) (CH₂)ₘSOR¹⁵,
(45) (CH₂)ₘSO₂R¹⁵,
(46) (CH₂)ₘNR¹⁵R¹⁶,
(47) =O und
(48) 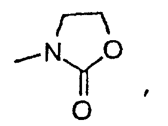
X¹ und X² jeweils unabhängig ausgewählt sind aus Ring-Stickstoff- oder Ring-Kohlenstoffatomen,
X³ ausgewählt ist aus der Gruppe, bestehend aus Sauerstoff, Schwefel, -NHoder Methylen,
Y¹ Sauerstoff oder Schwefel ist,
n eine ganze Zahl ist, die bei jedem Vorkommen unabhängig ausgewählt ist aus 0 und 1, und
m eine ganze Zahl ist, die bei jedem Vorkommen unabhängig ausgewählt ist aus 0 bis 4.

## Revendications

1. Procédé pour la préparation d'un composé de formule structurale III : 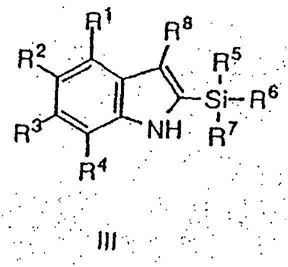 comprenant la réaction d'un composé de formule structurale I avec un acylsilane de formule structurale II : 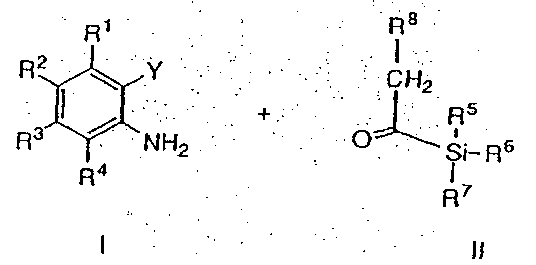 en présence d'un catalyseur au palladium et d'un accepteur de proton, dans lequel :
Y est choisi parmi Br, I et un triflate, et R¹, R², R³ et R⁴ sont chacun indépendamment choisis parmi :
(1) un hydrogène
(2) 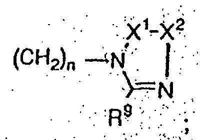
(3) un alkyle C₁₋₆
(4) -(CH₂)ₙ-Z
dans lequel Z représente :
(a) un hydrogène,
(b) un halogène,
(c) un cyano,
(d) un nitro,
(e) un trifluorométhyle,
(f) -OR¹⁰,
(g) -OCOR¹⁰,
(h) -OCONR¹⁰R¹¹,
(i) -OCH₂CN,
(j) -OCH₂CONR¹⁰R¹¹,
(k) -SR¹⁰,
(l) -SOR¹⁰
(m) -SO₂R¹⁰,
(n) -SO₂NR¹⁰R¹¹,
(o) -NR¹⁰R¹¹,
(p) -NR¹⁰COR¹¹,
(q) -NR¹⁰CO₂R¹¹,
(r) -NR¹⁰SO₂R¹¹,
(s) -COR¹⁰,
(t) -CO₂R¹⁰,
(u) -CONR¹⁰R¹¹,
ou un groupe de formule (Za), (Zb), (Zc), ou (Zd) : 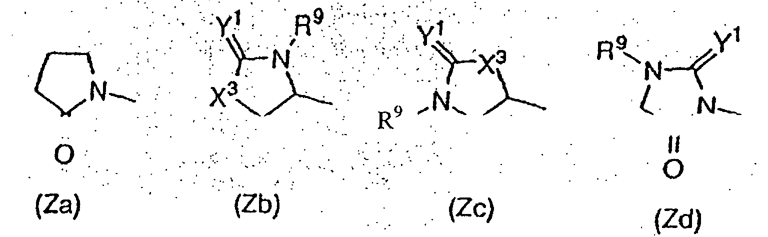 où Z représente un cycle hétéroaromatique à 5 chaînons facultativement substitué choisi parmi le furane, le thiophène, le pyrrole, l'oxazole, le thiazole, l'isoxazole, l'isothiazole, l'imidazole, le pyrazole, l'oxadiazole, le thiadiazole, le triazole et le tétrazole ;
R⁵, R⁶, et R⁷ sont chacun indépendamment choisis parmi ;
(1) un alkyle C₁₋₆,
(2) un -O-alkyle C₁₋₆,
(3) un phényle ;
R⁸ est choisi parmi :
(1) un hydrogène,
(2) -R¹⁹-OH,
(3) -R¹⁹-O-R¹⁷, et
(4) -R¹⁹NR¹²R¹³, et
(5) -R¹⁹-Z¹
dans lequel : Z¹ est un hétérocycle de 3 à 7 chaînons dans lequel les chaînons du cycle sont choisis parmi 1 ou 2 atomes d'azote et dans lequel l'hétérocycle peut être substitué par un ou plusieurs R¹⁴ ;
R⁹ est choisi parmi :
(1) un hydrogène, et
(2) un alkyle C₁₋₄ ;
R¹⁰ et R¹¹ sont chacun indépendamment choisis parmi :
(1) un hydrogène,
(2) un alkyle C₁₋₆,
(3) un trifluorométhyle,
(4) un phényle facultativement substitué avec un ou plusieurs substituants R²⁰, et
(5) un méthylphényle, substitué facultativement avec un ou plusieurs substituants R²⁰, et
(6) un groupe arylalkyle C₁₋₆ ou un groupe hétéroarylalkyle C₁₋₆ facultativement substitués avec un ou plusieurs substituants R²⁰, ou
R¹⁰ et R¹¹ lorsqu'ils sont liés par un atome d'azote, représentent ensemble le résidu d'une azétidine facultativement substituée, d'un cycle pyrrolydine, pipéridine, morpholine ou pipérazine facultativement substitués avec un ou plusieurs substituants R¹⁸ ;
R¹² et R¹³ sont chacun indépendamment choisis parmi :
(1) un alkyle C₁₋₄,
(2) un arylC₆-alkyle C₁₋₄ qui peut être non substitué ou substitué avec un à trois substituants choisis parmi un méthyle, un halogène, et un halogénométhyle,
R¹⁴ est choisi parmi :
(1) un arylalkyle C₁₋₆ non substitué ou substitué avec un à trois substituants R²⁰, et
(2) un hétéroarylalkyle C₁₋₆ non substitué ou substitué avec un à trois substituants R²⁰,
R¹⁵ et R¹⁶ sont chacun indépendamment choisis parmi
(1) un hydrogène,
(2) un alkyle C₁₋₆,
(3) un cycloalkyle C₃₋₇,
(4) un cycloalkylC₃₋₇-alkyle C₁₋₆
(5) un indanyle,
(6) un aryle,
(7) un arylalkyle C₁₋₆,
(8) un hétérocycloalkyle C₃₋₇,
(9) un hétérocycloalkylC₃₋₇-alkyle C₁₋₆,
(10) un hétéroaryle, et
(11) un hétéroarylalkyle C₁₋₆,
R¹⁷ est choisi parmi un groupe protecteur hydroxy qui est labile par hydrolyse acide douce ;
R¹⁸ est choisi parmi ;
(1) un alkyle C₁₋₆,
(2) un arylalkyle C₁₋₆,
(3) un alcoxy C₁₋₆,
(4) un alcoxycarbonyle C₂₋₆, et
(5) un alkylaminocarbonyle C₁₋₆,
R¹⁹ est une chaîne alkyle C₁₋₆ linéaire ou ramifiée ;
R²⁰ est choisi parmi :
(1) un fluoro,
(2) un cyano,
(3) un trifluorométhyle,
(4) un alkyle C₁₋₆,
(5) un halogénoalkyle C₁₋₆,
(6) un aryle,
(7) un triazolyle,
(8) un tétrazolyle,
(9) un tétrazolylalkyle C₁₋₆,
(10) un hydroxy,
(11) un alcoxy C₁₋₆,
(12) un alkylthio C₁₋₆,
(13) un alcoxycarbonyle C₂₋₆,
(14) un alkylcarbonyle C₂₋₆,
(15) un alkylsulfonyle C₁₋₆,
(16) un arylsulfonyle,
(17) un alkylcarbonylamino- C₂₋₆,
(18) un arylcarbonylamino-,
(19) un alcoxycarbonylamino- C₂₋₆,
(20) un N-alkylC₁₋₆-N-alcoxyamino- C₂₋₆,
(21) un carbonylamino-,
(22) un mono- ou un diarylaminocarbonylamino-,
(23) un pyrrolidinylcarbonylamino-,
(24) un pipéridinylcarbonylamino-,
(25) un aminocarbonyl-,
(26) un aminocarbonylamino-,
(27) un alkylC₁₋₆-aminocarbonyl-,
(28) un alkylC₁₋₆-aminocarbonyl-,
(29) un dialkylC₁₋₆-aminocarbonyl-,
(30) un dialkylC₁₋₆-aminocarbonylamino-,
(31) un pyrrolidinylcarbonylamino-,
(32) un pipéridinylcarbonylamino-,
(33) un aminosulfonyl-,
(34) un alkylC₁₋₆-aminosulfonyl-,
(35) un alkylC₁₋₆-sulfonylamino-,
(36) un alkylC₁₋₆-sulfonylaminométhyl-,
(37) un arylsulfonylamino-,
(38) un dialkylC₁₋₆-aminosulfonyl-,
(39) un aminosulfonylméthyl-,
(40) un alkylC₁₋₆-aminosulfonylméthyl-,
(41) un dialkylC₁₋₆-aminosulfonylméthyl-,
(42) un -(CH₂)ₘOR¹⁵,
(43) un -(CH₂)ₘSR¹⁵,
(44) un -(CH₂)ₘSOR¹⁵,
(45) un -(CH₂)ₘSO₂R¹⁵,
(46) un -(CH₂)ₘNR¹⁵R¹⁶,
(47) =O, et
(48) 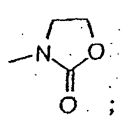
X¹ et X² sont chacun indépendamment choisis parmi des cycles azotés ou des cycles carbonés ;
X³ est choisi parmi le groupe constitué de l'oxygène, du soufre, de -NH- ou du méthylène ;
Y¹ est un oxygène ou un soufre ;
n est un nombre entier indépendamment choisi pour chaque événement compris entre 0 et 1 ; et
m est un nombre entier choisi indépendamment pour chaque événement compris entre 0 et 4.

2. Procédé selon la revendication 1 dans lequel R¹, R³ et R⁴ sont chacun un hydrogène.

3. Procédé selon la revendication 1 dans lequel le catalyseur au palladium est choisi parmi : un alkanoate de palladium, un acétonate de palladium, un halogénure de palladium, un complexe d'halogénure de palladium, un complexe acétone benzylidine-palladium et un complexe palladium triarylphosphine.

4. Procédé selon la revendication 3 dans lequel le catalyseur au palladium est choisi parmi :
de l'acétate de Pd(II), de l'acétylacétonate de Pd(II), de l'acétone de bis-dibenzylidène de Pd(0) (dba), du bromure de Pd(II), du chlorure de Pd(II), de l'iodure de Pd(II), du sulfate de Pd(II), du trifluoroacétate de Pd(II), du Cl₂(CH₃CN)₂ de Pd(II), du Pb₂(dba)₃, et du Cl₂(PhCN)₂ de Pd(II).

5. Procédé selon la revendication 4 dans lequel le catalyseur au palladium est de l'acétate de Pd(II).

6. Procédé selon la revendication 1 dans lequel l'accepteur de proton n'interagit pas avec le catalyseur au palladium pour inhiber son activité catalytique.

7. Procédé selon la revendication 6 dans lequel l'accepteur de proton est choisi parmi :
(a) une alkylamine,
(b) une amine aromatique,
(c) une amine hétérocyclique, et
(d) un phosphate.

8. Procédé selon la revendication 7 dans lequel l'accepteur de proton est une alkylamine.

9. Procédé selon la revendication 8 dans lequel l'alkylamine est choisie parmi :
(a) le 1,4-diazobicyclo[2.2.2]octane,
(b) la quinuclidine,
(c) la t-butylamine,
(d) la 2,2,6,6-tétraméthylpipéridine, et
(e) la di-t-butylamine.

10. Procédé selon la revendication 9 dans lequel l'alkylamine est le 1,4-diazobicyclo[2.2.2]octane.

11. Procédé selon la revendication 1 dans lequel la réaction est effectuée dans un solvant organique anhydre inerte pour les matières premières.

12. Procédé selon la revendication 11 dans lequel le solvant est choisi parmi :
(a) le DMSO,
(b) le DMF
(c) le DMAC, et
(d) le NMP.

13. Procédé selon la revendication 12 dans lequel le solvant est du DMF.

14. Procédé selon la revendication 1 dans lequel la réaction est effectuée à une température de 90°C à 120°C.

15. Procédé selon la revendication 1 comprenant de plus l'étape de déprotection du composé de formule structurale (III) pour obtenir le composé de formule structurale (IV) :

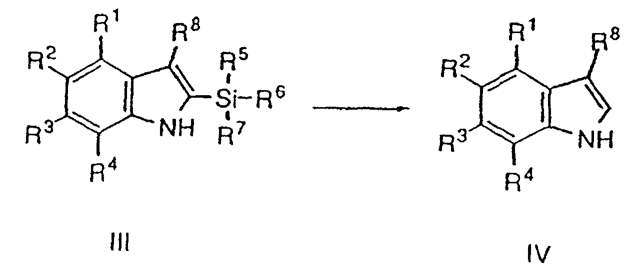

16. Procédé selon la revendication 15 dans lequel la déprotection est une déprotection catalysée par un acide de Lewis.

17. Procédé selon la revendication 15 dans lequel : R¹, R², R³ et R⁴ sont chacun indépendamment choisis parmi :
(1) un hydrogène
(2) 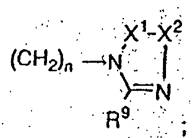
(3) un alkyle C₁₋₆
(4) -(CH₂)ₙ-Z
dans lequel Z représente :
(a) un hydrogène,
(b) un halogène,
(c) un cyano,
(d) un nitro,
(e) un trifluorométhyle,
(f) -OR¹⁰,
(g) -OCOR¹⁰,
(h) -OCONR¹⁰R¹¹,
(i) -OCH₂CN,
(j) -OCH₂CONR¹⁰R¹¹,
(k) -SR¹⁰, à condition que R¹⁰ ne soit pas un hydrogène
(l) -SOR¹⁰
(m) -SO₂R¹⁰,
(n) -SO₂NR¹⁰R¹¹,
(o) -NR¹⁰R¹¹,
(p) -NR¹⁰COR¹¹,
(q) -NR¹⁰CO₂R¹¹,
(r) -NR¹⁰SO₂R¹¹,
(s) -COR¹⁰,
(t) -CO₂R¹⁰,
(u) -CONR¹⁰R¹¹,
ou Z est un groupe de formule (Za), (Zb), (Zc), ou (Zd) : 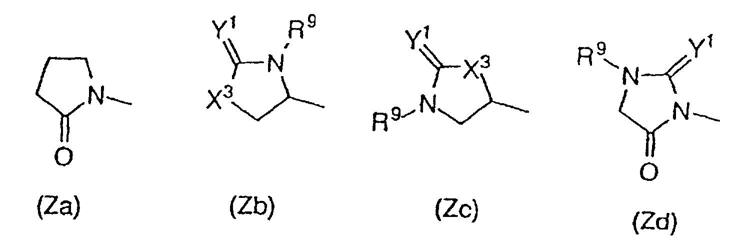 ou Z représente un cycle hétéroaromatique à 5 chaînons facultativement substitué choisi parmi le furane, le thiophène, le pyrrole, l'oxazole, le thiazole, l'isoxazole, l'isothiazole, l'imidazole, le pyrazole, l'oxadiazole, le thiadiazole, le triazole et le tétrazole ;
R⁵, R⁶, et R⁷ sont chacun indépendamment choisi parmi ;
(1) un alkyle C₁₋₆,
(2) un -O-alkyle C₁₋₆,
(3) un phényle ;
R⁸ est choisi parmi :
(1) un hydrogène,
(2) -R¹⁹-OH,
(3) -R¹⁹-O-R¹⁷, et
(4) -R¹⁹NR¹²R¹³, et
(5) -R¹⁹-Z¹
dans lequel : Z¹ est un hétérocycle de 3 à 7 chaînons dans lequel les chaînons du cycle sont choisis parmi 1 ou 2 atomes d'azote et dans lequel l'hétérocycle peut être substitué par un ou plusieurs R¹⁴ ;
R⁹ est choisi parmi :
(1) un hydrogène, et
(2) un alkyle C₁₋₄ ;
R¹⁰ et R¹¹ sont chacun indépendamment choisis parmi :
(1) un hydrogène,
(2) un alkyle C₁₋₆,
(3) un trifluorométhyle,
(4) un phényle facultativement substitué avec un ou plusieurs substituants R²⁰, et
(5) un méthylphényle, substitué facultativement avec un ou plusieurs substituants R²⁰, et
(6) un groupe arylalkyle C₁₋₆ ou un groupe hétéroarylalkyle C₁₋₆ facultativement substitués avec un ou plusieurs substituants R²⁰, ou
R¹⁰ et R¹¹ lorsqu' ils sont liés par un atome d'azote, représentent ensemble le résidu d'une azétidine facultativement substituée, d'un cycle pyrrolydine, pipéridine, morpholine ou pipérazine facultativement substitués avec un ou plusieurs substituants R¹⁸ ;
R¹² et R¹³ sont chacun indépendamment choisis parmi :
(1) un alkyle C₁₋₄,
(2) un arylC₆-alkyle C₁₋₄ qui peut être non substitué ou substitué avec un à trois substituants choisis parmi un méthyle, un halogène, et un halogénométhyle,
R¹⁴ est choisi parmi :
(1) un arylalkyle C₁₋₆ non substitué ou substitué avec un à trois substituants R²⁰, et
(2) un hétéroarylalkyle C₁₋₆ non substitué ou substitué avec un à trois substituants R²⁰,
R¹⁵ et R¹⁶ sont chacun indépendamment choisis parmi
(1) un hydrogène,
(2) un alkyle C₁₋₆,
(3) un cycloalkyle C₃₋₇,
(4) un cycloalkylC₃₋₇-alkyle C₁₋₆
(5) un indanyle,
(6) un aryle,
(7) un arylalkyle C₁₋₆,
(8) un hétérocycloalkyle C₃₋₇,
(9) un hétérocycloalkylC₃₋₇-alkyle C₁₋₆,
(10) un hétéroaryle, et
(11) un hétéroarylalkyle C₁₋₆,
R¹⁷ est choisi parmi un groupe protecteur hydroxy qui est labile par hydrolyse acide douce ;
R¹⁸ est choisi parmi ;
(1) un alkyle C₁₋₆,
(2) un arylalkyle C₁₋₆,
(3) un alcoxy C₁₋₆,
(4) un alcoxycarbonyle C₂₋₆,
(5) un alkylaminocarbonyle C₁₋₆,
R¹⁹ est une chaîne alkyle C₁₋₆ linéaire ou ramifiée ;
R²⁰ est choisi parmi :
(1) un fluoro,
(2) un cyano,
(3) un trifluorométhyle,
(4) un alkyle C₁₋₆,
(5) un halogénoalkyle C₁₋₆,
(6) un aryle,
(7) un triazolyle,
(8) un tétrazolyle,
(9) un tétrazolylalkyle C₁₋₆,
(10) un hydroxy,
(11) un alcoxy C₁₋₆,
(12) un alkylthio C₁₋₆,
(13) un alcoxycarbonyle C₂₋₆,
(14) un alkylcarbonyle C₂₋₆,
(15) un alkylsulfonyle C₁₋₆,
(16) un arylsulfonyle,
(17) un alkylcarbonylamino- C₂₋₆,
(18) un arylcarbonylamino-,
(19) un alcoxycarbonylamino- C₂₋₆,
(20) un N-alkylC₁₋₆-N-alcoxyamino- C₂₋₆,
(21) un carbonylamino-,
(22) un mono- ou un diarylaminocarbonylamino-,
(23) un pyrrolidinylcarbonylamino-,
(24) un pipéridinylcarbonylamino-,
(25) un aminocarbonyl-,
(26) un aminocarbonylamino-,
(27) un alkylC₁₋₆-aminocarbonyl-,
(28) un alkylC₁₋₆-aminocarbonyl-,
(29) un dialkylC₁₋₆-aminocarbonyl-,
(30) un dialkylC₁₋₆-aminocarbonylamino-,
(31) un pyrrolidinylcarbonylamino-,
(32) un pipéridinylcarbonylamino-,
(33) un aminosulfonyl-,
(34) un alkylC₁₋₆-aminosulfonyl-,
(35) un alkylC₁₋₆-sulfonylamino-,
(36) un alkylC₁₋₆-sulfonylaminométhyl-,
(37) un arylsulfonylamino-,
(38) un dialkylC₁₋₆-aminosulfonyl-,
(39) un aminosulfonylméthyl-,
(40) un alkylC₁₋₆-aminosulfonylméthyl-,
(41) un dialkylC₁₋₆-aminosulfonylméthyl-,
(42) un -(CH₂)ₘOR¹⁵,
(43) un -(CH₂)ₘSR¹⁵, à condition que R¹⁵ ne soit pas un hydrogène
(44) un -(CH₂)ₘSOR¹⁵,
(45) un -(CH₂)ₘSO₂R¹⁵,
(46) un -(CH₂)ₘNR¹⁵R¹⁶,
(47) =O, et
(48) 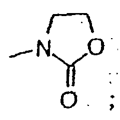
X¹ et X² sont chacun indépendamment choisis parmi des cycles azotés ou des cycles carbonés ;
X³ est choisi parmi le groupe constitué de l'oxygène, du soufre, de -NH- ou du méthylène ;
Y¹ est un oxygène ou un soufre ;
n est un nombre entier indépendamment choisi pour chaque événement compris entre 0 et 1 ; et
m est un nombre entier choisi indépendamment pour chaque événement compris entre 0 et 4.

18. Procédé selon la revendication 17 dans lequel R¹, R³, et R⁴ sont chacun un hydrogène.

19. Procédé selon la revendication 18 dans lequel le composé selon la formule structurale IV est choisi parmi :
(1) la 1-benzyl-4-[3-(5-(1,2,4-triazol-4-yl)-1H-indol-3-yl)propyl]pipérazin-2-one;
(2) la 1-(2-phényléthyl)-4-[3-(5-(1,2,4-triazol-4-yl)-1H-indol-3-yl)propyl]pipérazin-2-one ;
(3) la 1-[2-(3-fluorophényl)éthyl]-4-[3-(5-(1,2,4-triazol-4-yl)-1H-indol-3-yl)propyl]pipérazin-2-one;
(4) la (3S)-3-(N-benzyl)aminométhyl-1-[2-(5-(N-méthyl)-aminosulfonylméthyl)-1H-indol-3-yl)éthyl]pyrrolidine ;
(5) la (3S)-3-(N-benzyl)aminométhyl-1-[2-(5-(amino-sulfonylméthyl)-1H-indol-3-yl)éthyl]pyrrolidine ;
(6) la (3S)-3-(N-benzyl)aminométhyl-(S)-1-[2-(5-(2-oxo-1,3-oxazolidin-4-ylméthyl)-1H-indol-3-yl)éthyl]pyrrolidine ;
(7) la (3S)-3-[N-(R)-α-(hydroxyméthyl)benzyl]-aminométhyl-(S)-1-[2-(5-(2-oxo-1,3-oxazolidin-4-yl-méthyl)-1H-indol-3-yl)éthyl]pyrrolidine ;
(8) la (3S)-3-[N-(S)-α-méthylbenzyl]aminométhyl-(S)-1-[2-(5-(2-oxo-1,3-oxazolidin-4-ylméthyl)-1H-indol-3-yl)éthyl]pyrrolidine ;
(9) la 4-[N-(R)-α-(hydroxyméthyl)benzyl]amino-(S)-1-[3-(5-(2-oxo-1,3-oxazolidin-4-ylméthyl)-1H-indol-3-yl)propyl]pipéridine ;
(10) la (3S)-3-(N-benzyl-N-méthyl)aminométhyl-(S)-1-[2-(5-(3-méthyl-2-oxo-1,3-oxazolidin-4-ylméthyl)-1H-indol-3-yl)éthyl]pyrrolidine ;
(11) la (3R)-3-[N-(S)-α-méthylbenzyl-N-méthyl]amino-méthyl-(S)-1-[2-(5-(2-oxo-1,3-oxazolidin-4-ylméthyl)-1H-indol-3-yl)éthyl]pyrrolidine;
(12) la (3R)-3-[N-(S)-α-méthylbenzyl-N-méthyl]amino-méthyl-(S)-1-[2-(5-(3-méthyl-2-oxo-1,3-oxazolidin-4-ylméthyl)-1H-indol-3-yl)éthyl]pyrrolidine ;
(13) la (3S)-3-[N-(4-fluorobenzyl)-N-méthyl]aminométhyl-(S)-1-[2-(5-(3-méthyl-2-oxo-1,3-oxazolidin-4-ylméthyl)-1H-indol-3-yl)éthyl]pyrrolidine ;
(14) la 4-benzyl-12-[2-fluorométhyl-3-(5-(1,2,4-triazol-4-yl)-1H-indol-3-yl)propyl]pipérazine ;
(15) la 4-[2-(3-fluorophényl)éthyl]-1-[2-fluoro-3-(5-(1,2,4-triazol-4-yl)-1H-indol-3-yl)propyl]pipérazine ;
(16) la 4-benzyl-1-[2-fluoro-3-(5-(1,2,4-triazol-4-yl)-1H-indol-3-yl)propyl]pipéridine;
(17) la 4-(N-benzyl-N-méthylamino)-1-[2-fluoro-3-(5-(1,2,4-triazol-4-yl)-1H-indol-3-yl)propyl]pipéridine ;
(18) la 4-[(R)-2-hydroxy-1-(4-fluorophényl)éthylamino]-1-[2-fluoro-3-(5-(1,2,4-triazol-4-yl)-1H-indol-3-yl)propyl]pipéridine ;
(19) le 7-benzyl-2-[2-(5-(1,2,4-triazol-4-yl)-1H-indol-3-yl)éthyl]-*cis*-2,7-diazabicyclo[3.3.0]octane ;
(20) le 7-(3-furylméthyl-2-[2-(5-(1,2,4-triazol-4-yl)-1H-indol-3-yl)éthyl]-*cis*-2,7-diazabicyclo[3.3.0]-octane ;
(21) le 7-(2-phényléthyl-2-[2-(5-(1,2,4-triazol-4-yl)-1H-indol-3-yl)éthyl]-*cis*-2,7-diazabicyclo[3.3.0]-octane ;
(22) le 7-(4-fluorobenzyl-2-[2-(5-(1,2,4-triazol-4-yl)-1H-indol-3-yl)éthyl]-*cis*-2,7-diazabicyclo[3.3.0]-octane ;
(23) le 7-(2,4-difluorobenzyl-2-[2-(5-(1,2,4-triazol-4-yl)-1H-indol-3-yl)éthyl]-*cis*-2,7-diazabicyclo[3.3.0]-octane ;
(24) la 4-(2,2-difluoro-1-oxo-2-phényléthyl)-1-[3-(5-(1,2,4-triazol-4-yl)-1H-indol-3-yl)propyl]pipérazine ;
(25) la 4-benzyl-3-méthyl-1-[3-(5-(1,2,4-triazol-4-yl)-1H-indol-3-yl)propyl]-1,2,5,6-tétrahydropyridine ;
(26) la 4-benzyl-3-méthoxyméthyl-1-[3-(5-(1,2,4-triazol-4-yl)-1H-indol-3-yl)propyl]-1,2,5,6-tétrahydropyridine ;
(27) la 1-(2-hydroxy-1-phényléthyl)-4-[3-(5-(1,2,4-triazol-4-yl)-1H-indol-3-yl)propyl]pipéridine ;
(28) la 1-[2-(2-fluorophényl)éthyl]-4-[3-(5-(1,2,4-triazol-4-yl)-1H-indol-3-yl)propyl]pipéridine ;
(29) la 1-benzyl-4-[3-(5-(1,2,4-triazol-4-yl)-1H-indol-3-yl)propyl]pipéridine ;
(30) la 1-(3,3-diméthylbutyl)-4-[3-(5-(1,2,4-triazol-4-yl)-1H-indol-3-yl)propyl]pipéridine ;
(31) la 1-(2-phényléthyl)-4-[3-(5-(1,2,4-triazol-4-yl) -1H-indol-3-yl)propyl]pipéridine;
(32) la 1-cyclohexylméthyl-4-[3-(5-(1,2,4-triazol-4-yl)-1H-indol-3-yl)propyl]pipéridine ;
(33) la 1-(3-phénylpropyl)-4-[3-(5-(1,2,4-triazol-4-yl)-1H-indol-3-yl)propyl]pipéridine ;
(34) la 1-[2-(3-fluorophényl)éthyl]-4-[3-(5-(1,2,4-triazol-4-yl)-1H-indol-3-yl)propyl]pipéridine;
(35) la 1-[2-(4-trifluorométhylphényl)éthyl]-4-[3-(5-(1,2,4-triazol-4-yl)-1H-indol-3-yl)propyl]pipéridine ;
(36) la 1-[2-(3,4-difluorophényl)éthyl]-4-[3-(5-(1,2,4-triazol-4-yl)-1H-indol-3-yl)propyl]pipéridine;
(37) le N-méthyl-2-phényl-2-[4-(3-(5-(1,2,4-triazol-4-yl)-1H-indol-3-yl)propyl)pipéridin-1-yl]acétamide ;
(38) la 1-(2-oxo-2-phényléthyl)-4-[3-(5-(1,2,4-triazol-4-yl)-1H-indol-3-yl)propyl]pipéridine ;
(39) la 1-(2-phénylpropyl)-4-[3-(5-(1,2,4-triazol-4-yl)-1H-indol-3-yl)propyl]pipéridine ;
(40) la 4-benzyl-4-fluoro-1-[3-(5-(1,2,4-triazol-4-yl)-1H-indol-3yl)propyl]pipéridine ;
(41) la 4-fluoro-4-[2-(3-fluorophényl)éthyl]-1-[3-(5-(1,2,4-triazol-4-yl)-1H-indol-3-yl)propyl]pipéridine ;
(42) la 4-fluoro-4-(3-fluorobenzyl)-1-[3-(5-(1,2,4-triazol-4-yl)-1H-indol-3-yl)propyl]pipéridine ;
(43) la 4-fluoro-4-(2-fluorobenzyl)-1-[3-(5-(1,2,4-triazol-4-yl)-1H-indol-3-yl)propyl]pipéridine ;
(44) la 4-benzyl-4-méthoxy-1-[3-(5-(1,2,4-triazol-4-yl)-1H-indol-3-yl)propyl]pipéridine ;
(45) la 4-benzyl-4-méthoxy-1-[3-(5-(1,2,4-triazol-1-ylméthyl)-1H-indol-3-yl)propyl]pipéridine ;
(46) la 4-(2-fluorobenzyl)-4-méthoxy-1-[3-(5-(1,2,4-triazol-4-yl)-1H-indol-3-yl)propyl]pipéridine;
(47) la 4-(3-fluorobenzyl)-4-méthoxy-1-[3-(5-(1,2,4-triazol-4-yl)-1H-indol-3-yl)propyl]pipéridine ;
(48) la 4-(4-fluorobenzyl)-4-méthoxy-1-[3-(5-(1,2,4-triazol-4-yl)-1H-indol-3-yl)propyl]pipéridine;
(49) la 1-[3-(5-(1,2,4-triazol-4-yl)-1H-indol-3-yl)propyl]-4-[2-(oxazol-2-on-3-yl)-1-phényléthyl]pipérazine ;
(50) la 1-[3-(5-(1,2,4-triazol-4-yl)-1H-indol-3-yl)propyl]-4-[2-(oxazolidin-2-on-3-yl)-1-phényléthyl]-pipérazine ;
(51) la 1-[3-(5-(1,2,4-triazol-4-yl)-1H-indol-3-yl)propyl]-4-[1-(4-fluorophényl)-2-(oxazolidin-2-on-3-yl)-éthyl]pipérazine ;
(52) la 1-[3-(5-(1,2,4-triazol-4-yl)-1H-indol-3-yl)propyl]-4-(3-hydroxy-1-phénylpropyl)pipérazine;
(53) la 1-[3-(5-(1,2,4-triazol-4-yl)-1H-indol-3-yl)propyl]-4-[2-(imidazol-1-yl)-1-phényléthyl]-pipérazine ;
(54) la 1-[3-(5-(1,2,4-triazol-4-yl)-1H-indol-3-yl)propyl]-4-[1-(4-fluorophényl)-2-hydroxyéthyl]-pipérazine ;
(55) la 1-[3-(5-(1,2,4-triazol-4-yl)-1H-indol-3-yl)propyl]-4-[1-(4-fluorophényl)-2-méthoxyéthyl]-pipérazine ;
(56) la 1-[3-(5-(1,2,4-triazol-4-yl)-1H-indol-3-yl)propyl]-4-[3-(5-méthyl-1,2,4-oxadiazol-3-yl)-1-phénylpropyl]pipérazine ;
(57) la 1-[3-(5-(1,2,4-triazol-4-yl)-1H-indol-3-yl)propyl]-4-[2-benzyloxy-1-(4-fluorophényl)éthyl]-pipérazine ;
(58) la 1-[3-(5-(1,2,4-triazol-4-yl)-1H-indol-3-yl)propyl]-4-[1-(4-fluorophényl)-3-méthoxypropyl]-pipérazine ;
(59) la 1-[3-(5-(1,2,4-triazol-4-yl)-1H-indol-3-yl)propyl]-4-[1-(4-fluorophényl)-2-(imidazom-1-yl)éthyl]pipérazine ;
(60) la 1-[3-(5-(1,2,4-triazol-4-yl)-1H-indol-3-yl)propyl]-4-[1-phényl-2-(pyrrolidin-1-yl)éthyl]-pipérazine ;
(61) la 1-[3-(5-(1,2,4-triazol-4-yl)-1H-indol-3-yl)propyl]-4-[1-(4-fluorophényl)-3-hydroxypropyl]-pipérazine ;
(62) la 1-[3-(5-(1,2,4-triazol-4-yl)-1H-indol-3-yl)propyl]-4-[3-(imidazol-1-yl)-1-phénylpropyl]-pipérazine ;
(63) la 1-[3-(5-(1,2,4-triazol-4-yl)-1H-indol-3-yl)propyl]-4-(3-hydroxy-2-phénylpropyl)pipérazine ;
(64) la 1-[3-(5-(1,2,4-triazol-4-yl)-1H-indol-3-yl)propyl]-4-(3-méthoxy-2-phénylpropyl)pipérazine ;
(65) la 1-[3-(5-(1,2,4-triazol-4-yl)-1H-indol-3-yl)propyl]-4-[2-(4-fluorophényl)-3-hydroxypropyl]-pipérazine ;
(66) la 1-[3-(5-(1,2,4-triazol-4-yl)-1H-indol-3-yl)propyl]-4-[1-(4-fluorophényl)prop-2-yl]pipérazine;
(67) la l-[3-(5-(1,2,4-triazol-4-yl)-1H-indol-3-yl)propyl]-4-[2-(4-fluorophényl)propyl]pipérazine;
(68) la 1-[3-(5-(1,2,4-triazol-4-yl)-1H-indol-3-yl)propyl]-4-[1-(4-fluorophényl)-3-hydroxyprop-2-yl]pipérazine ;
(69) la 1-[3-(5-(1,2,4-triazol-4-yl)-1H-indol-3-yl)propyl]-4-[1-(4-fluorophényl)-3-méthoxyprop-2-yl]-pipérazine ;
(70) la 1-[3-(5-(1,2,4-triazol-4-yl)-1H-indol-3-yl)propyl]-4-[2-(3-fluorophényl)éthyl]pipérazine;
(71) la 1-[3-(5-(1,2,4-triazol-4-yl)-1H-indol-3-yl)propyl]-4-[2-(2-fluorophényl)éthyl]pipérazine;
(72) la 1-[3-(5-(1,2,4-triazol-4-yl)-1H-indol-3-yl)propyl]-4-[2-(4-fluorophényl)éthyl]pipérazine;
(73) la 1-[3-(5-(1,2,4-triazol-4-yl)-1H-indol-3-yl)propyl]-4-[2-(3-méthoxyphényl)éthyl]pipérazine ;
(74) la 1-[3-(5-(1,2,4-triazol-4-yl)-1H-indol-3-yl)propyl]-4-[2-(3-trifluorométhyl-phényl)éthyl]-pipérazine ;
(75) la 1-[3-(5-(1,2,4-triazol-4-yl)-1H-indol-3-yl)propyl]-4-[2-(3,4-difluorophényl)éthyl]pipérazine ;
(76) la 1-[3-(5-(1,2,4-triazol-4-yl)-1H-indol-3-yl)propyl]-4-[2-(2,4-difluorophényl)éthyl]pipérazine ;
(77) la 1-[3-(5-(1,2,4-triazol-4-yl)-1H-indol-3-yl)propyl]-4-[2-(3,5-difluorophényl)éthyl]pipérazine;
(78) la 1-[3-(5-(1,2,4-triazol-4-yl)-1H-indol-3-yl)propyl]-4-[2-(3-(oxazolidin-2-on-3-yl)phényl)éthyl]-pipérazine ;
(79) la N-méthyl-3-[5-(1,2,4-triazol-4-yl)-1H-indol-3-yl]pyrrolidine ;
(80) la N-méthyl-4-[5-(1,2,4-triazol-4-yl)-1H-indol-3-yl]pipéridine ;
(81) la N,N-diméthyl-2-[5-(1,2,4-triazol-4-yl)-1H-indol-3-yl]éthylamine;
(82) la 4-(1-phényléthyl)-1-[3-(5-(1,2,4-triazol-4-yl)-1H-indol-3-yl)propyl]-1,2,5,6-tétrahydropyridine ;
(83) la 4-(α-isopropyloxy)phénylméthyl)-1-[3-(5-(1,2,4-triazol-4-yl)-1H-indol-3-yl)propyl]-1,2,5,6-tétrahydro-pyridine;
(84) la 4-(α-méthoxy)phénylméthyl)-1-[3-(5-(1,2,4-triazol-4-yl)-1H-indol-3-yl)propyl]-1,2,5,6-tétrahydropyridine ;
(85) la 4-[α-(2-méthoxyéthyl)oxy]phénylméthyl-1-[3-(5-(1,2,4-triazol-4-yl)-1H-indol-3-yl)propyl]-1,2,5,6-tétrahydropyridine ;
(86) la 4-benzyl-1-[3-(2,3-dihydro-5-(1,2,4-triazol-4-yl)-1H-indol-3-yl)propyl]pipérazine ;
(87) la 1-[3-(2,3-dihydro-5-(1,2,4-triazol-4-yl)-1H-indol-3-yl)propyl]-4-(pyridin-3-ylméthyl)pipérazine ;
(88) la 1-[2-(5-(1,2,4-triazol-4-yl)-1H-indol-3-yl)propyl]-4-[2-(4-fluorophényl)-4-méthylpipérazin-1-yl]pipéridine ;
(89) la 1-[2-(5-(1,2,4-triazol-1-ylméthyl)-1H-indol-3-yl)éthyl]-3(R)-(3(R)-phénylmorpholin-4-ylméthyl)-pyrrolidine ;
(90) la 1-[3-(5-(1,2,4-triazol-4-yl)-1H-indol-3-yl)propyl]-4-(3-oxo-2-phénylpipérazin-1-yl)méthylpipéridine ;
(91) la 1-[3-(5-(1,2,4-triazol-4-yl)-1H-indol-3-yl)propyl]-4-(4-méthyl-2-phénylpipérazin-1-yl)-pipéridine ;
(92) la 1-[3-(5-(1,2,4-triazol-4-yl)-1H-indol-3-yl)propyl]-4-(benzimidazol-2-on-1-yl)pipéridine ;
(93) la 1-[3-(5-(1,2,4-triazol-4-yl)-1H-indol-3-yl)propyl]-4-[6-(4-fluorophényl)-4-méthyl-3-oxopipérazin-1-yl]pipéridine ;
(94) la [3-(3-(4-(2-(3,4-difluorophényl)éthyl)-pipérazin-1-yl)propyl)-1H-indol-5-ylméthyl]oxazolidin-2-one ;
(95) la (S)-4-[3-(3-(4-(2-(3,4-difluorophényl)éthyl)-pipérazin-1-yl)propyl)-1H-indol-5-ylméthyl]-3-méthyl-oxazolidin-2-one ;
(96) la 1-[3-(5-(N-méthylaminosulfonylméthyl)-1H-indol-3-ylpropyl]-4-[2-(4-(acétylamino)phényl)éthyl]-pipérazine ;
(97) le 3-benzyl-7-[3-(5-(1,2,4-triazol-4-yl)-1H-indol-3-ylpropyl]-*cis*-3,7-diazabicyclo[3.3.0]octane ;
(98) le 3-(pyridin-3-yl)méthyl-7-[3-(5-(1,2,4-triazol-4-yl)-1H-indol-3-ylpropyl]-cis-3,7-diazabicyclo[3.3.0]-octane;
(99) le 3-[2-(4-acétylamino)phényl)éthyl]-7-[3-(5-(1,2,4-triazol-4-yl)-1H-indol-3-ylpropyl]-cis-3,7-diazabicyclo[3.3.0]octane;
(100) le 3-benzoyl-7-[3-(5-(1,2,4-triazol-4-yl)-1H-indol-3-ylpropyl]-cis-3, 7-diazabicyclo[3.3.0]octane ;
(101) le (1RS,3RS,5RS)-7-benzyl-3-[5-(imidazol-1-yl)-1H-indol-3-ylméthyl]-2-méthyl-2,7-diazabicyclo[3.3.0]-octane ;
(102) le (1RS,3RS,5RS)-7-[2-(3-fluorophényl)éthyl]-2-méthyl-3-[5-(1,2,4-triazol-4-yl)-1H-indol-3-ylméthyl]-2,7-diazabicyclo[3.3.0]octane ;
(103) le (1RS,3RS,5RS)-7-(4-fluorobenzyl)-2-méthyl-3-[5-(1,2,4-triazol-4-yl)-1H-indol-3-ylméthyl]-2,7-diazabicyclo[3.3.0]octane ;
(104) la 4-[1-(phényl)-N,N-diméthylcarboxamidométhyl]-1-[3-(5-(1,2,4-triazol-4-yl)-1H-indol-3-yl)propyl]-pipérazine;
(105) la 4-(2-méthoxycarbonylamino-1-phényl)éthyl-1-[3-(5-(1,2,4-triazol-4-yl)-1H-indol-3-yl)propyl]-pipérazine ;
(106) la 4-(2-méthylamino-1-phényléthyl)-1-[3-(5-(1,2,4-triazol-4-yl)-1H-indol-3-yl)propyl]pipérazine;
(107) la 1-[3-(5-(1,2,4-triazol-4-yl)-1H-indol-3-yl)propyl]-4-(4-fluorophényl)méthylpipérazine ;
(108) la 4-[2-(N-méthyl-N-méthoxycarbonyl)amino-1-phényléthyl]-1-[3-(5-(1,2,4-triazol-4-yl)-1H-indol-3-yl)propyl]pipérazine ;
(109) la 1-benzyl-4-[(R,S)-2-hydroxyméthyl-3-(5-(1,2,4-triazol-4-yl)-1H-indol-3-yl)propyl]pipérazine;
(110) la 1-[3-(5-(1,2,4-triazol-4-yl)-1H-indol-3-yl)propyl]-4-[2-(1H-tétrazol-5-yl)phényl]méthylpipérazine ;
(111) la 1-[3-(5-(1,2,4-triazol-4-yl)-1H-indol-3-yl)propyl]-4-(2-phényléthyl)pipérazine ;
(112) la 4-benzyl-1-[3-(5-(1,2,4-triazol-4-yl)-1H-indol-3-yl)propyl]pipérazine ;
(113) la 4-[2-(2-méthyltétrazol-5-yl)phényl]méthyl-1-[3-(5-(1,2,4-triazol-4-yl)-1H-indol-3-yl)propyl]-pipérazine ;
(114) la 4-[2-(1-méthyltétrazol-5-yl)phényl]méthyl-1-[3-(5-(1,2,4-triazol-4-yl)-1H-indol-3-yl)propyl]-pipérazine ;
(115) la 4-[2-(N-méthylcarboxamido)phényl]méthyl-1-[3-(5-(1,2,4-triazol-4-yl)-1H-indol-3-yl)propyl]-pipérazine ;
(116) la 4-[2-(N,N-diméthylaminométhyl)phényl]méthyl-1-[3-(5-(1,2,4-triazol-4-yl)-1H-indol-3-yl)propyl]-pipérazine ;
(117) la 4-(but-3-ényl)-1-[3-(5-(1,2,4-triazol-4-yl)-1H-indol-3-yl)propyl]pipérazine ;
(118) la 4-(3-méthylbut-2-ényl)-1-[3-(5-(1,2,4-triazol-4-yl)-1H-indol-3-yl)propyl]pipérazine ;
(119) la 4-(prop-2-ényl)-1-[3-(5-(1,2,4-triazol-4-yl)-1H-indol-3-yl)propyl]pipérazine ;
(120) la 4-(prop-2-ynyl)-1-[3-(5-(1,2,4-triazol-4-yl)-1H-indol-3-yl)propyl]pipérazine ;
(121) la 4-[(R,S)-1-(phényl)carboxamidométhyl]-1-[3-(5-(1,2,4-triazol-4-yl)-1H-indol-3-yl)propyl]pipérazine ;
(122) la 4-[1-(phényl)-N-méthylcarboxamidométhyl]-1-[3-(5-(1,2,4-triazol-4-yl)-1H-indol-3-yl)propyl]-pipérazine ;
(123) la 1-[3-(5-(1,2,4-triazol-1-ylméthyl)-1H-indol-3-yl)propyl]-4-[2-(4-(acétylamino)phényl)éthyl]-pipérazine ;
(124) la 1-[3-(5-(1,2,4-triazol-4-yl)-1H-indol-3-yl)propyl]-4-[2-(3-(acétylamino)phényl)éthyl]-pipérazine ;
(125) la 1-[3-(5-(1,2,4-triazol-4-yl)-1H-indol-3-yl)propyl]-4-[4-(aminosulphonyl)phényl]méthylpipérazine ;
(126) la 1-[3-(5-(1,2,4-triazol-4-yl)-1H-indol-3-yl)propyl]-4-(furan-3-yl)méthylpipérazine ;
(127) la 1-[3-(5-(1,2,4-triazol-4-yl)-1H-indol-3-yl)propyl]-4-(furan-2-yl)méthylpipérazine ;
(128) la 1-[3-(5-(1,2,4-triazol-4-yl)-1H-indol-3-yl)propyl]-4-(thien-2-yl)méthylpipérazine;
(129) la 1-benzyl-4-[(R,S)-2-hydroxy-3-(5-(1,2,4-triazol-4-yl)-1H-indol-3-yl)propyl]pipérazine ;
(130) la 1-[2-(4-(acétylamino)phényl)éthyl]-4-[(R,S)-2-hydroxy-3-(5-(1,2,4-triazol-4-yl)-1H-indol-3-yl)propyl]pipérazine;
(131) la 1-[3-(5-(1,2,4-triazol-4-yl)-1H-indol-3-yl)propyl]-4-[2-(4-(aminocarbonylamino)phényl)éthyl]-pipérazine ;
(132) la 1-[3-(5-(1,2,4-triazol-4-yl)-1H-indol-3-yl)propyl]-4-(4-(cyanophényl)méthylpipérazine;
(133) la 1-[3-(5-(1,2,4-triazol-4-yl)-1H-indol-3-yl)propyl]-4-[2-(4-cyanophényl)éthyl]pipérazine ;
(134) la 1-[3-(5-(1,2,4-triazol-4-yl)-1H-indol-3-yl)propyl]-4-[2-(4-(1,2,4-triazol-4-yl)phényl)éthyl]-pipérazine ;
(135) la 1-[3-(5-(1,2,4-triazol-1-yl)-1H-indol-3-yl)propyl]-4-[2-(4-(acétylamino)phényl)éthyl]-pipérazine ;
(136) la 1-[3-(5-(1,2,4-triazol-1-yl)-1H-indol-3-yl)propyl]-4-benzylpipérazine ;
(137) la 1-[3-(5-(1,2,4-triazol-1-ylméthyl)-1H-indol-3-yl)propyl]-4-benzylpipérazine ;
(138) la 4-(4-acétylaminophényl)méthyl-1-[3-(5-(1,2,4-triazol-4-yl)-1H-indol-3-yl)propyl]pipérazine ;
(139) la 4-benzyl-1-[3-(5-(1,2,4-triazol-4-yl)-1H-indol-3-yl)propyl]-1,2,5,6-tétrahydropyridine ;
(140) la 1-[3-(5-(1,2,4-triazol-4-yl)-1H-indol-3-yl)propyl]-4-(2-aminopyridine-5-yl)méthylpipérazine ;
(141) la l-[3-(5-(1,2,4-triazol-4-yl)-1H-indol-3-yl)propyl]-4-(4-aminophényl)méthylpipérazine ;
(142) la 1-[4-(5-(1,2,4-triazol-4-yl)-1H-indol-3-yl)butyl]-4-benzylpipérazine ;
(143) la 1-[4-(5-(1,2,4-triazol-4-yl)-1H-indol-3-yl)butyl]-4-(pyridine-2-yl)méthylpipérazine ;
(144) la 1-[4-(5-(1,2,4-triazol-4-yl)-1H-indol-3-yl)butyl]-4-(pyridine-3-yl)méthylpipérazine ;
(145) la 1-[3-(5-(1,2,4-triazol-4-yl)-1H-indol-3-yl)propyl]-4-[2-(4-aminophényl)éthyl]pipérazine ;
(146) la 1-[3-(5-(1,2,4-triazol-4-yl)-1H-indol-3-yl)propyl]-4-[2-(4-acétylamino)phényl)éthyl]-pipérazine ;
(147) la 1-[3-(5-(1,2,4-triazol-4-yl)-1H-indol-3-yl)propyl]-4-(imidazol-2-yl)méthylpipérazine ;
(148) la 1-[3-(5-(1,2,4-triazol-4-yl)-1H-indol-3-yl)propyl]-4-[3-(acétylamino)phényl]méthylpipérazine;
(149) la 1-[3-(5-(1,2,4-triazol-4-yl)-1H-indol-3-yl)propyl]-4-[4-(acétylamino)phényl]méthylpipérazine;
(150) la 1-[4-(5-(1,2,4-triazol-4-yl)-1H-indol-3-yl)butyl]-4-[4-(acétylamino)phényl]méthylpipérazine;
(151) la 1-[3-(5-(1,2,4-triazol-4-yl)-1H-indol-3-yl)propyl]-4-(2-méthoxyphényl)méthylpipérazine ;
(152) la 1-[3-(5-(1,2,4-triazol-4-yl)-1H-indol-3-yl)propyl]-4-benzylpipérazine ;
(153) la 1-[3-(5-(1,2,4-triazol-4-yl)-1H-indol-3-yl)propyl]-4-(pyridine-3-yl)méthylpipérazine ;
(154) la 1-[3-(5-(1,2,4-triazol-4-yl)-1H-indol-3-yl)propyl]-4-(pyridine-2-yl)méthylpipérazine;
(155) la 1-[3-(5-(1,2,4-triazol-4-yl)-1H-indol-3-yl)propyl]-4-(pyridine-4-yl)méthylpipérazine;
(156) la (3R)-3-benzyloxyméthyl-1-[2-(5-(1,2,4-triazol-4-yl)-1H-indol-3-yl)éthyl]pyrrolidine;
(157) la (3S)-3-(N-benzyl-N-méthyl)aminométhyl-1-[2-(5-(1,2,4-triazol-4-yl)-1H-indol-3-yl)éthyl]pyrrolidine ;
(158) la (2S)-2-(N-benzyl-N-méthyl)aminométhyl-1-[2-(5-(1,2,4-triazol-4-yl)-1H-indol-3-yl)éthyl]pyrrolidine ;
(159) la (3S)-3-(N-benzyl)aminométhyl-1-[2-(5-(1,2,4-triazol-4-yl)-1H-indol-3-yl)éthyl]pyrrolidine;
(160) la 4-(4-acétylaminophényl)méthylamino-1-[3-(5-(1,2,4-triazol-4-yl)-1H-indol-3-yl)propyl]pipéridine ;
(161) la 1-[3-(5-(imidazol-1-yl)-1H-indol-3-yl)propyl]-4-[(R)-α-(méthoxyméthyl)benzylamino]pipéridine;
(162) la 1-[3-(5-(1,2,4-triazol-1-ylméthyl)-1H-indol-3-yl)propyl]-4-[(R)-1-(4-fluorophényl)-2-méthoxyéthyl-amino]pipéridine ;
(163) la 1-[3-(5-(1,2,4-triazol-1-ylméthyl)-1H-indol-3-yl)propyl]-4-[N-(4-fluorobenzyl)-N-méthylamino]-pipéridine ;
(164) la 1-[3-(5-(1,2,4-triazol-4-yl)-1H-indol-3-yl)propyl]-4-(2-phénylpipéridine-1-yl)pipéridine;
(165) la 1-[3-(5-(1,2,4-triazol-4-yl)-1H-indol-3-yl)propyl]-4-[(R)-1-(4-fluorophényl)-2-méthoxyéthylamino]pipéridine ;
(166) la 1-[3-(5-(1,2,4-triazol-4-yl)-1H-indol-3-yl)propyl]-4-(indan-1-ylaminométhyl)pipéridine;
(167) la 1-[3-(5-(1,2,4-triazol-4-yl)-1H-indol-3-yl)propyl]-4-[N-(R)-α-(hydroxyméthyl)benzyl-N-méthylaminométhyl]pipéridine ;
(168) la (3R)-3-(benzylthio)méthyl-1-[2-(5-(1,2,4-triazol-4-yl)-1H-indol-3-yl)éthyl]pyrrolidine ;
(169) la (±)-1-[3-(5-(1,2,4-triazol-4-yl)-1H-indol-3-yl)propyl]-4-[1-benzylamino-2-hydroxyéthyl)pipéridine ;
(170) la 1-[3-(5-(1,2,4-triazol-1-yl)-1H-indol-3-yl)propyl]-4-[(R)-α-(hydroxyméthyl)benzylamino]-pipéridine ;
(171) la 1-[3-(5-(imidazol-1-yl)-1H-indol-3-yl)propyl]-4-[(R)-α-(méthyl)benzylamino]pipéridine ;
(172) la 1-[3-(5-(imidazol-1-yl)-1H-indol-3-yl)propyl]-4-[(R)-α-(hydroxyméthyl)benzylamino]pipéridine ;
(173) la 1-[3-(5-(1,2,4-triazol-1-ylméthyl)-1H-indol-3-yl)propyl]-4-[(R)-α-(hydroxyméthyl)benzylamino]-pipéridine;
(174) la 1-[3-(5-(1,2,4-triazol-4-yl)-1H-indol-3-yl)propyl]-4-[(R)-α-(méthoxyméthyl)benzylamino]-pipéridine ;
(175) la 1-[3-(5-(1,2,4-triazol-4-yl)-1H-indol-3-yl)propyl]-4-[N-(R)-α-(méthoxyméthyl)benzyl-N-méthylamino]pipéridine ;
(176) la (3R)-3-benzyloxy-1-[2-(5-(1,2,4-triazol-4-yl)-1H-indol-3-yl)éthyl]pyrrolidine ;
(177) la (3R)-3-(4-méthoxyphényl)méthoxy-1-[2-(5-(1,2,4-triazol-4-yl)-1H-indol-3-yl)éthyl]pyrrolidine;
(178) la (3R)-3-(pyridine-3-yl)méthoxy-1-[2-(5-(1,2,4-triazol-4-yl)-1H-indol-3-yl)éthyl]pyrrolidine ;
(179) la (3R)-3-benzyloxyméthyl-1-[2-(5-(1,2,4-triazol-4-yl)-1H-indol-3-yl)éthyl]pyrrolidine;
(180) la (3S)-3-(N-benzyl-N-méthyl)aminométhyl-1-[2-(5-(1,2,4-triazol-4-yl)-1H-indol-3-yl)éthyl]pyrrolidine ;
(181) la (2S)-2-(N-benzyl-N-méthyl)aminométhyl-1-[2-(5-(1,2,4-triazol-4-yl)-1H-indol-3-yl)éthyl]pyrrolidine ;
(182) la (3S)-3-(N-benzyl)aminométhyl-1-[2-(5-(1,2,4-triazol-4-yl)-1H-indol-3-yl)éthyl]pyrrolidine ;
(183) la 4-(4-(acétylaminophényl)méthylamino-1-[3-(5-(1,2,4-triazol-4-yl)-1H-indol-3-yl)propyl]pipéridine ;
(184) la 4-benzylamino-1-[3-(5-(1,2,4-triazol-4-yl)-1H-indol-3-yl)propyl]pipéridine ;
(185) la 4-(N-benzyl-N-méthyl)amino-1-[3-(5-(1,2,4-triazol-4-yl)-1H-indol-3-yl)propyl]pipéridine;
(186) la 4-(N-benzyl-N-méthyl)aminométhyl-1-[3-(5-(1,2,4-triazol-4-yl)-1H-indol-3-yl)propyl]pipéridine;
(187) la 1-[3-(5-(1,2,4-triazol-4-yl)-1H-indol-3-yl)propyl)]-4-[α-(méthyl)benzylamino]pipéridine;
(188) la 1-[3-(5-(1,2,4-triazol-4-yl)-1H-indol-3-yl)propyl)]-4-[α-(hydroxyméthyl)benzylamino]-pipéridine ;
(189) la 1-[3-(5-(1,2,4-triazol-4-yl)-1H-indol-3-yl)propyl)]-4-[(1-hydroxyméthyl-2-phényl)éthylamino]-pipéridine ;
(190) la 4-(N-benzyl)aminométhyl-1-[3-(5-(1,2,4-triazol-4-yl)-1H-indol-3-yl)propyl]pipéridine ;
(191) la 1-[3-(5-(1,2,4-triazol-4-yl)-1H-indol-3-yl)propyl)]-4-[(2-hydroxy-1-méthyl-2-phényl)éthylamino]pipéridine ;
(192) la 1-[3-(5-(1,2,4-triazol-4-yl)-1H-indol-3-yl)propyl)]-4-[2-(4-acétylaminophényl)éthylamino]-pipéridine ;
(193) la 1-[3-(5-(1,2,4-triazol-4-yl)-1H-indol-3-yl)propyl)]-4-[α-(méthyl)benzylamino]méthylpipéridine;
(194) la 1-[3-(5-(1,2,4-triazol-4-yl)-1H-indol-3-yl)propyl)]-4-[1-(4-acétylaminophényl)éthylamino]-méthylpipéridine ;
(195) la 1-[3-(5-(1,2,4-triazol-4-yl)-1H-indol-3-yl)propyl)]-4-[N-[α-(hydroxyméthyl)benzyl]-N-méthylamino]pipéridine ;
(196) la 1-[3-(5-(1,2,4-triazol-4-yl)-1H-indol-3-yl)propyl)]-4-[N-(2-(4-acétylaminophényl)éthyl)-N-méthylamino]pipéridine ;
(197) la 1-[3-(5-(1,2,4-triazol-4-yl)-1H-indol-3-yl)propyl)]-4-[N-(4-acétylaminobenzyl)-N-méthylamino]-méthylpipéridine ;
(198) la 1-[3-(5-(1,2,4-triazol-4-yl)-1H-indol-3-yl)propyl]-4-[N-(thien-2-yl)méthyl-N-méthylamino]-pipéridine ;
(199) la 1-[3-(5-(1,2,4-triazol-4-yl)-1H-indol-3-yl)propyl]-4-[(R)-α-(hydroxyméthyl)benzylamino]méthylpipéridine ;
(200) la 3-(4-acétylaminobenzyl)aminométhyl-1-[2-(5-(1,2,4-triazol-4-yl)-1H-indol-3-yl)éthyl]pyrrolidine ;
(201) la (3R)-3-(N-benzyl)aminométhyl-1-[2-(5-(1,2,4-triazol-4-yl)-1H-indol-3-yl)éthyl]pyrrolidine;
(202) la (3S)-3-(pyridine-4-ylméthyl)aminométhyl-1-[2-(5-(1,2,4-triazol-4-yl)-1H-indol-3-yl)éthyl]-pyrrolidine ;
(203) la 3-(N-benzyl)aminométhyl-1-[2-(5-(1,2,4-triazol-4-yl)-1H-indol-3-yl)éthyl]azétidine ;
(204) la 4-benzyl-4-hydroxy-1-[3-(5-(1,2,4-triazol-4-yl)-1H-indol-3-yl)propyl]pipéridine ;
(205) la 3-(N-benzyl)aminométhyl-1-[3-(5-(1,2,4-triazol-4-yl)-1H-indol-3-yl)propyl]azétidine;
(206) la 4-(N-benzyl)aminométhyl-4-hydroxy-1-[3-(5-(1,2,4-triazol-4-yl)-1H-indol-3-yl)propyl]pipéridine ;
(207) la 4-(N-benzyl-N-méthyl)aminométhyl-4-hydroxy-1-[3-(5-(1,2,4-triazol-4-yl)-1H-indol-3-yl) propyl]-pipéridine ;
(208) la 3-(N-benzyl-N-méthyl)aminométhyl-1-[2-(5-(1,2,4-triazol-4-yl)-1H-indol-3-yl)éthyl]azétidine ;
(209) la (3S)-3-[N-α-(méthyl)benzyl]aminométhyl-1-[2-(5-(1,2,4-triazol-4-yl)-1H-indol-3-yl)éthyl]-pyrrolidine ;
(210) la (3S)-3-[N-(furan-3-ylméthyl)amino]méthyl-1-[2-(5-(1,2,4-triazol-4-yl)-1H-indol-3-yl)éthyl]-pyrrolidine ;
(211) la (3S)-3-[N-(furan-2-ylméthyl)amino]méthyl-1-[2-(5-(1,2,4-triazol-4-yl)-1H-indol-3-yl)éthyl]-pyrrolidine ;
(212) la (3S)-3-[N-α-(hydroxyméthyl)benzyl]aminométhyl-1-[2-(5-(1,2,4-triazol-4-yl)-1H-indol-3-yl)éthyl]-pyrrolidine ;
(213) la (3S)-3-[N-benzyl-N-(2-hydroxy)éthyl]amino-méthyl-1-[2-(5-(1,2,4-triazol-4-yl)-1H-indol-3-yl)éthyl]pyrrolidine;
(214) la (3S)-3-[N-(2-phényléthyl)amino]méthyl-1-[2-(5-(1,2,4-triazol-4-yl)-1H-indol-3-yl)éthyl]pyrrolidine ;
(215) la (3S)-3-[N-(2-phényléthyl)-N-méthylamino]-méthyl-1-[2-(5-(1,2,4-triazol-4-yl)-1H-indol-3-yl)éthyl]pyrrolidine ;
(216) la (3S)-3-(N-α-diméthylbenzyl)aminométhyl-1-[2-(5-(1,2,4-triazol-4-yl)-1H-indol-3-yl)éthyl]-pyrrolidine ;
(217) la (3S)-3-[N-(S)-α-méthylbenzyl]aminométhyl-1-[2-(5-(1,2,4-triazol-1-yl)-1H-indol-3-yl)éthyl]-pyrrolidine ;
(218) la (3S)-3-[N-(R)-α-(hydroxyméthyl)benzyl]amino-méthyl-1-[2-(5-(1,2,4-triazol-1-yl)-1H-indol-3-yl)éthyl]pyrrolidine;
(219) la (3S)-3-(N-benzyl)aminométhyl-1-[2-(5-(1,2,4-triazol-1-ylméthyl)-1H-indol-3-yl)éthyl]pyrrolidine ;
(220) la (3S)-3-[(N-(S)-α-méthylbenzyl]aminométhyl-1-[2-(5-(1,2,4-triazol-1-ylméthyl)-1H-indol-3-yl)éthyl]-pyrrolidine ;
(221) la (3S)-3-[(N-(R)-α-(hydroxyméthyl)benzyl]amino-méthyl-1-[2-(5-(1,2,4-triazol-1-ylméthyl)-1H-indol-3-yl)éthyl]pyrrolidine ;
(222) la (3S)-3-(N-benzyl-N-méthyl)aminométhyl-1-[2-(5-(imidazole-1-yl)-1H-indol-3-yl)éthyl]pyrrolidine ;
(223) la (3S)-3-(N-benzyl-N-méthyl)aminométhyl-1-[2-(5-(1,2,4-triazol-1-ylméthyl)-1H-indol-3-yl)éthyl]-pyrrolidine ;
(224) la (3R)-3-(N-méthyl-N-(S)-α-méthylbenzyl)amino-méthyl-1-[2-(5-(1,2,4-triazol-1-ylméthyl)-1H-indol-3-yl)éthyl]pyrrolidine;
(225) la (3R)-3-[N-méthyl-N-(R)-α-hydroxyméthylbenzyl]-aminométhyl-1-[2-(5-(1,2,4-triazol-1-ylméthyl)-1H-indol-3-yl)éthyl]pyrrolidine;
(226) la (3R)-3-[N-méthyl-N-(S)-α-méthylcyclohexyl-méthyl]aminométhyl-1-[2-(5-(1,2,4-triazol-1-ylméthyl)-1H-indol-3-yl)éthyl]pyrrolidine ;
(227) la (3R)-3-[3-(R)-hydroxy-2-(R)-phénylpipéridine-1-yl]méthyl-1-[2-(5-(1,2,4-triazol-1-ylméthyl)-1H-indol-3-yl)éthyl]pyrrolidine ;
(228) la (3R)-3-[3-(R)-hydroxy-2-(R)-phénylpipéridine-1-yl]méthyl-1-[2-(5-(1,2,4-triazol-1-yl)-1H-indol-3-yl)éthyl]pyrrolidine ;
(229) la 4-hydroxy-4-(phénylsulfinyl)méthyl-1-[3-(5-(1,2,4-triazol-4-yl)-1H-indol-3-yl)propyl]pipéridine ;
(230) (3R)-3-[2-(R,S)-phénylpipéridine-1-yl]méthyl-1-[2-(5-(1,2,4-triazol-1-yl)-1H-indol-3-yl)éthyl]-pyrrolidine ;
(231) la 4-(3,3-diméthylpipéridine-1-yl)méthyl-4-hydroxy-1-[3-(5-(1,2,4-triazol-4-yl)-1H-indol-3-yl)propyl]pipéridine ;
(232) la 4-hydroxy-4-(1,2,3,4-tétrahydroisoquinoline-2-yl)méthyl-1-[3-(5-(1,2,4-triazol-4-yl)-1H-indol-3-yl)propyl]pipéridine;
(233) la 4-hydroxy-4-(N-isobutyl-N-méthyl)aminométhyl-1-[3-(5-(1,2,4-triazol-4-yl)-1H-indol-3-yl)propyl]-pipéridine ;
(234) la 4-[N-benzyl-N-(2-hydroxyéthyl)amino]méthyl-4-hydroxy-1-[3-(5-(1,2,4-triazol-4-yl)-1H-indol-3-yl)propyl]pipéridine ;
(235) la 4-[N-(2,2-diméthylpropyl-N-méthylamino]méthyl-4-hydroxy-1-[3-(5-(1,2,4-triazol-4-yl)-1H-indol-3-yl)propyl]pipéridine ;
(236) la 4-[N-(R)-α-hydroxyméthylbenzyl-N-méthylamino]-méthyl-4-hydroxy-1-[3-(5-(1,2,4-triazol-4-yl)-1H-indol-3-yl)propyl]pipéridine;
(237) la 4-hydroxy-4-(2-pyridylméthyl)amino]méthyl-1-[3-(5-(1,2,4-triazol-4-yl)-1H-indol-3-yl)propyl]-pipéridine ;
(238) la 4-hydroxy-4-(2-méthylphénylméthyl)aminométhyl-1-[3-(5-(1,2,4-triazol-4-yl)-1H-indol-3-yl)propyl]-pipéridine ;
(239) la 4-hydroxy-4-[N-(2-méthylphénylméthyl)-N-méthylamino]méthyl-1-[3-(5-(1,2,4-triazol-4-yl)-1H-indol-3-yl)propyl]pipéridine ;
(240) la 3-(benzylamino)méthyl-3-hydroxy-1-[3-(5-(1,2,4-triazol-4-yl)-1H-indol-3-yl)propyl]pyrrolidine ;
(241) la 3-(benzylamino)méthyl-3-hydroxy-1-[2-(5-(1,2,4-triazol-4-yl)-1H-indol-3-yl)éthyl]pyrrolidine ;
(242) la 1-[3-(5-(1,2,4-triazol-4-yl)-1H-indol-3-yl)propyl]-4-[(R)-α-(carbamoyl-oxyméthyl)benzylamino)-pipéridine ;
(243) la 1-[3-(5-(1,2,4-triazol-4-yl)-1H-indol-3-yl)propyl]-4-[(1R,2S)-2-(hydroxy-1-phénylpropylamino]-pipéridine ;
(244) la 1-[3-(5-(1,2,4-triazol-4-yl)-1H-indol-3-yl)propyl]-4-[(1R,2R)-2-(hydroxy-1-phénylpropylamino]-pipéridine ;
(245) la 1-[3-(5-(1,2,4-triazol-4-yl)-1H-indol-3-yl)propyl]-4-[(R,S)-1-(hydroxy-2-phénylprop-2-ylamino]-pipéridine ;
(246) la 1-[3-(5-(1,2,4-triazol-4-yl)-1H-indol-3-yl)propyl]-4-[(R)-2-(hydroxy-1-(4-fluorophényl)éthylamino]pipéridine ;
(247) la 1-[3-(5-(1,2,4-triazol-4-yl)-1H-indol-3-yl)propyl]-4-[(1R,2R)-2-(hydroxyindan-1-ylamino]-pipéridine ;
(248) la 1-[3-(5-(1,2,4-triazol-4-yl)-1H-indol-3-yl)propyl]-4-[(R,S)-indan-1-ylamino]pipéridine ;
(249) la 1-[3-(5-(1,2,4-triazol-4-yl)-1H-indol-3-yl)propyl]-4-[(R,S)-1-(4-fluorophényl)éthylamino]-pipéridine ;
(250) la 1-[3-(5-(1,2,4-triazol-4-yl)-1H-indol-3-yl)propyl]-4-[(R)-1-phénylprop-2-ylamino]pipéridine ;
(251) la 1-[3-(5-(1,2,4-triazol-4-yl)-1H-indol-3-yl)propyl]-4-[N-(thien-3-ylméthyl)-N-méthylamino]-pipéridine ;
(252) la 1-[3-(5-(1,2,4-triazol-4-yl)-1H-indol-3-yl)propyl]-4-[N-(furan-3-ylméthyl)-N-méthylamino]-pipéridine ;
(253) la 1-[3-(5-(1,2,4-triazol-4-yl)-1H-indol-3-yl)propyl]-4-(furan-3-ylméthyl)aminopipéridine ;
(254) la 1-[3-(5-(1,2,4-triazol-4-yl)-1H-indol-3-yl)propyl]-4-[N,N-di-(furan-3-ylméthyl)amino]-pipéridine ;
(255) la 1-[3-(5-(1,2,4-triazol-4-yl)-1H-indol-3-yl)propyl]-4-[N-allyl-N-méthylamino]pipéridine;
(256) la 1-[3-(5-(1,2,4-triazol-4-yl)-1H-indol-3-yl)propyl]-4-(N-(3,3-diméthylallyl-N-méthylamino)-pipéridine ;
(257) la N,N-Diméthyl-2-[5-(1,2,4-triazol-1-ylméthyl)-1H-indol-3-yl]éthylamine ;
(258) la N,N-Diméthyl-2-[5-(1,3-imidazol-1-ylméthyl)-1H-indol-3-yl]éthylamine ;
(259) la N,N-Diméthyl-2-[5-(5-méthyl-1,2,3,4-triazol-1-ylméthyl)-1H-indol-3-yl]éthylamine;
(260) la N,N-Diméthyl-2-[5-(1,3,4-triazol-1-ylméthyl)-1H-indol-3-yl]éthylamine ;
(261) la N,N-Diméthyl-2-[5-(1,3,4-triazol-1-yl)-1H-indol-3-yl]éthylamine;
(262) la N,N-Diéthyl-2-[5-(1,2,4-triazol-1-ylméthyl)-1H-indol-3-yl]éthylamine ;
(263) la N,N-Diéthyl-2-[5-(1,3-imidazol-1-ylméthyl)-1H-indol-3-yl]éthylamine;
(264) la N,N-Diéthyl-2-[5-(5-méthyl-1,2,3,4-tétrazol-1-ylméthyl)-1H-indol-3-yl]éthylamine ;
(265) la N,N-Diéthyl-2-[5-(1,3,4-triazol-1-ylméthyl)-1H-indol-3-yl]éthylamine ;
(266) la N,N-Diéthyl-2-[5-(1,3,4-triazol-1-yl)-1H-indol-3-yl]éthylamine ;
(267) la N,N-Diméthyl-[5-(1,2,4-triazol-1-ylméthyl)-1H-indol-3-yl]méthylamine ;
(268) la N,N-Diméthyl-[5-(1,3-imidazol-1-ylméthyl)-1H-indol-3-yl]méthylamine;
(269) la N,N-Diméthyl-[5-(5-méthyl-1,2,3,4-tétrazol-1-ylméthyl)-1H-indol-3-yl]méthylamine;
(270) la N,N-Diméthyl-[5-(1,3,4-triazol-1-ylméthyl)-1H-indol-3-yl]méthylamine;
(271) la N,N-Diméthyl-[5-(1,3,4-triazol-1-yl)-1H-indol-3-yl]méthylamine;
(272) la N,N-Diéthyl-3-[5-(1,2,4-triazol-1-ylméthyl)-1H-indol-3-yl]propylamine;
(273) la N,N-Diméthyl-3-[5-(1,3-imidazol-1-yl)-1H-indol-3-yl]propylamine;
(274) la N,N-Diéthyl-3-[5-(5-méthyl-1,2,3,4-tétrazol-1-ylméthyl)-1H-indol-3-yl]propylamine;
(275) la N,N-Diméthyl-3-[5-(1,3,4-triazol-1-ylméthyl)-1H-indol-3-yl]propylamine ;
(276) la N,N-Diéthyl-3-[5-(1,3,4-triazol-1-yl)-1H-indol-3-yl]propylamine ;
(277) la N,N-Diméthyl-4-[5-(3-méthyl-1,2,4,5-tétrazol-1-ylméthyl)-1H-indol-3-yl]butylamine;
(278) la N,N-Diméthyl-4-[5-(2-éthyl-1,3-éthylimidazol-1-ylméthyl)-1H-indol-3-yl]butylamine ;
(279) la N,N-Diméthyl-4-[5-(5-éthyl-1,2,3,4-triazol-1-ylméthyl)-1H-indol-3-yl]butylamine;
(280) la N,-Diméthyl-4-[5-(2-méthyl-1,3,4-triazol-1-ylméthyl)-1H-indol-3-yl]butylamine;
(281) la N,N-Diméthyl-4-[5-(2-éthyl-1,3,4-triazol-1-yl)-1H-indol-3-yl]butylamine;
(282) le 2-[5-(1,2,4-triazol-1-ylméthyl)-1H-indol-3-yl]éthylalcool ;
(283) le 2-[5-(1,3-imidazol-1-ylméthyl)-1H-indol-3-yl]éthylalcool ;
(284) le 2-[5-(5-méthyl-1,2,3,4-tétrazol-1-ylméthyl)-1H-indol-3-yl]éthylalcool ;
(285) le 2-[5-(1,3,4-triazol-1-ylméthyl)-1H-indol-3-yl]éthylalcool ;
(286) le 2-[5-(1,3,4-triazol-1-yl)-1H-indol-3-yl]éthylalcool ;
(287) le [5-(1,2,4-triazol-1-ylméthyl)-1H-indol-3-yl]méthylalcool ;
(288) le 3-[5-(1,3-imidazol-1-ylméthyl)-1H-indol-3-yl]propylalcool ;
(289) le 4-[5-(5-méthyl-1,2,3,4-tétrazol-1-ylméthyl)-1H-indol-3-yl]butylalcool ;
(290) le 2-[5-(2-méthyl-1,3,4-triazol-1-ylméthyl)-1H-indol-3-yl]éthylalcool; et
(291) le 2-[5-(5-méthyl-1,3,4-triazol-1-yl)-1H-indol-3-yl]éthylalcool

20. Procédé selon la revendication 18 dans lequel le composé de formule structurale IV est choisi parmi : 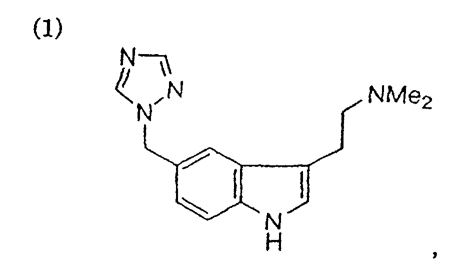 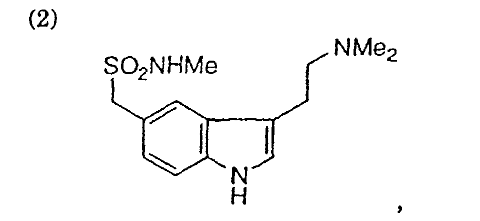 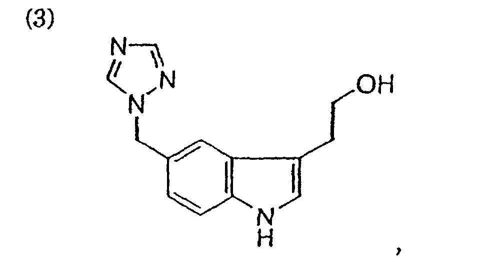 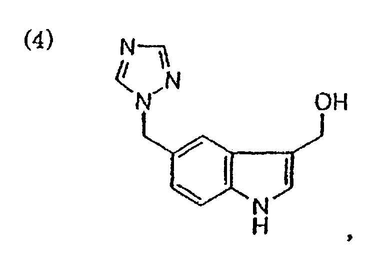 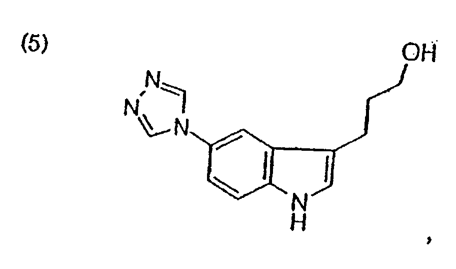 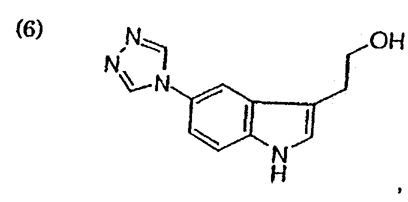 et

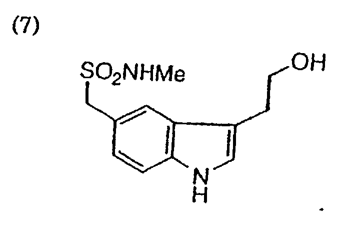

21. Composés de formules structurales (V) et (VI) : 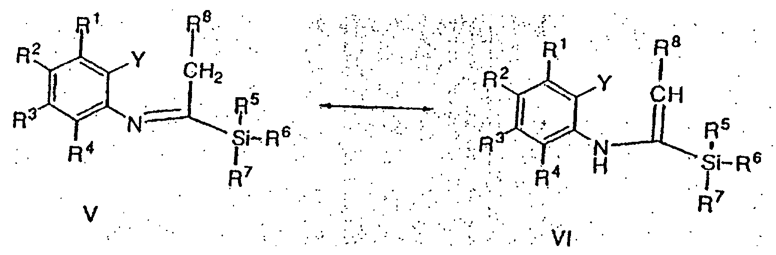 dans lesquelles :
Y est choisi parmi un Br, un I et un triflate ;
R¹, R², R³ et R⁴ sont chacun indépendamment choisi parmi :
(1) un hydrogène
(2) 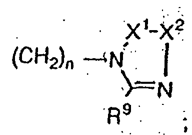
(3) un alkyle C₁₋₆
(4) -(CH₂)ₙ-Z
dans lequel Z représente :
(a) un hydrogène,
(b) un halogène,
(c) un cyano,
(d) un nitro,
(e) un trifluorométhyle,
(f) -OR¹⁰,
(g) -OCOR¹⁰,
(h) -OCONR¹⁰R¹¹,
(i) -OCH₂CN,
(j) -OCH₂CONR¹⁰R¹¹,
(k) -SR¹⁰,
(l) -SOR¹⁰
(m) -SO₂R¹⁰, à condition que R¹⁰ ne soit pas un hydrogène
(n) -SO₂NR¹⁰R¹¹,
(o) -NR¹⁰R¹¹,
(p) -NR¹⁰COR¹¹,
(q) -NR¹⁰CO₂R¹¹,
(r) -NR¹⁰SO₂R¹¹,
(s) -COR¹⁰,
(t) -CO₂R¹⁰,
(u) -CONR¹⁰R¹¹,
ou Z est un groupe de formule (Za), (Zb), (Zc), ou (Zd) : 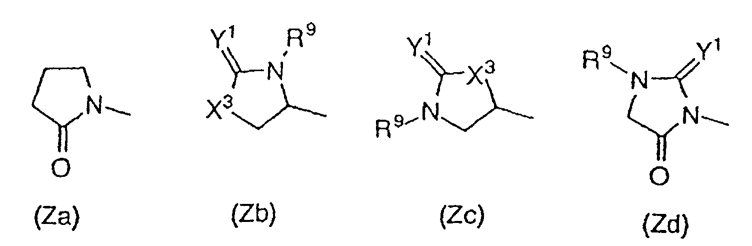 où Z représente un cycle hétéroaromatique à 5 chainons facultativement substitué choisi parmi le furane, le thiophène, le pyrrole, l'oxazole, le thiazole, l'isoxazole, l'isothiazole, l'imidazole, le pyrazole, l'oxadiazole, le thiadiazole, le triazole et le tétrazole ;
R⁵, R⁶, et R⁷ sont chacun indépendamment choisi parmi ;
(1) un alkyle C₁₋₆,
(2) un -O-alkyle C₁₋₆,
(3) un phényle ;
R⁸ est choisi parmi :
(1) un hydrogène,
(2) -R¹⁹-OH,
(3) -R¹⁹-O-R¹⁷, et
(4) -R¹⁹NR¹²R¹³, et
(5) -R¹⁹-Z¹
dans lequel : Z¹ est un hétérocycle de 3 à 7 chaînons dans lequel les chaînons du cycle sont choisis parmi 1 ou 2 atomes d'azote et dans lequel l'hétérocycle peut être substitué par un ou plusieurs R¹⁴ ;
R⁹ est choisi parmi :
(1) un hydrogène, et
(2) un alkyle C₁₋₄ ;
R¹⁰ et R¹¹ sont chacun indépendamment choisis parmi :
(1) un hydrogène,
(2) un alkyle C₁₋₆,
(3) un trifluorométhyle,
(4) un phényle facultativement substitué avec un ou plusieurs substituants R²⁰, et
(5) un méthylphényle, substitué facultativement avec un ou plusieurs substituants R²⁰, et
(6) un groupe arylalkyle C₁₋₆ ou un groupe hétéroarylalkyle C₁₋₆ facultativement substitués avec un ou plusieurs substituants R²⁰, ou
R¹⁰ et R¹¹ lorsqu'ils sont liés par un atome d'azote, représentent ensemble le résidu d'une azétidine facultativement substituée, d'un cycle pyrrolydine, pipéridine, morpholine ou pipérazine facultativement substitués avec un ou plusieurs substituants R¹⁸ ;
R¹² et R¹³ sont chacun indépendamment choisis parmi :
(1) un alkyle C₁₋₄,
(2) un arylC₆-alkyle C₁₋₄ qui peut être non substitué ou substitué avec un à trois substituants choisis parmi un méthyle, un halogène, et un halogénométhyle,
R¹⁴ est choisi parmi :
(1) un arylalkyle C₁₋₆ non substitué ou substitué avec un à trois substituants R²⁰, et
(2) un hétéroarylalkyle C₁₋₆ non substitué ou substitué avec un à trois substituants R²⁰,
R¹⁵ et R¹⁶ sont chacun indépendamment choisis parmi
(1) un hydrogène,
(2) un alkyle C₁₋₆,
(3) un cycloalkyle C₃₋₇,
(4) un cycloalkylC₃₋₇-alkyle C₁₋₆
(5) un indanyle,
(6) un aryle,
(7) un arylalkyle C₁₋₆,
(8) un hétérocycloalkyle C₃₋₇,
(9) un hétérocycloalkylC₃₋₇-alkyle C₁₋₆,
(10) un hétéroaryle, et
(11) un hétéroarylalkyle C₁₋₆,
R¹⁷ est choisi parmi un groupe protecteur hydroxy qui est labile par hydrolyse acide douce ;
R¹⁸ est choisi parmi ;
(1) un alkyle C₁₋₆,
(2) un arylalkyle C₁₋₆,
(3) un alcoxy C₁₋₆,
(4) un alcoxycarbonyle C₂₋₆,
(5) un alkylaminocarbonyle C₁₋₆,
R¹⁹ est une chaîne alkyle C₁₋₆ linéaire ou ramifiée ;
R²⁰ est choisi parmi :
(1) un fluoro,
(2) un cyano,
(3) un trifluorométhyle,
(4) un alkyle C₁₋₆,
(5) un halogénoalkyle C₁₋₆,
(6) un aryle,
(7) un triazolyle,
(8) un tétrazolyle,
(9) un tétrazolylalkyle C₁₋₆,
(10) un hydroxy,
(11) un alcoxy C₁₋₆,
(12) un alkylthio C₁₋₆,
(13) un alcoxycarbonyle C₂₋₆,
(14) un alkylcarbonyle C₂₋₆,
(15) un alkylsulfonyle C₁₋₆,
(16) un arylsulfonyle,
(17) un alkylcarbonylamino- C₂₋₆,
(18) un arylcarbonylamino-,
(19) un alcoxycarbonylamino-C₂₋₆,
(20) un N-alkylC₁₋₆-N-alcoxyamino- C₂₋₆,
(21) un carbonylamino-,
(22) un mono- ou un diarylaminocarbonylamino-,
(23) un pyrrolidinylcarbonylamino-,
(24) un pipéridinylcarbonylamino-,
(25) un aminocarbonyl-,
(26) un aminocarbonylamino-,
(27) un alkylC₁₋₆-aminocarbonyl-,
(28) un alkylC₁₋₆-aminocarbonylamino-,
(29) un dialkylC₁₋₆-aminocarbonyl-,
(30) un dialkylC₁₋₆-aminocarbonylamino-,
(31) un pyrrolidinylcarbonylamino-,
(32) un pipéridinylcarbonylamino-,
(33) un aminosulfonyl-,
(34) un alkylC₁₋₆-aminosulfonyl-,
(35) un alkylC₁₋₆-sulfonylamino-,
(36) un alkylC₁₋₆-sulfonylaminométhyl-,
(37) un arylsulfonylamino-,
(38) un dialkylC₁₋₆-aminosulfonyl-,
(39) un aminosulfonylméthyl-,
(40) un alkylC₁₋₆-aminosulfonylméthyl-,
(41) un dialkylC₁₋₆-aminosulfonylméthyl-,
(42) un -(CH₂)ₘOR¹⁵,
(43) un -(CH₂)ₘSR¹⁵, à condition que R¹⁵ ne soit pas de l'hydrogène
(44) un -(CH₂)ₘSOR¹⁵,
(45) un -(CH₂)ₘSO₂R¹⁵,
(46) un -(CH₂)ₘNR¹⁵R¹⁶,
(47) =O, et
(48) 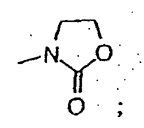
X¹ et X² sont chacun indépendamment choisis parmi des cycles azotés ou des cycles carbonés ;
X³ est choisi parmi le groupe constitué de l'oxygène, du soufre, de -NH- ou du méthylène ;
Y¹ est un oxygène ou un soufre ;
n est un nombre entier indépendamment choisi pour chaque événement compris entre 0 et 1 ; et
m est un nombre entier choisi indépendamment pour chaque événement compris entre 0 et 4.
